# EUROPEAN PATENT SPECIFICATION

(11) **EP 4 340 582 B1**
(45) Date of publication and mention of the grant of the patent: **07.10.2026**
(21) Application number: 22214889.2
(22) Date of filing: 20.12.2022
(51) Int. Cl.: H10K 85/60, H10K 85/30, C07F 1/08, C09K 11/00, H10K 50/17, H10K 50/19

(54) **ORGANIC ELECTRONIC DEVICE COMPRISING A CHARGE GENERATION LAYER**
ORGANISCHE ELEKTRONISCHE VORRICHTUNG MIT EINER LADUNGSERZEUGUNGSSCHICHT
DISPOSITIF ÉLECTRONIQUE ORGANIQUE COMPRENANT UNE COUCHE DE GÉNÉRATION DE CHARGE

(30) Priority: 19.09.2022 WO PCT/EP2022/075945
(43) Date of publication of application: 20.03.2024
(73) Proprietor: Novaled GmbH, 01099 Dresden (DE)
(72) Inventor: UVAROV, Vladimir, 01099 Dresden (DE); PINTER, Piermaria, 01099 Dresden (DE); LUSCHTINETZ, Regina, 01099 Dresden (DE); ROSENOW, Thomas, 01099 Dresden (DE)
(74) Representative: Michalski Hüttermann & Partner mbB

(56) References cited:
- EP-A1- 3 462 516
- EP-A1- 4 199 131
- WO-A1-2021/048044
- WO-A1-2022/023260

## Description

### Technical Field

The present invention relates to an organic electronic device comprising an anode layer, a cathode layer, a hole injection layer, a first emission layer and a charge generation layer, wherein the charge generation layers, wherein the charge generation layer comprises a p-type charge generation layer and a n-type charge generation layer, wherein the p-type charge generation layer comprises at least one organic hole transport compound and at least one compound of formula (I).

### Background Art

Organic electronic devices, such as organic light-emitting diodes OLEDs, which are selfemitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

EP 4 199 131 A1 refers to a to a Ce(IV) metal complex, an organic electronic device comprising an anode layer, a cathode layer and a charge generation layer, wherein the charge generation layer comprises a p-type charge generation layer that comprises the Ce(IV) metal complex and a n-type charge generation layer.

EP 3 462 516 A1 refers to an organic light-emitting diodes (OLEDs), which are selfemitting devices.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the p-type charge generation layer, and among them, may be affected by characteristics of the organic hole transport compound and/or the compound of formula (I) which are contained in the p-type charge generation layer.

There remains a need to improve performance of organic electronic devices comprising a charge generation layer, in particular to achieve improved operating voltage, cd/A efficiency and/or lifetime through improving the characteristics of the compounds comprised therein.

Furthermore, there remains a need to provide compounds which are suitable for mass production of organic electronic devices, in particular with regards to thermal properties and/or reduced health and safety risks compared to the art.

### DISCLOSURE

An aspect of the present invention provides an organic electronic device comprising an anode layer, a cathode layer, a hole injection layer, a first emission layer and a charge generation layer, wherein the charge generation layer comprises a p-type charge generation layer and a n-type charge generation layer,
wherein
- the n-type charge generation layer comprising an organic electron transport compound and a metal dopant, wherein the metal dopant is selected from an alkali or rare earth metal;
- the p-type charge generation layer comprises:
   - an organic hole transport compound, and
   - a compound of formula (I):

      M^{n⊕}(L^{⊝})ₙ(AL)ₘ (I),

      wherein
      - M: is a metal ion;
      - n: is the valency of M and selected from 1 to 3;
      - L: is a ligand of formula (II) 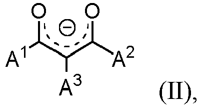
      wherein
      - A¹ and A²: are independently selected from substituted or unsubstituted C₁ to C₁₂ alkyl, substituted or unsubstituted C₆ to C₁₂ aryl, substituted or unsubstituted C₃ to C₁₂ heteroaryl;
      wherein the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, SO₂R¹, halogen, F or CN,
      wherein R¹ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
      - A³: is selected from H, D, CN, substituted or unsubstituted C₁ to C₄ alkyl, partially or perfluorinated C₁ to C₄ alkyl, substituted or unsubstituted C₆ to C₁₀ aryl, substituted or unsubstituted C₃ to C₉ heteroaryl, or whereby A³ form a substituted or unsubstituted cyclic structure with either A¹ or A², preferably A³ is selected from a cyclic structure with either A¹ or A² of 5- or 6- membered aliphatic or aromatic rings, whereby the ring optional comprise one or more heteroatoms, preferably selected from the group comprising N, O and S,
      wherein the one or more substituents of A³ or the substituents of the substituted cyclic structure are independently selected from halogen, F, Cl, CN, CF₃;
      - AL: is an ancillary ligand which coordinates to the metal M;
      - m: is an integer selected from 0 to 2;
wherein the hole injection layer, the first emission layer and the charge generation layer are arranged between the anode layer and the cathode layer, and the n-type charge generation layer is arranged closer to the anode layer and the p-type charge generation layer is arranged closer to the cathode layer.

### Definitions

It should be noted that throughout the application and the claims the p-type charge generation layer is not a hole injection layer (HIL).

It should be noted that throughout the application and the claims any A¹, A², A³, AL, n, m, M, L, R¹, R², R³, Ar¹, Ar², Ar³, Ar⁴ and Ar⁵, T¹, T², T³, T⁴, T⁵ and T⁶, Aa¹, Aa², Aa³ each R', Aar¹, Aar², Aar³, Z¹, Z² and Z³, Ar³¹ and Ar³², R³¹, R³² and R³³, r31, r32 and r33, Ar⁴¹ and Ar⁴², L⁴¹ and L⁴², R⁴¹ to R⁴⁸, etc. always refer to the same moieties as defined in the specification, unless otherwise noted.

In the present specification, when a definition is not otherwise provided, "substituted" refers to at least one substituted selected from the group comprising D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, SO₂R¹, halogen, F or CN,
wherein R¹ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

In the present specification, when a definition is not otherwise provided, a substituted aryl group with at least 6 C-ring atoms can be substituted with 1, 2, 3, 4 or 5 substituents. For example a substituted C₆ aryl group may have 1, 2, 3, 4 or 5 phenyl substituents.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, a substituted heteroaryl group with at least 2 C-ring atoms can be substituted with one or more substituents. For example a substituted C₂ heteroaryl group may have 1 or 2 substituents.

In the present specification, when a definition is not otherwise provided, an "alkyl group" refers to a saturated aliphatic hydrocarbyl group. The alkyl group may be a C₁ to C₆ alkyl group. More specifically, the alkyl group may be a C₁ to C₄ alkyl group or a C₁ to C₃ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an iso-propyl group, a butyl group, an iso-butyl group, a sec-butyl group, a tert-butyl group, a pentyl group, a branched pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methyl cyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from N, P, O, S, F; more preferably from N, O, F.

In the present specification, "aryl group" and "aromatic rings" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluorenyl.

Analogously, under "heteroaryl" and "heteroaromatic", it is especially where suitable understood a group derived by formal abstraction of one ring hydrogen from a heterocyclic aromatic ring in a compound comprising at least one such ring.

The term "non-heterocycle" is understood to mean a ring or ring-system comprising no hetero-atom as a ring member.

The term "heterocycle" is understood to mean that the heterocycle comprises at least one ring comprising one or more hetero-atoms. A heterocycle comprising more than one ring means that all rings comprising a hetero-atom or at least one ring comprising a hetero atom and at least one ring comprising C-atoms only and no hetero atom.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common sp²-hybridized carbon atoms.

The term "fused ring system" is understood to mean a ring system wherein two or more rings share at least two atoms.

The term "5-, 6- or 7-member ring" is understood to mean a ring comprising 5, 6 or 7 atoms. The atoms may be selected from C and one or more hetero-atoms.

In the present specification, the single bond refers to a direct bond.

In the present specification, when a definition is not otherwise provided, "substituted" refers to one substituted with a H, deuterium, C₁ to C₁₂ alkyl, unsubstituted C₆ to C₁₈ aryl, and unsubstituted C₃ to C₁₈ heteroaryl.

In the present specification, when a substituent is not named, the substituent can be a H.

In the context, wherein "different" means that the compounds do not have an identical chemical structure.

The term "cyano moiety" refers to a CN substituent.

In the present specification, the single bond refers to a direct bond.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" and organic electronic device are used synonymously.

The terms anode, anode layer and anode electrode are used synonymously.

The terms cathode, cathode layer and cathode electrode are used synonymously.

The term "n-type charge generation layer" may also be named n-type CGL or n-CGL or electron generation layer. These terms are used synonymously.

The term "n-type charge generation layer" is understood to mean a layer which improves electron generation and transport layer into further layers in the organic electronic device.

The term "p-type charge generation layer" may also be named p-type CGL or p-CGL or hole generation layer. These terms are used synonymously.

The term "p-type charge generation layer" is understood to mean a layer which improves hole generation and transport into further layers in the organic electronic device.

The term "charge generation connecting layer" is may also be named c-CGL or interlayer or buffer layer. These terms are used synonymously.

The term "charge generation connecting layer" is understood to mean a layer which enables charge generation and transport between the n-type CGL and the p-type CGL.

The term "hole injection layer" is understood to mean a layer which enables hole injection from the anode layer into further layers in the organic electronic device.

The term "hole transport layer" is understood to mean a layer which transports holes between the anode layer and the cathode layer.

The term "top emission device" is understood to mean an organic electronic device wherein the light is emitted through the cathode layer.

The term "bottom emission device" is understood to mean an organic electronic device wherein the light is emitted through the substrate.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) energy.

The term "HOMO energy" is understood to mean the highest occupied molecular orbital and is determined in eV (electron volt).

The term "HOMO energy further away from vacuum level" is understood to mean that the absolute value of the HOMO energy is higher than the absolute value of the HOMO energy of the reference compound. For example, the term "further away from vacuum level than the HOMO energy of N2,N2,N2',N2',N7,N7,N7',N7'-octakis(4-methoxyphenyl)-9,9'-spirobi[fluorene]-2,2',7,7'-tetraamine is understood to mean that the absolute value of the HOMO energy of the organic hole transport compound of the p-type charge generation layer is higher than the HOMO energy of N2,N2,N2',N2', N7,N7, N7',N7'-octakis(4-methoxyphenyl)-9,9'-spirobi[fluorene]-2,2',7,7'-tetraamine.

The term "absolute value" is understood to mean the value without the "-" symbol.

### Advantageous Effects

Surprisingly, it was found that the organic electronic device according to the invention solves the problem underlying the present invention by enabling organic electronic devices, such as organic light-emitting diodes, in various aspects superior over the organic electronic devices known in the art, with respect to operating voltage, external quantum efficiency EQE, lifetime and/or voltage stability over time and in particular to operating voltage, cd/A efficiency and/or lifetime.

Additionally, it was found that the problem underlying the present invention can be solved by using compounds which may be suitable for deposition through vacuum thermal evaporation under conditions suitable for mass production. In particular, the thermal stability and/or rate onset temperature of the compound of formula (I) and/or the organic hole transport compound of the present invention may be selected such being in a range suitable for mass production.

Additionally, the LUMO of the compound of formula (I) used may be particularly suitable for efficient hole generation in the p-type charge generation layer.

### Embodiments of the organic electronic device

According to one embodiment, wherein the organic electronic device comprises an anode layer, a cathode layer, a hole injection layer, a first emission layer and a charge generation layer, wherein the charge generation layer comprises a p-type charge generation layer and a n-type charge generation layer,
wherein
- the n-type charge generation layer comprising an organic electron transport compound and a metal dopant, wherein the metal dopant is selected from an alkali or rare earth metal;
- the p-type charge generation layer comprises:
- an organic hole transport compound, and
- a compound of formula (I):

   M^{n⊕}(L^{⊝})ₙ(AL)ₘ (I),

   wherein
   - M: is a metal ion;
   - n: is the valency of M and selected from 1 to 3;
   - L: is a ligand of formula (II) 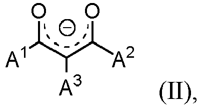
   wherein
   - A¹ and A²: are independently selected from substituted or unsubstituted C₁ to C₁₂ alkyl, substituted or unsubstituted C₆ to C₁₂ aryl, substituted or unsubstituted C₃ to C₁₂ heteroaryl;
   wherein the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, SO₂R¹, halogen, F or CN,
   wherein R¹ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
   - A³: is selected from H, D, CN, substituted or unsubstituted C₁ to C₄ alkyl, partially or perfluorinated C₁ to C₄ alkyl, substituted or unsubstituted C₆ to C₁₀ aryl, substituted or unsubstituted C₃ to C₉ heteroaryl, or whereby A³ form a substituted or unsubstituted cyclic structure with either A¹ or A², preferably A³ is selected from a substituted or unsubstituted cyclic structure with either A¹ or A² of 5- or 6-membered aliphatic or aromatic rings, whereby the ring optional comprise one or more heteroatoms, preferably selected from the group comprising N, O and S,
   wherein the one or more substituents of A³ or the substituents of the substituted cyclic structure are independently selected from halogen, F, Cl, CN, CF₃;
   wherein
   - L: comprises at least four groups selected from F, CN and/or CF₃, also preferred at least four F atoms and less than 30 F atoms;
   - AL: is an ancillary ligand which coordinates to the metal M;
   - m: is an integer selected from 0 to 2;
wherein the hole injection layer, the first emission layer and the charge generation layer are arranged between the anode layer and the cathode layer, and the n-type charge generation layer is arranged closer to the anode layer and the p-type charge generation layer is arranged closer to the cathode layer.

Thereby, the compound of formula (I) may have properties which are particularly suitable for mass production of organic electronic devices, in particular thermal properties and/or LUMO.

According to one embodiment, wherein the organic electronic device comprises an anode layer, a cathode layer, a hole injection layer, a first emission layer and a charge generation layer, wherein the charge generation layer comprises a p-type charge generation layer and a n-type charge generation layer,
wherein
- the n-type charge generation layer comprising an organic electron transport compound and a metal dopant, wherein the metal dopant is selected from an alkali or rare earth metal;
- the p-type charge generation layer comprises:
   - an organic hole transport compound, and
   - a compound of formula (I):

      M^{n⊕}(L^{⊝})ₙ(AL)ₘ (I),

      wherein
      - M: is a metal ion;
      - n: is the valency of M and selected from 1 to 3;
      - L: is a ligand of formula (II) 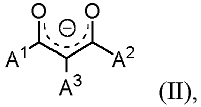
      wherein
      - A¹ and A²: are independently selected from substituted or unsubstituted C₁ to C₁₂ alkyl, substituted or unsubstituted C₆ to C₁₂ aryl, substituted or unsubstituted C₃ to C₁₂ heteroaryl;
      wherein the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, SO₂R¹, halogen, F or CN,
      wherein R¹ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
      - A³: is selected from H, D, CN, substituted or unsubstituted C₁ to C₄ alkyl, partially or perfluorinated C₁ to C₄ alkyl, substituted or unsubstituted C₆ to C₁₀ aryl, substituted or unsubstituted C₃ to C₉ heteroaryl, or whereby A³ form a substituted or unsubstituted cyclic structure with either A¹ or A², preferably A³ is selected from a cyclic structure with either A¹ or A² of 5- or 6- membered aliphatic or aromatic rings, whereby the ring optional comprise one or more heteroatoms, preferably selected from the group comprising N, O and S,
      wherein the one or more substituents of A³ or the substituents of the substituted cyclic structure cyclic structure are independently selected from halogen, F, Cl, CN, CF₃;
      - AL: is an ancillary ligand which coordinates to the metal M;
      - m: is an integer selected from 0 to 2;
      wherein the hole injection layer, the first emission layer and the charge generation layer are arranged between the anode layer and the cathode layer, and the n-type charge generation layer is arranged closer to the anode layer and the p-type charge generation layer is arranged closer to the cathode layer, and the charge generation layer is arranged between the first emission layer and the cathode layer.

According to one embodiment, wherein the organic electronic device comprises an anode layer, a cathode layer, a hole injection layer, a first emission layer and a charge generation layer, wherein the charge generation layer comprises a p-type charge generation layer and a n-type charge generation layer,
wherein
- the n-type charge generation layer comprising an organic electron transport compound and a metal dopant, wherein the metal dopant is selected from an alkali or rare earth metal;
- the p-type charge generation layer comprises:
   - an organic hole transport compound, and
   - a compound of formula (I):

      M^{n⊕}(L^{⊝})ₙ (AL)ₘ (I),

      wherein
      - M: is a metal ion;
      - n: is the valency of M and selected from 1 to 3;
      - L: is a ligand of formula (II) 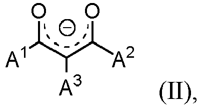
      wherein
      - A¹ and A²: are independently selected from substituted or unsubstituted C₁ to C₁₂ alkyl, substituted or unsubstituted C₆ to C₁₂ aryl, substituted or unsubstituted C₃ to C₁₂ heteroaryl;
      wherein the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, SO₂R¹, halogen, F or CN,
      wherein R¹ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
      - A³: is selected from a substituted or unsubstituted cyclic structure with either A¹ or A² of 5- or 6- membered aliphatic or aromatic rings, whereby the ring optional comprise one or more heteroatoms, preferably selected from the group comprising N, O and S, wherein the one or more substituents of the cyclic structure are independently selected from halogen, F, Cl CN, CF₃;
      - AL: is an ancillary ligand which coordinates to the metal M;
      - m: is an integer selected from 0 to 2;
      wherein the hole injection layer, the first emission layer and the charge generation layer are arranged between the anode layer and the cathode layer, and the n-type charge generation layer is arranged closer to the anode layer and the p-type charge generation layer is arranged closer to the cathode layer, and optional the charge generation layer is arranged between the first emission layer and the cathode layer.

According to one embodiment, wherein the organic electronic device comprises an anode layer, a cathode layer, a hole injection layer, a first emission layer and a charge generation layer, wherein the charge generation layer comprises a p-type charge generation layer and a n-type charge generation layer,
wherein
- the n-type charge generation layer comprising an organic electron transport compound and a metal dopant, wherein the metal dopant is selected from an alkali or rare earth metal;
- the p-type charge generation layer comprises:
   - an organic hole transport compound, and
   - a compound of formula (I):

      M^{n⊕}(L^{⊝})ₙ (AL)ₘ (I),

      wherein
      - M: is a metal ion;
      - n: is the valency of M and selected from 1 to 3;
      - L: is a ligand of formula (II) 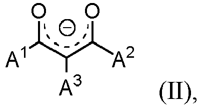
      wherein
      - A¹ and A²: are independently selected from substituted or unsubstituted C₁ to C₁₂ alkyl, substituted or unsubstituted C₆ to C₁₂ aryl, substituted or unsubstituted C₃ to C₁₂ heteroaryl;
      wherein the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, SO₂R¹, halogen, F or CN,
      wherein R¹ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
      - A³: is selected from H, D, CN, unsubstituted C₁ to C₄ alkyl, partially or perfluorinated C₁ to C₄ alkyl, or whereby A³ form a substituted or unsubstituted cyclic structure with either A¹ or A², wherein the one or more substituent of A³ or the substituents of the substituted cyclic structure are independently selected from halogen, F, Cl, CN, CF₃;
      - AL: is an ancillary ligand which coordinates to the metal M;
      - m: is an integer selected from 0 to 2;
      wherein the hole injection layer, the first emission layer and the charge generation layer are arranged between the anode layer and the cathode layer, and the n-type charge generation layer is arranged closer to the anode layer and the p-type charge generation layer is arranged closer to the cathode layer, and optional the charge generation layer is arranged between the first emission layer and the cathode layer.

According to one embodiment, wherein the organic electronic device comprises an anode layer, a cathode layer, a hole injection layer, a first emission layer and a charge generation layer, wherein the charge generation layer comprises a p-type charge generation layer and a n-type charge generation layer,
wherein
- the n-type charge generation layer comprising an organic electron transport compound and a metal dopant, wherein the metal dopant is selected from an alkali or rare earth metal;
- the p-type charge generation layer comprises:
   - an organic hole transport compound, and
   - a compound of formula (I):

      M^{n⊕}(L^{⊝})ₙ (AL)ₘ (I),

      wherein
      - M: is a metal ion;
      - n: is the valency of M and selected from 1 to 3;
      - L: is a ligand of formula (II) 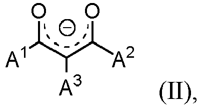
      wherein
      - A¹ and A²: are independently selected from substituted or unsubstituted C₁ to C₁₂ alkyl, substituted or unsubstituted C₆ to C₁₂ aryl, substituted or unsubstituted C₃ to C₁₂ heteroaryl;
      wherein the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, SO₂R¹, halogen, F or CN,
      wherein R¹ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
      - A³: is selected from H, D, CN, unsubstituted C₁ to C₄ alkyl, partially or perfluorinated C₁ to C₄ alkyl;
      - AL: is an ancillary ligand which coordinates to the metal M;
      - m: is an integer selected from 0 to 2;
      wherein the hole injection layer, the first emission layer and the charge generation layer are arranged between the anode layer and the cathode layer, and the n-type charge generation layer is arranged closer to the anode layer and the p-type charge generation layer is arranged closer to the cathode layer, and optional the charge generation layer is arranged between the first emission layer and the cathode layer.

According to one embodiment the organic electronic device comprises an anode layer, a cathode layer, a hole injection layer, a first emission layer and a charge generation layer, wherein the charge generation layer comprises a p-type charge generation layer and a n-type charge generation layer, wherein the n-type charge generation layer comprises an organic electron transport compound and a metal dopant, wherein the metal dopant is selected from a metal comprising Li or Yb; and
the p-type charge generation layer comprises:
   - an organic hole transport compound, and
   - a compound of formula (I):

      M^{n⊕}(L^{⊝})ₙ (AL)ₘ (I)

      wherein
      - M: is a metal ion;
      - n: is the valency of M and selected from 1 to 3;
      - L: is a ligand of formula (II) 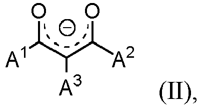
      wherein
      - A¹ and A²: are independently selected from substituted or unsubstituted C₁ to C₁₂ alkyl, substituted or unsubstituted C₆ to C₁₂ aryl, substituted or unsubstituted C₃ to C₁₂ heteroaryl;
      wherein the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, SO₂R¹, halogen, F or CN,
      wherein R¹ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
      - A³: is selected from H, D, CN, substituted or unsubstituted C₁ to C₄ alkyl, partially or perfluorinated C₁ to C₄ alkyl, substituted or unsubstituted C₆ to C₁₀ aryl, substituted or unsubstituted C₃ to C₉ heteroaryl, or whereby A³ form a substituted or unsubstituted cyclic structure with either A¹ or A², preferably A³ is selected from CN, unsubstituted C₁ to C₄ alkyl, partially or perfluorinated C₁ to C₄ alkyl,
      wherein the one or more substituents of A³ or the substituent of the substituted cyclic structure are independently selected from halogen, F, Cl, CN, CF₃;
      - AL: is an ancillary ligand which coordinates to the metal M;
      - m: is an integer selected from 0 to 2;
wherein the hole injection layer, the first emission layer and the charge generation layer are arranged between the anode layer and the cathode layer, and the n-type charge generation layer is arranged closer to the anode layer and the p-type charge generation layer is arranged closer to the cathode layer, and optional the charge generation layer is arranged between the first emission layer and the cathode layer.

According to another embodiment the organic electronic device comprises an anode layer, a cathode layer, a hole injection layer, a first emission layer and a charge generation layer, wherein the charge generation layer comprises a p-type charge generation layer and a n-type charge generation layer, wherein the n-type charge generation layer comprises an organic electron transport compound and a metal dopant, wherein the metal dopant is selected from a metal comprising Li or Yb; and
the p-type charge generation layer comprises:
   - an organic hole transport compound, and
   - a compound of formula (I):

      M^{n⊕}(L^{⊝})ₙ (AL)ₘ (I),

      wherein
      - M: is a metal ion selected from the group comprising an alkali, alkaline earth, transition metal, rare earth metal or group III metal, early transition metal, period 4 transition metal, and preferably selected from the group comprising Li, Na, K, Rb or Cs, Mg, Ca, Sr or Ba, Sc, Y, La, Ti, Zr, V, Nb, Ta, Cr, Mo, W, Mn, Re, Fe, Ru, Co, Ni, Cu, Ag, Au, Zn, Al, Fe, Cr, Mn, Ru or Cu, further preferred M is selected from Fe or Cu, and more preferred Fe;
      - n: is the valency of M and selected from 1 to 3;
      - L: is a ligand of formula (II) 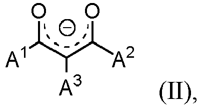
      wherein
      - A¹ and A²: are independently selected from substituted or unsubstituted C₁ to C₁₂ alkyl, substituted or unsubstituted C₆ to C₁₂ aryl, substituted or unsubstituted C₃ to C₁₂ heteroaryl;
      wherein the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, SO₂R¹, halogen, F or CN,
      wherein R¹ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
      - A³: is selected from H, D, CN, substituted or unsubstituted C₁ to C₄ alkyl, partially or perfluorinated C₁ to C₄ alkyl, substituted or unsubstituted C₆ to C₁₀ aryl, substituted or unsubstituted C₃ to C₉ heteroaryl, or whereby A³ form a substituted or unsubstituted cyclic structure with either A¹ or A², preferably A³ is selected from CN, unsubstituted C₁ to C₄ alkyl, partially or perfluorinated C₁ to C₄ alkyl,
      wherein the one or more substituents of A³ or the substituent of the substituted cyclic structure are independently selected from halogen, F, Cl, CN, CF₃;
      - AL: is an ancillary ligand which coordinates to the metal M;
      - m: is an integer selected from 0 to 2;
wherein the hole injection layer, the first emission layer and the charge generation layer are arranged between the anode layer and the cathode layer, and the n-type charge generation layer is arranged closer to the anode layer and the p-type charge generation layer is arranged closer to the cathode layer, and optional the charge generation layer is arranged between the first emission layer and the cathode layer.

According to one embodiment the organic electronic device comprises an anode layer, a cathode layer, a hole injection layer, a first emission layer and a charge generation layer, wherein the charge generation layer comprises a p-type charge generation layer and a n-type charge generation layer, wherein the n-type charge generation layer comprises an organic electron transport compound and a metal dopant, wherein the metal dopant is selected from a metal comprising Li or Yb; and
the p-type charge generation layer comprises:
   - an organic hole transport compound, and
   - a compound of formula (I):

      M^{n⊕}(L^{⊝})ₙ (AL)ₘ (I),

      wherein
      - M: is a metal ion;
      - n: is the valency of M and selected from 1 to 3;
      - L: is a ligand of formula (II) 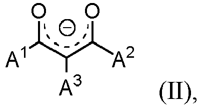
      wherein
      - A¹ and A²: are independently selected from substituted or unsubstituted C₁ to C₁₂ alkyl, substituted or unsubstituted C₆ to C₁₂ aryl, substituted or unsubstituted C₃ to C₁₂ heteroaryl;
      wherein the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, SO₂R¹, halogen, F or CN,
      wherein R¹ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
      - A³: is selected from H, D, CN, substituted or unsubstituted C₁ to C₄ alkyl, partially or perfluorinated C₁ to C₄ alkyl, substituted or unsubstituted C₆ to C₁₀ aryl, substituted or unsubstituted C₃ to C₉ heteroaryl, or whereby A³ form a substituted or unsubstituted cyclic structure with either A¹ or A², preferably A³ is selected from CN, unsubstituted C₁ to C₄ alkyl, partially or perfluorinated C₁ to C₄ alkyl,
      wherein the one or more substituents of A³ or the substituent of the substituted cyclic structure are independently selected from halogen, F, Cl, CN, CF₃;

      wherein L comprises at least four groups selected from F, CN and/or CF₃, also preferred at least four F atoms and less than 30 F atoms;
         - AL: is an ancillary ligand which coordinates to the metal M;
         - m: is an integer selected from 0 to 2;
      wherein the hole injection layer, the first emission layer and the charge generation layer are arranged between the anode layer and the cathode layer, and the n-type charge generation layer is arranged closer to the anode layer and the p-type charge generation layer is arranged closer to the cathode layer, and optional the charge generation layer is arranged between the first emission layer and the cathode layer.

According to another embodiment the organic electronic device comprises an anode layer, a cathode layer, a hole injection layer, a first emission layer and a charge generation layer, wherein the charge generation layer comprises a p-type charge generation layer and a n-type charge generation layer, wherein the n-type charge generation layer comprises an organic electron transport compound and a metal dopant, wherein the metal dopant is selected from a metal comprising Li or Yb; and
the p-type charge generation layer comprises:
- an organic hole transport compound, and
- a compound of formula (I):

   M^{n⊕}(L^{⊝})ₙ (AL)ₘ (I),

   wherein
   - M: is a metal ion selected from the group comprising an alkali, alkaline earth, transition metal, rare earth metal or group III metal, early transition metal, period 4 transition metal, and preferably selected from the group comprising Li, Na, K, Rb or Cs, Mg, Ca, Sr or Ba, Sc, Y, La, Ti, Zr, V, Nb, Ta, Cr, Mo, W, Mn, Re, Fe, Ru, Co, Ni, Cu, Ag, Au, Zn, Al, Fe, Cr, Mn, Ru or Cu, further preferred M is selected from Fe or Cu, and more preferred Fe;
   - n: is the valency of M and selected from 1 to 3;
   - L: is a ligand of formula (II)

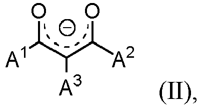

   wherein the ligand L of formula (II) comprises ≥ 16 covalently bound atoms and ≤ 80 covalently bound atoms, wherein ≥ 8 atoms and ≤ 24 are selected from carbon atoms and ≥ 4 atoms and ≤ 24 are selected from hetero atoms selected from the group comprising N, O and F, preferably ≥ 8 atoms and ≤ 24 are selected from carbon atoms and ≥ 4 atoms and ≤ 24 are selected from hetero atoms selected from the group comprising N, O and F, wherein the number of O atoms is 2 and the number of N atoms is 0 to 4, preferably the number of N atoms is 0, 1 or 2;
      - A¹ and A²: are independently selected from substituted or unsubstituted C₁ to C₁₂ alkyl, substituted or unsubstituted C₆ to C₁₂ aryl, substituted or unsubstituted C₃ to C₁₂ heteroaryl;
      wherein the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, SO₂R¹, halogen, F or CN,
      wherein R¹ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
      - A³: is selected from H, D, CN, substituted or unsubstituted C₁ to C₄ alkyl, partially or perfluorinated C₁ to C₄ alkyl, substituted or unsubstituted C₆ to C₁₀ aryl, substituted or unsubstituted C₃ to C₉ heteroaryl or whereby A³ form a substituted or unsubstituted cyclic structure with either A¹ or A², preferably A³ is selected from CN, unsubstituted C₁ to C₄ alkyl, partially or perfluorinated C₁ to C₄ alkyl, and further more preferred A³ is selected from a cyclic structure with either A¹ or A² of 5- or 6- membered aliphatic or aromatic rings, whereby the ring optional comprise one or more heteroatoms, preferably selected from the group comprising N, O and S,
      wherein the one or more substituents of A³ or the substituent of the substituted cyclic structure are independently selected from halogen, F, Cl, CN, CF₃;
      - AL: is an ancillary ligand which coordinates to the metal M;
      - m: is an integer selected from 0 to 2;
   wherein the hole injection layer, the first emission layer and the charge generation layer are arranged between the anode layer and the cathode layer, and the n-type charge generation layer is arranged closer to the anode layer and the p-type charge generation layer is arranged closer to the cathode layer, and optional the charge generation layer is arranged between the first emission layer and the cathode layer.

### A¹, A² and A³

According to one embodiment, wherein
- A¹ and A²: are independently selected from substituted or unsubstituted C₁ to C₁₂ alkyl, substituted or unsubstituted C₆ to C₁₂ aryl, substituted or unsubstituted C₃ to C₁₂ heteroaryl, preferably substituted C₁ to C₁₂ alkyl, substituted C₆ to C₁₂ aryl, substituted C₃ to C₁₂ heteroaryl, wherein
the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, halogen, F or CN, wherein
R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy; and
- A³: is selected from H, D, CN, substituted or unsubstituted C₁ to C₄ alkyl, partially or perfluorinated C₁ to C₄ alkyl, substituted or unsubstituted C₆ to C₁₀ aryl, substituted or unsubstituted C₃ to C₉ heteroaryl, or whereby A³ is selected form a substituted or unsubstituted cyclic structure with either A¹ or A², wherein the one or more substituent of A³ or the substituent of the substituted cyclic structure are independently selected from halogen, F, Cl, CN, CF₃.

According to one embodiment, wherein
- A¹ and A²: are independently selected from substituted or unsubstituted C₁ to C₁₂ alkyl, substituted or unsubstituted C₆ to C₁₂ aryl, substituted or unsubstituted C₃ to C₁₂ heteroaryl, preferably substituted C₁ to C₁₂ alkyl, substituted C₆ to C₁₂ aryl, substituted C₃ to C₁₂ heteroaryl, wherein
the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, halogen, F or CN,
wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
- A³: is selected from H, D or CN, preferably H or CN.

According to one embodiment of the organic electronic device, wherein
- the substituents on A¹ and A² are independently selected from halogen, preferably F, C₁ to C₃ perhalogenated, perfluorinated alkyl or perfluorinated alkoxy, preferably perfluorinated C₁ to C₃ alkyl or perfluorinated C₁ to C₃ alkoxy, or -(O)₁-CₘH₂ₘ-CₙHalOₙ₂ₙ₊₁ with l= 0 or 1, preferably 0, m = 1 or 2, preferably 1 and n = 1 to 3, preferably n =1 or 2 and Halo = halogen, preferably F; and/or
- at least one of A¹ and A² is substituted alkyl and the substituents of the alkyl are fluorine with the number n_{F} (of fluorine substituents) and n_{H} (of hydrogens) follow the equation: n_{F} > n_{H} + 2; and/or
- at least one of A¹ and A² is selected from perfluorinated C₁ to C₆ alkyl, phenyl substituted with F or CF₃; and/or
- at least one of A¹ and A² is selected from perfluorinated alkyl or aryl.

According to one embodiment of the organic electronic device, wherein the substituents on A¹ and A² are independently selected from halogen, preferably F, C₁ to C₃ perhalogenated, perfluorinated alkyl or perfluorinated alkoxy, preferably perfluorinated C₁ to C₃ alkyl or perfluorinated C₁ to C₃ alkoxy, or -(O)ₗ-CₘH₂ₘ-CₙHaloₙ₂ₙ₊₁ with l= 0 or 1, preferably 0, m = 1 or 2, preferably 1 and n = 1 to 3, preferably n =1 or 2 and Halo = halogen, preferably F.

According to one embodiment of the organic electronic device, wherein at least one of A¹ and A² is substituted alkyl and the substituents of the alkyl are fluorine with the number n_{F} (of fluorine substituents) and n_{H} (of hydrogens) follow the equation: n_{F} > n_{H} + 2.

According to one embodiment of the organic electronic device, wherein at least one of A¹ and A² is selected from perfluorinated C₁ to C₆ alkyl, phenyl substituted with F or CF.

According to one embodiment of the organic electronic device, wherein at least one of A¹ and A² is selected from perfluorinated alkyl or perfluorinated aryl, preferably perfluorinated C₁ to C₃ alkyl.

According to one embodiment of the organic electronic device, wherein at least one of A¹ and A² comprises a substituent, wherein at least one of the substituents of A¹ and A² are independently selected from C₃ to C₉ heteroaryl, C₁ to C₆ alkoxy, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkoxy, COR¹, COOR¹, halogen, F or CN; preferably at least one of A¹ and A² comprises at least two substituents, wherein the substituents on A¹ and A² are independently selected from C₃ to C₉ heteroaryl, C₁ to C₆ alkoxy, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkoxy, COR¹, COOR¹, halogen, F or CN; and further preferred A¹ and A² comprise at least one substituent independently selected from halogen, F, CF₃, C₂F₅, C₃F₇, C₄F₉, OCF₃, OC₂F₅, or CN; and in addition preferred A¹ and A² may comprise at least two substituents independently selected from halogen, F, CF₃, C₂F₅, C₃F₇, C₄F₉, OCF₃, OC₂F₅ or CN;
wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

According to one embodiment of the organic electronic device, wherein the sum of A¹ and A² comprise ≥ 3 carbon atoms and ≤ 30 carbon atoms.

Preferably the sum of A¹ and A² comprise ≥ 5 carbon atoms and ≤ 28 carbon atoms, further preferred the sum of A¹ and A² comprise ≥ 7 carbon atoms and ≤ 26 carbon atoms, in addition preferred the sum of A¹ and A² comprise ≥ 8 carbon atoms and ≤ 24 carbon atoms, also preferred the sum of A¹ and A² comprise ≥ 9 carbon atoms and ≤ 22 carbon atoms, or the sum of A¹ and A² comprise ≥ 10 carbon atoms and ≤ 20 carbon atoms, or the sum of A¹ and A² comprise ≥ 11 carbon atoms and ≤ 18 carbon atoms, or the sum of A¹ and A² comprise ≥ 12 carbon atoms and ≤ 16 carbon atoms, the sum of A¹ and A² comprise ≥ 13 carbon atoms and ≤ 15 carbon atoms.

According to one embodiment, wherein A² is aryl or heteroaryl, whereby the substituents of the aryl and/or heteroaryl moiety are selected from hydrogen, halogen, F, CN or trifluoro methyl.

According to one embodiment of the present invention A² is phenyl or six-membered heteroaryl, which is substituted with 1 to 5 F atoms.

According to one embodiment, wherein A¹ is substituted or unsubstituted C₁ to C₆ alkyl or substituted phenyl and A² is substituted C₃ to C₆ alkyl; alternatively, A¹ is substituted or unsubstituted C₁ to C₄ alkyl or substituted phenyl and A² is substituted C₃ to C₄ alkyl or substituted phenyl.

According to one embodiment, wherein A³ is selected from H or D, preferably H and A¹ and A² may be independently selected from substituted or unsubstituted C₁ to C₁₂ alkyl, substituted or unsubstituted C₆ to C₁₂ aryl, substituted or unsubstituted C₃ to C₁₂ heteroaryl; wherein at least one of A¹ and A² may comprises a substituent, wherein at least one of the substituents of A¹ and A² may be independently selected from C₃ to C₉ heteroaryl, C₁ to C₆ alkoxy, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkoxy, COR¹, COOR¹, halogen, F or CN; preferably at least one of A¹ and A² may comprises at least two substituents, wherein the substituents on A¹ and A² may be independently selected from C₃ to C₉ heteroaryl, C₁ to C₆ alkoxy, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkoxy, COR¹, COOR¹, halogen, F or CN; and further preferred A¹ and A² comprise at least one substituent independently selected from halogen, F, CF₃, C₂F₅, C₃F₇, C₄F₉, OCF₃, OC₂F₅, or CN; and in addition preferred A¹ and A² may comprise at least two substituents independently selected from halogen, F, CF₃, C₂F₅, C₃F₇, C₄F₉, OCF₃, OC₂F₅ or CN.

According to one embodiment, wherein the compound of formula (I) is free of alkoxy, COR¹ and/or COOR¹ groups, wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

According to one embodiment, wherein A³ is selected form a substituted or unsubstituted cyclic structure with either A¹ or A² of 5- or 6- membered aliphatic or aromatic rings, whereby the ring optional comprise one or more heteroatoms, preferably selected from the group comprising N, O and S, wherein the one or more substituent of the substituted cyclic structure are independently selected from halogen, F, Cl, CN, CF₃.

According to one embodiment, wherein A¹ and/or A² are selected from the following Formulas D1 to D68: 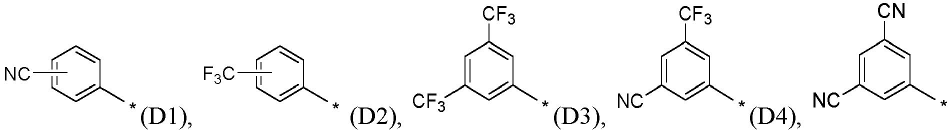 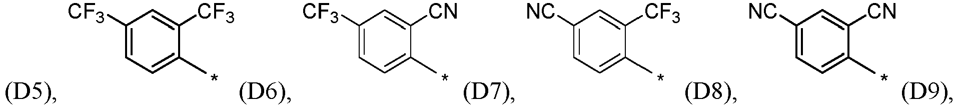 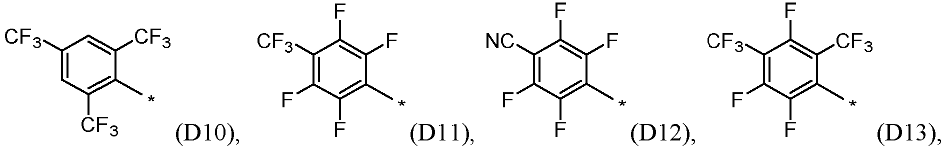 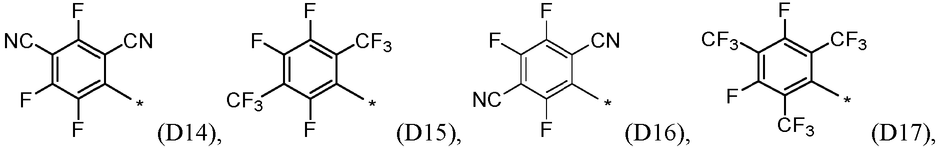 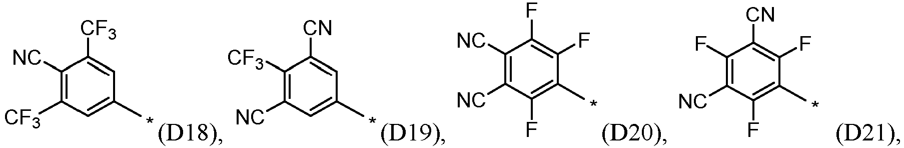 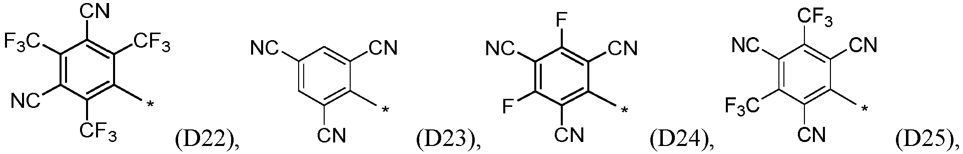 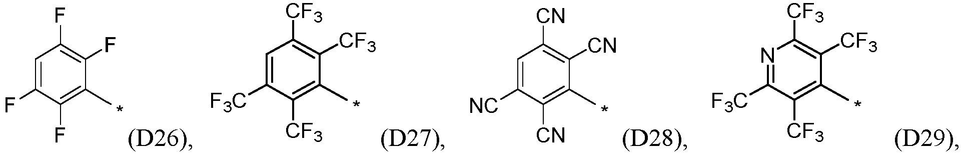 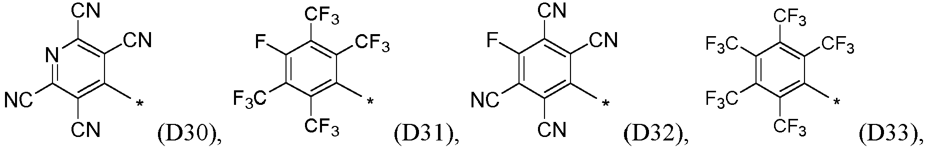 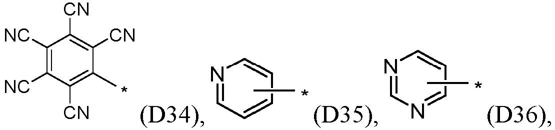 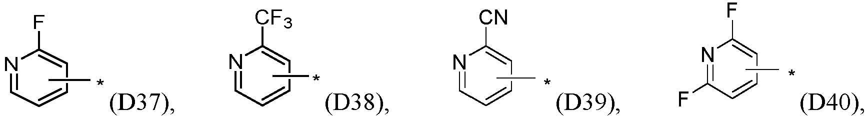 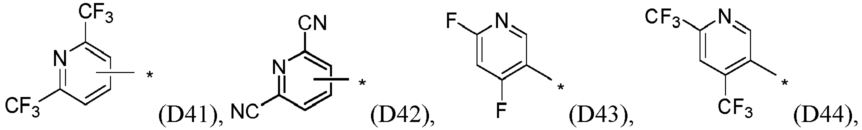 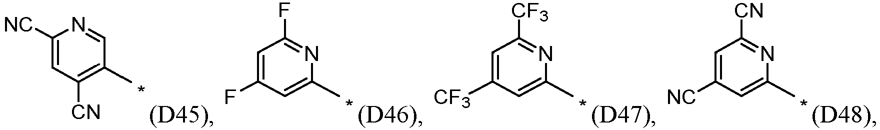 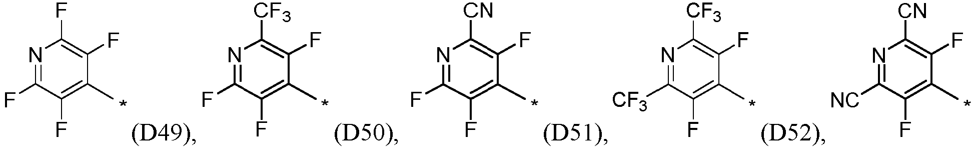 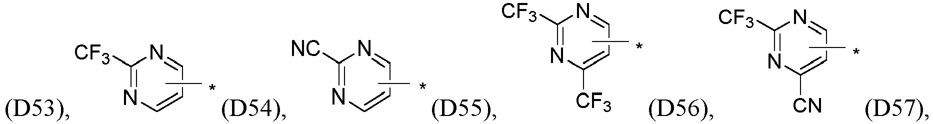 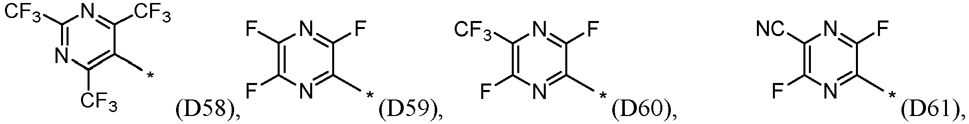 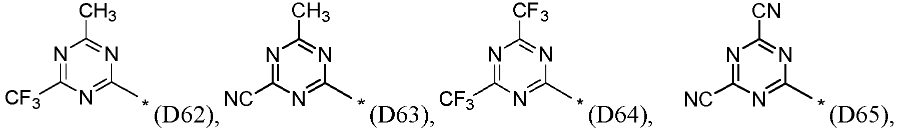 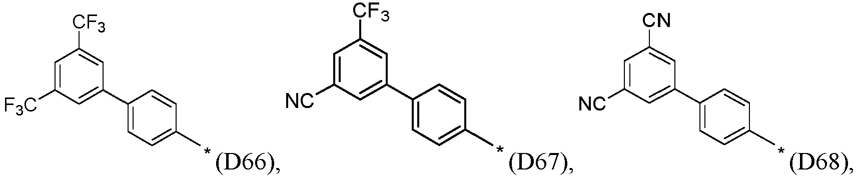 wherein the "*" denotes the binding position.

According to one embodiment, wherein L the ligand of Formula (II) may be selected such, wherein A¹ is selected from formula D1 to D68 and A² is selected from substituted or unsubstituted C₁ to C₁₂ alkyl, preferably substituted C₁ to C₁₂ alkyl and A³ is selected from H, D, CN, substituted or unsubstituted C₁ to C₄ alkyl, partially or perfluorinated C₁ to C₄ alkyl, or whereby A³ form a substituted or unsubstituted cyclic structure with either A¹ or A², wherein the one or more substituent of the substituted cyclic structure are independently selected from halogen, F, Cl, CN, CF₃.

According to one embodiment, wherein L the ligand of Formula (II) may be selected such, wherein A¹ is selected from formula D1 to D68 and A² is selected from substituted or unsubstituted C₁ to C₁₂ alkyl, preferably substituted C₁ to C₁₂ alkyl and A³ is selected form a cyclic structure with either A¹ or A² of 5- or 6- membered aliphatic or aromatic rings, whereby the ring optional comprise one or more heteroatoms, preferably selected from the group comprising N, O and S.

According to one embodiment, wherein A¹ and A² may be selected different. According to one embodiment, wherein A¹ and A² may be selected the same.

### Compound of formula (I)

The compound of formula (I) is non-emissive. In the context of the present specification the term "essentially non-emissive" or "non-emissive" means that the contribution of the compound according to formula (I) to the visible emission spectrum from an organic electronic device, such as OLED or display device, is less than 10 %, preferably less than 5 % relative to the visible emission spectrum. The visible emission spectrum is an emission spectrum with a wavelength of about ≥ 380 nm to about ≤ 780 nm.

According to one embodiment, the compound of formula (I) may have a molecular weight Mw of ≥ 287 g/mol and ≤ 2000 g/mol, preferably a molecular weight Mw of ≥ 300 g/mol and ≤ 2000 g/mol.

According to one embodiment, the compound of formula (I) is a metal complex.

According to one embodiment of the organic electronic device, wherein the LUMO energy of the compound of formula (I) may be selected ≤ -4.6 eV and ≥ 6 eV, preferably ≤ -4.65 eV and ≥ 6 eV, also preferred ≤ -4.7 eV and ≥ 6 eV; wherein the LUMO energy is calculated as single point energy calculation with the program package TURBOMOLE V6.5 (TURBOMOLE GmbH, Litzenhardtstrasse 19, 76135 Karlsruhe, Germany) from the optimized geometry by applying the hybrid functional B3LYP with a Def2-TZVP basis set in the gas phase and inclusion of dispersion forces as D3BJ. Geometry is optimized by applying the hybrid functional B3LYP with a Def2-SVP basis set in the gas phase and inclusion of dispersion forces as D3BJ. If more than one conformation is viable, the conformation with the lowest total energy is selected.

### M of the compound of formula (I)

According to one embodiment, wherein the valency n of M of the compounds of formula (I) is 1, 2 or 3, preferably 1 or 2, or further preferred 3.

According to one embodiment, wherein M of the compounds of formula (I) may be selected from a metal ion wherein the corresponding metal has an electronegativity value according to Allen of less than 2.4.

The term "electronegativity value according to Allen" especially refers to Allen, Leland C. (1989). "Electronegativity is the average one-electron energy of the valence-shell electrons in ground-state free atoms", Journal of the American Chemical Society 111 (25): 9003-9014.

According to one embodiment, wherein M is a is a metal ion and selected from the group comprising an alkali, alkaline earth, transition metal, rare earth metal or group III metal, early transition metal, period 4 transition metal, and preferably selected from the group comprising Li, Na, K, Rb or Cs, Mg, Ca, Sr or Ba, Sc, Y, La, Ti, Zr, V, Nb, Ta, Cr, Mo, W, Mn, Re, Fe, Ru, Co, Ni, Cu, Ag, Au, Zn, Al, Fe, Cr, Mn, Ru or Cu, further preferred M is selected from Fe or Cu, and more preferred Fe.

Preferably M may be selected from an alkali, alkaline earth, transition metal, rare earth metal or group III metal, preferably early transition metal, also preferred period 4 transition metal.

The alkali metals may be selected from the group comprising Li, Na, K, Rb or Cs. The alkaline earth metals may be selected from the group comprising Mg, Ca, Sr or Ba. The transition metals may be selected from Sc, Y, La, Ti, Zr, V, Nb, Ta, Cr, Mo, W, Mn, Re, Fe, Ru, Co, Ni, Cu, Ag, Au or Zn. The group III metal may be selected from Al.

According to one embodiment, wherein M is selected from Fe, Cr, Mn, Ru or Cu; preferably M is selected from Fe or Cu, also preferred Fe.

According to one embodiment, wherein M is not Bi or Ce.

### Ligand L

Preferably, L comprises at least four fluorine atoms and less than 60 fluorine atoms, preferably at least six fluorine atoms and less than 40 fluorine atoms, also preferred at least six fluorine atoms and less than 30 fluorine atoms.

The negative charge in ligand L of formula (I), may be delocalised partially or fully over the C(CO₂)₂ group and optionally also over the A¹, A² and A³ groups.

According to one embodiment, wherein the ligand L of formula (I) comprising per ligand L at least 14 covalently bound atoms; or
the ligand L of formula (I) comprising:
- at least three carbon atoms, alternatively at least four carbon atoms, and/or
- at least two oxygen atoms or one oxygen and one nitrogen atom, two to four oxygen atoms, two to four oxygen atoms and zero to two nitrogen atoms, and/or
- at least one or more groups selected from halogen, F, CN, substituted or unsubstituted C₁ to C₆ alkyl, substituted or unsubstituted C₁ to C₆ alkoxy, alternatively two or more groups selected from halogen, F, CN, substituted or unsubstituted C₁ to C₆ alkyl, substituted or unsubstituted C₁ to C₆ alkoxy, at least one or more groups selected from halogen, F, CN, substituted C₁ to C₆ alkyl, substituted C₁ to C₆ alkoxy, alternatively two or more groups selected from halogen, F, CN, perfluorinated C₁ to C₆ alkyl, perfluorinated C₁ to C₆ alkoxy, one or more groups selected from substituted or unsubstituted C₁ to C₆ alkyl, substituted or unsubstituted C₆ to C₁₂ aryl, and/or substituted or unsubstituted C₃ to C₁₂ heteroaryl,
   wherein the substituents are selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR³, COOR³, halogen, F or CN;
   wherein R³ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

According to one embodiment, wherein the ligand L of formula (I) is selected from G1 to G73:

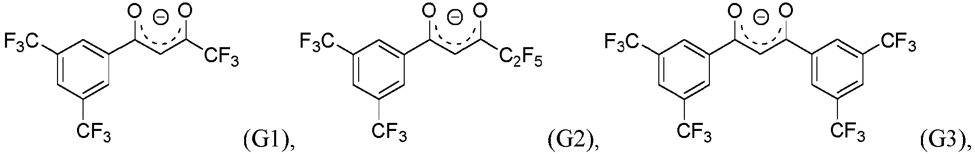

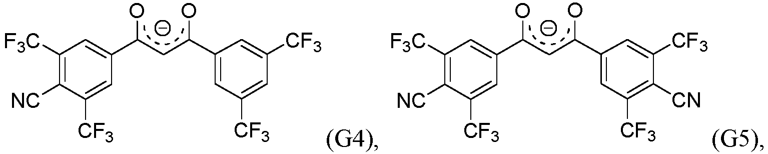

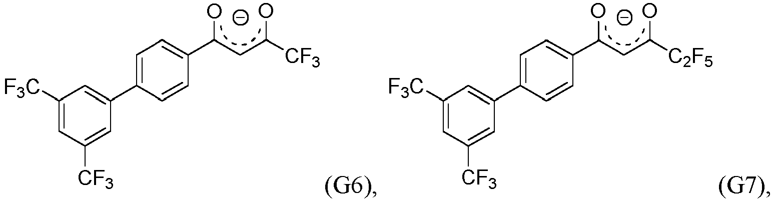

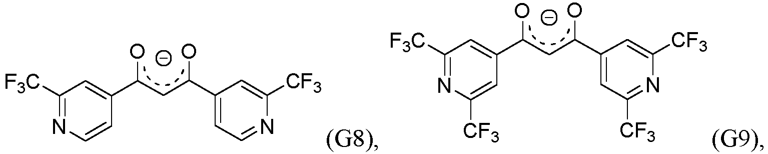

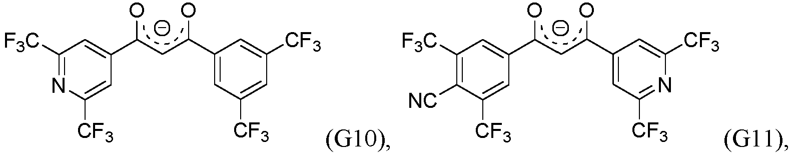

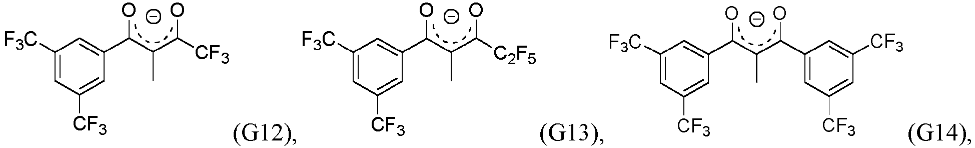

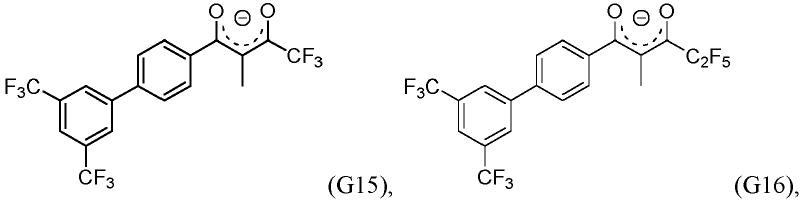

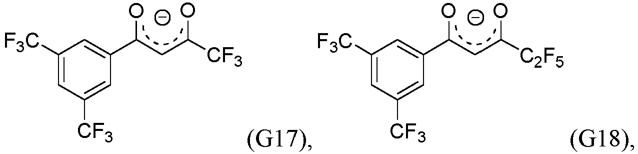

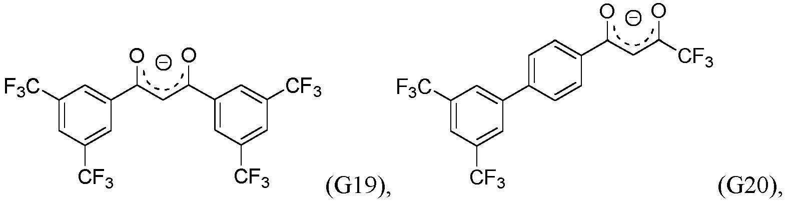

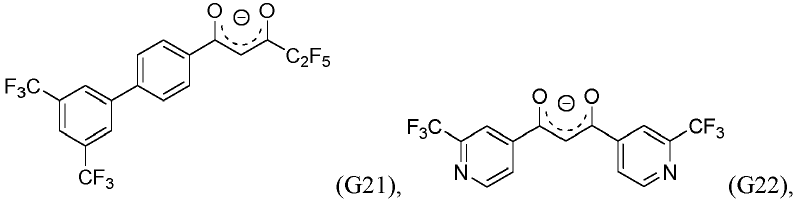

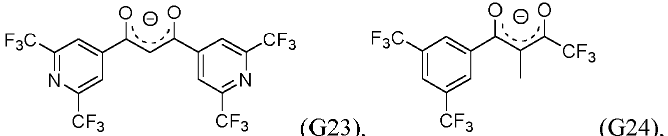

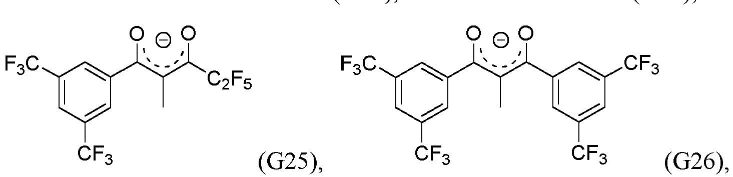

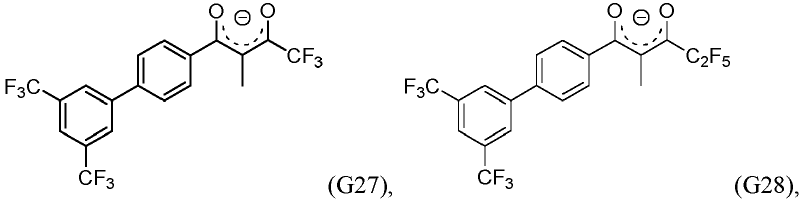

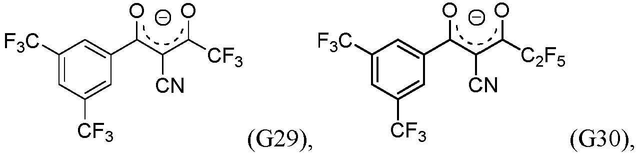

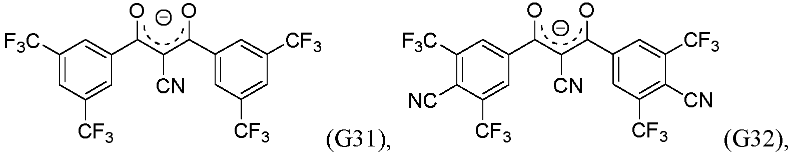

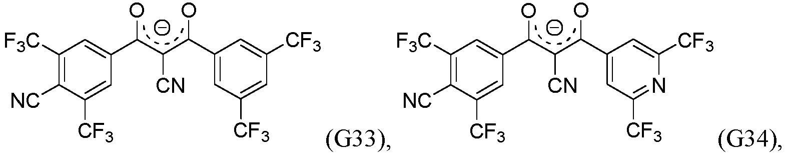

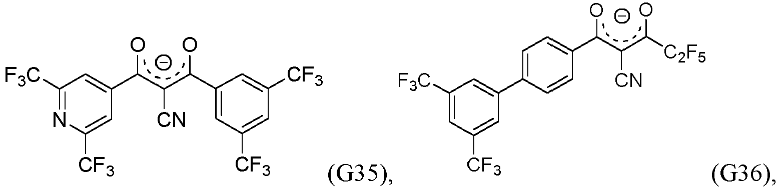

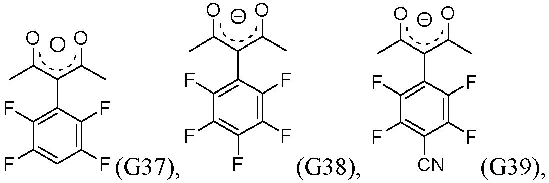

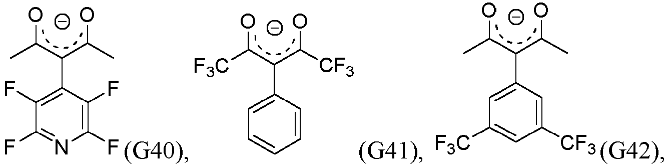

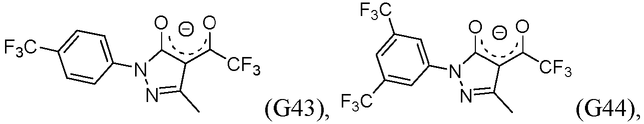

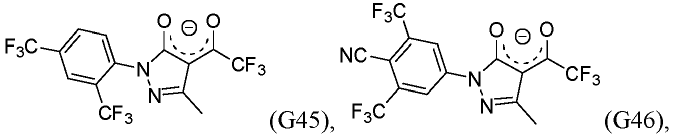

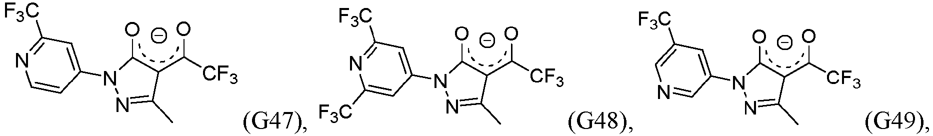

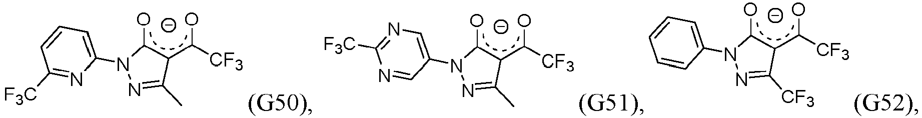

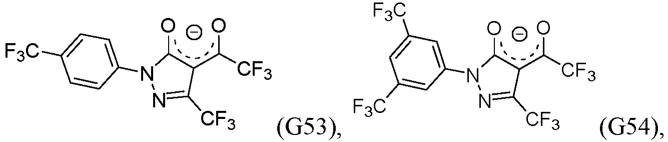

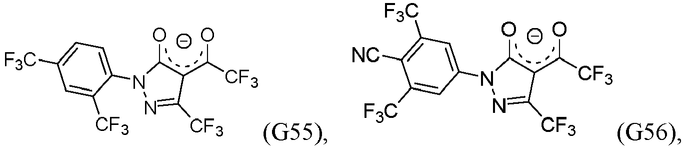

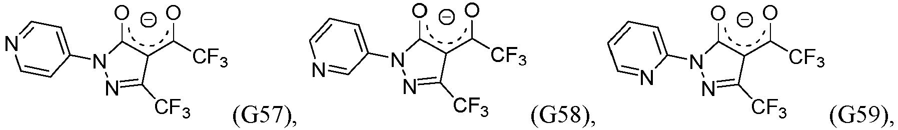

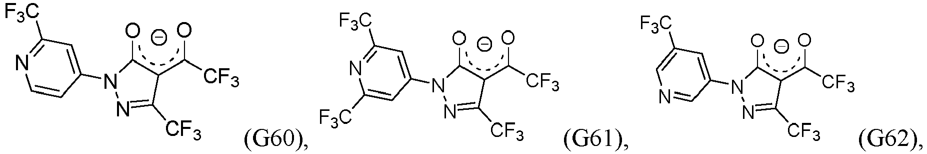

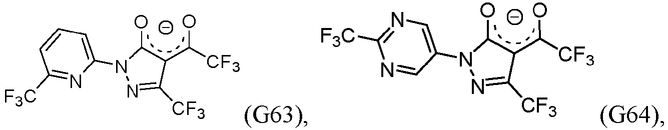

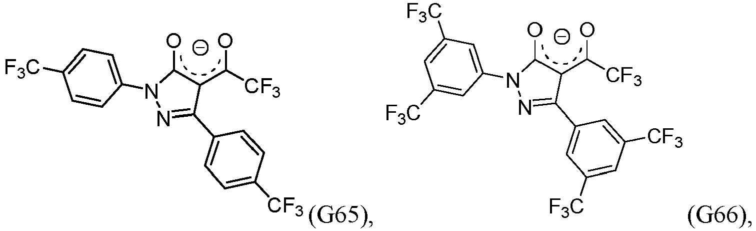

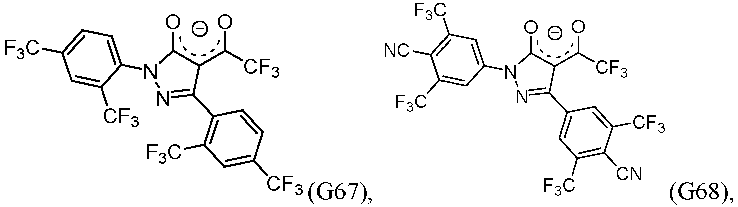

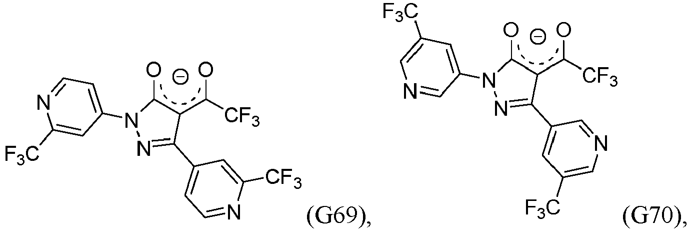

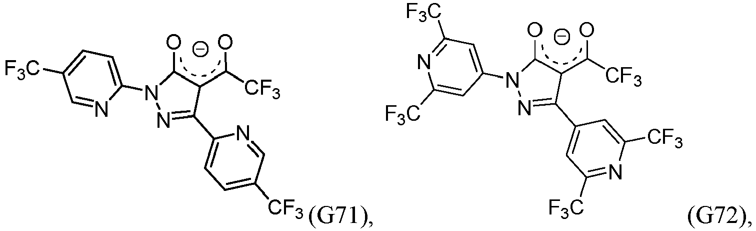

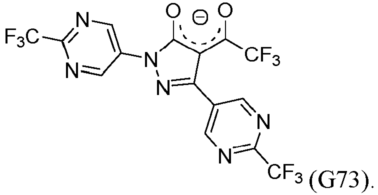

### Ancillary ligand AL

The Ancillary ligands may be defined as those ligands that provide the appropriate steric and electronic environment around the central element but remain innocent in any transformation that the compound undergoes. In contrast, reactive ligands are those groups that undergo changes.

According to one embodiment of the organic electronic device, wherein the ancillary ligand AL is from the group comprising H₂0, C₂ to C₄₀ mono- or multi-dentate ethers and C₂ to C₄₀ thioethers, C₂ to C₄₀ amines, C₂ to C₄₀ phosphine, C₂ to C₂₀ alkyl nitrile or C₂ to C₄₀ aryl nitrile, or a compound according to Formula (AL-I); 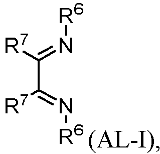 wherein
R⁶ and R⁷ are independently selected from C₁ to C₂₀ alkyl, C₁ to C₂₀ heteroalkyl, C₆ to C₂₀ aryl, heteroaryl with 5 to 20 ring-forming atoms, halogenated or perhalogenated C₁ to C₂₀ alkyl, halogenated or perhalogenated C₁ to C₂₀ heteroalkyl, halogenated or perhalogenated C₆ to C₂₀ aryl, halogenated or perhalogenated heteroaryl with 5 to 20 ring-forming atoms, or at least one R⁶ and R⁷ are bridged and form a 5 to 20 member ring, or the two R⁶ and/or the two R⁷ are bridged and form a 5 to 40 member ring or form a 5 to 40 member ring comprising an unsubstituted or C₁ to C₁₂ substituted phenanthroline.

### Compound of formula (I)

According to one embodiment of the organic electronic device, wherein the compound of formula (I) is selected from the group of MC-1 to MC-17:

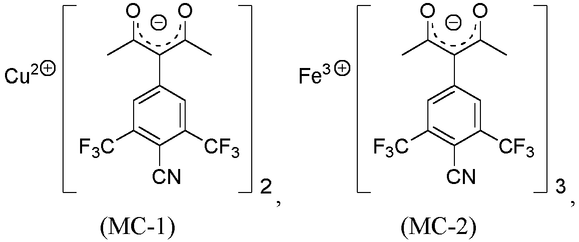

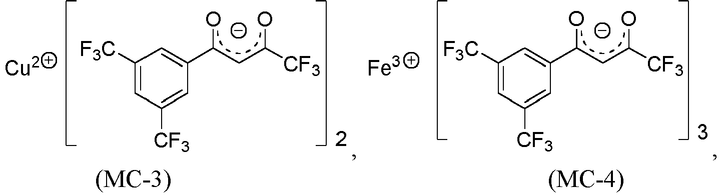

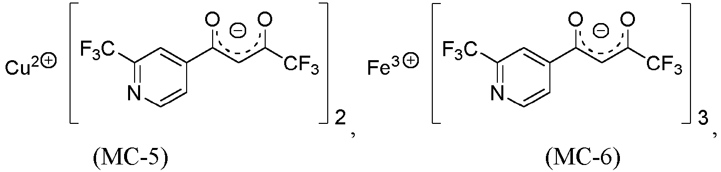

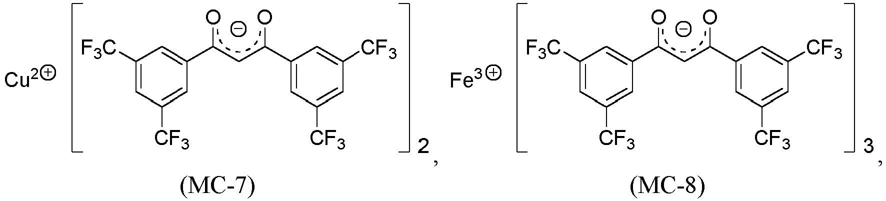

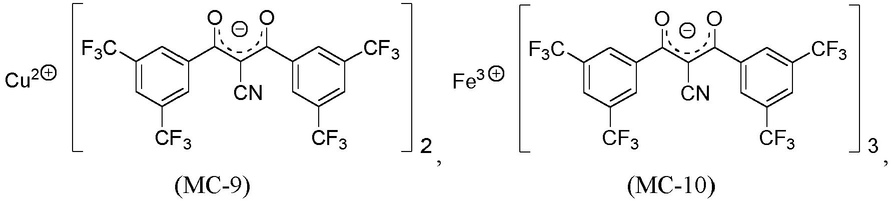

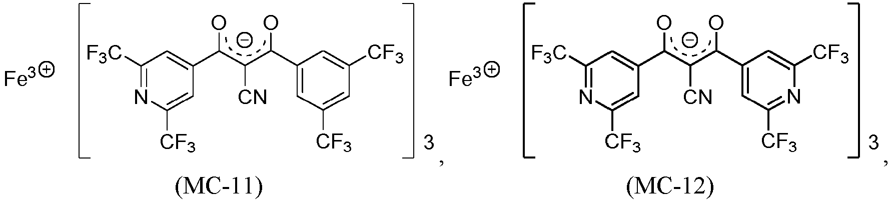

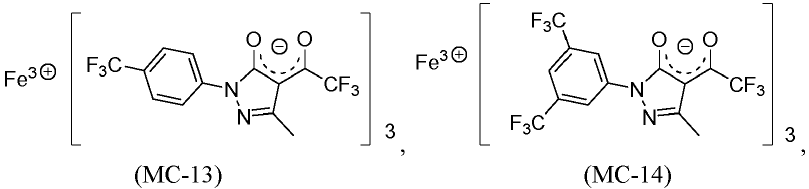

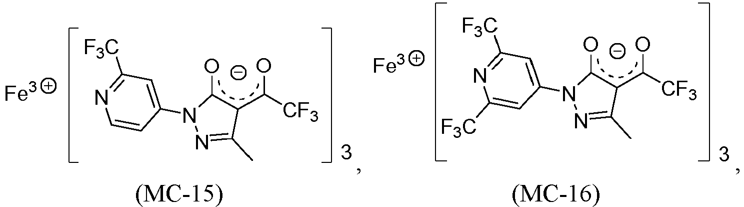

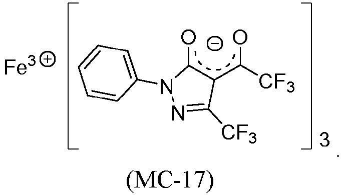

According to one embodiment of the organic electronic device, wherein the compound of formula (I) is preferably selected from MC-10, MC-11 or MC-12.

When the compound of formula (I) is selected from the compounds of MC-1 to MC-17, preferably MC-10, MC-11 and MC-12, particularly efficient hole injection from the p-CGL into the adjacent hole transport layer may be achieved. Additionally, the compounds of formula (I) may have improved thermal properties and a rate onset temperature in the range suitable for mass production of organic electronic devices. Furthermore, the compounds of formula (I) may potentially have reduced health and safety risks compared to the art.

Therefore, compounds of formula (I) may be particularly suitable for mass production of organic electronic devices via vacuum thermal evaporation.

### Compound of formula (I)

According to a further aspect of the invention there is provided a compound of formula (I):

M^{n⊕}(L^{⊖})ₙ (AL)ₘ (I),

wherein
- M: is Fe³⁺;
- n: is 3;
- m: is an integer selected from 0 to 2, preferably m is 0;
- AL: is an ancillary ligand which coordinates to the metal M;
- L: is a ligand of formula G35:

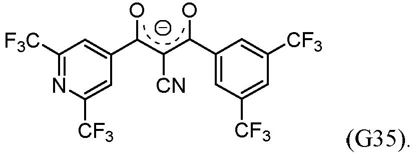

### Organic hole transport compound

According to one embodiment, the p-type charge generation layer comprises an organic hole transport compound, also referred to as "covalent organic matrix compound", "organic matrix compound" or "substantially covalent organic matrix compound", preferably the organic hole transport compound is a covalent organic matrix compound.

Preferred examples of the organic hole transport compounds are organic compounds comprising substantially of covalently bound C, H, O, N, S, which may optionally comprise also covalently bound B, P or Si.

In one embodiment, the organic hole transport compound is free of metal atoms and majority of its skeletal atoms may be selected from C, O, S, N. Alternatively, the organic hole transport compound is free of metal atoms and majority of its skeletal atoms may be selected from C and N.

According to one embodiment, the organic hole transport compound of the p-type charge generation layer may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

In one embodiment, the HOMO energy of the organic hole transport compound may be more negative than the HOMO energy of N2,N2,N2',N2',N7,N7,N7',N7'-octakis(4-methoxyphenyl)-9,9'-spirobi[fluorene]-2,2',7,7'-tetraamine (CAS 207739-72-8) when determined under the same conditions.

According to one embodiment, wherein the HOMO energy of the organic hole transport compound of the p-type charge generation layer may be calculated by quantum mechanical methods.

In one embodiment, wherein the organic hole transport compound may be free of alkoxy groups.

Preferably, the organic hole transport compound comprises at least one arylamine moiety, alternatively a diarylamine moiety, alternatively a triarylamine moiety.

Preferably, the organic hole transport compound of the p-type charge generation layer is free of metals and/or ionic bonds.

### Compound of formula (IIIa) or a compound of formula (IIIb)

According to one embodiment, wherein the organic hole transport compound of the p-type charge generation layer is selected from the group comprising arylamine compound, diarylamine compound, triarylamine compound, a compound of formula (IIIa) or a compound of formula (IIIb).

According to another aspect, wherein the organic hole transport compound of the p-type charge generation layer, or the p-type charge generation layer and the hole transport layer may comprises at least one arylamine compound, diarylamine compound, triarylamine compound, a compound of formula (IIIa) or a compound of formula (IIIb): 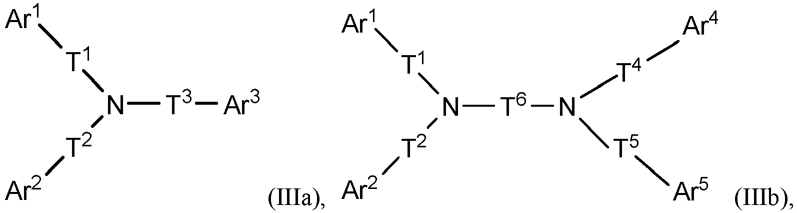 wherein:
- T¹, T², T³, T⁴ and T⁵: are independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

According to an embodiment of the organic electronic device, wherein the organic hole transport compound of the p-type charge generation layer, or the p-type charge generation layer and the hole transport layer may comprises a compound of formula (IIIa) or formula (IIIb): 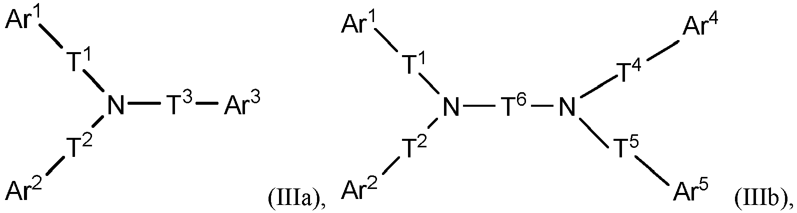 wherein
- T¹, T², T³, T⁴ and T⁵: may be independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴and Ar⁵: may be independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein the substituents of Ar¹, Ar², Ar³, Ar⁴ and Ar⁵ are selected the same or different from the group comprising H, straight-chain alkyl having 1 to 20 carbon atoms, branched alkyl having 1 to 20 carbon atoms, cyclic alkyl having 3 to 20 carbon atoms, alkenyl or alkynyl groups having 2 to 20 carbon atoms, alkoxy groups having 1 to 20 carbon atoms, C₆ to C₁₈ aryl, C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle.

Preferably, the substituents of Ar¹, Ar², Ar³, Ar⁴ and Ar⁵ are selected the same or different from the group comprising H, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, alkoxy groups having 1 to 6 carbon atoms, C₆ to C₁₈ aryl, C₃ to C₁₈ heteroaryl, a fused ring system comprising 2 to 4 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle; more preferred the substituents are selected the same or different from the group comprising of H, straight-chain alkyl having 1 to 4 carbon atoms, branched alkyl having 1 to 4 carbon atoms, cyclic alkyl having 3 to 4 carbon atoms and/or phenyl.

Thereby, the compound of formula (IIIa) or (IIIb) may have a rate onset temperature suitable for mass production.

According to an embodiment of the organic electronic device, wherein the organic hole transport compound of the p-type charge generation layer, or the p-type charge generation layer and the hole transport layer may comprises a compound of formula (IIIa) or formula (IIIb): 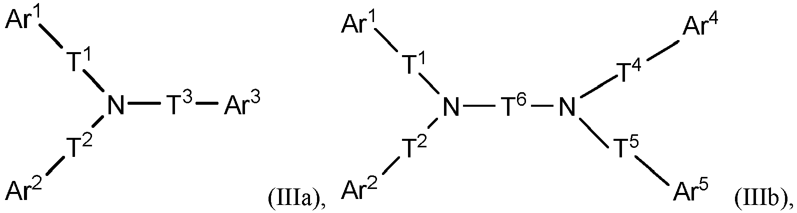 wherein
- T¹, T², T³, T⁴ and T⁵: may be independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene], or a unsubstituted aromatic fused ring system comprising at least three unsubstituted aromatic rings selected from the group comprising unsubstituted non-hetero, unsubstituted hetero 5-member rings, unsubstituted 6-member rings and/or unsubstituted 7-member rings, unsubstituted fluorene, or a fused ring system comprising 2 to 6 unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle.

According to an embodiment of the organic electronic device, wherein the organic hole transport compound of the p-type charge generation layer and/or hole transport layer comprises a compound of formula (IIIa) or formula (IIIb): 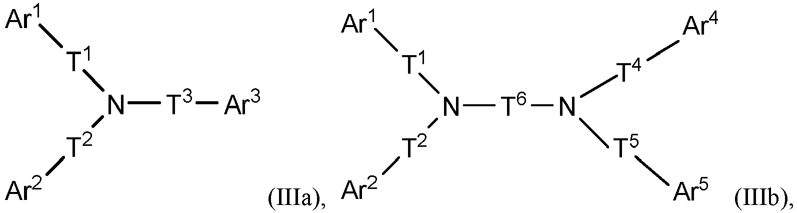 wherein
- T¹, T², T³, T⁴ and T⁵: may be independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene].

Thereby, the compound of formula (IIIa) or (IIIb) may have a rate onset temperature suitable for mass production.

According to an embodiment wherein T¹, T², T³, T⁴ and T⁵ may be independently selected from a single bond, phenylene, biphenylene or terphenylene. According to an embodiment wherein T¹, T², T³, T⁴ and T⁵ may be independently selected from phenylene, biphenylene or terphenylene and one of T¹, T², T³, T⁴ and T⁵ are a single bond. According to an embodiment wherein T¹, T², T³, T⁴ and T⁵ may be independently selected from phenylene or biphenylene and one of T¹, T², T³, T⁴ and T⁵ are a single bond. According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond. According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene. According to an embodiment wherein T⁶ may be biphenylene. According to an embodiment wherein T⁶ may be terphenylene.

According to an embodiment wherein Ar¹, Ar², Ar³, Ar⁴ and Ar⁵ may be independently selected from K1 to K16: 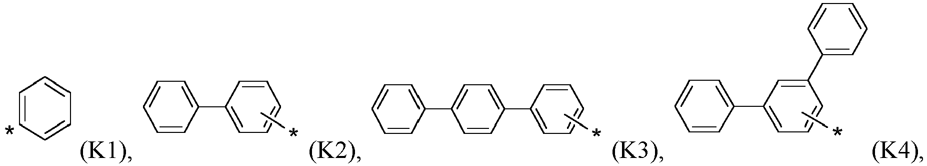 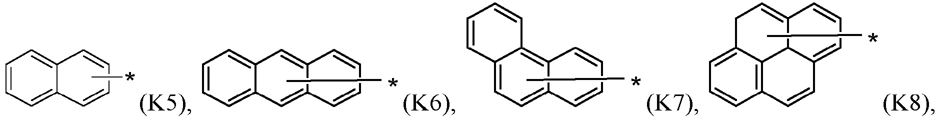 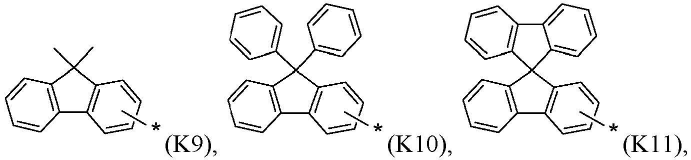 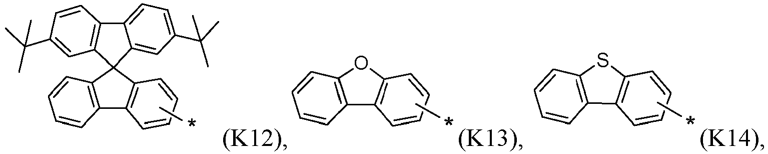 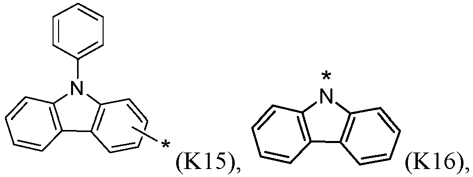 wherein the asterix "*" denotes the binding position.

According to an embodiment, wherein Ar¹, Ar², Ar³, Ar⁴ and Ar⁵ may be independently selected from K1 to K15; alternatively selected from K1 to K10 and K13 to K15.

According to an embodiment, wherein Ar¹, Ar², Ar³, Ar⁴ and Ar⁵ may be independently selected from the group comprising of K1, K2, K5, K7, K9, K10, K13 to K16.

The rate onset temperature may be in a range particularly suited to mass production, when Ar¹, Ar², Ar³, Ar⁴ and Ar⁵ are selected in this range.

According to one embodiment the organic hole transport compound of formula (IIIa) or formula (IIIb) may comprises at least ≥ 1 to ≤ 6 substituted or unsubstituted aromatic fused ring systems comprising heteroaromatic rings.

According to one embodiment the organic hole transport compound of formula (IIIa) or formula (IIIb) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings.

According to one embodiment the organic hole transport compound of formula (IIIa) or formula (IIIb) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings, and at least ≥ 1 to ≤ 3 substituted or unsubstituted unsaturated 5- to 7-member ring of a heterocycle, further preferred 3 or 4 substituted or unsubstituted aromatic fused ring systems comprising heteroaromatic rings and optional at least ≥ 1 to ≤ 3 substituted or unsubstituted unsaturated 5- to 7-member ring of a heterocycle, and additional preferred wherein the aromatic fused ring systems comprising heteroaromatic rings are unsubstituted and optional at least ≥ 1 to ≤ 3 unsubstituted unsaturated 5- to 7-member ring of a heterocycle.

According to one embodiment the organic hole transport compound of formula (IIIa) or formula (IIIb) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems.

According to one embodiment the organic hole transport compound of formula (IIIa) or formula (IIIb) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings.

According to one embodiment the organic hole transport compound of formula (IIIa) or formula (IIIb) may comprises at least ≥ 1 to ≤ 3 or 2 substituted or unsubstituted unsaturated 5- to 7-member ring of a heterocycle.

According to one embodiment the organic hole transport compound of formula (IIIa) or formula (IIIb) may comprises at least ≥ 1 to ≤ 3 or 2 substituted or unsubstituted unsaturated 7-member ring of a heterocycle.

According to one embodiment substituted or unsubstituted aromatic fused ring systems of the organic hole transport compound of formula (IIIa) or formula (IIIb) may comprises at least ≥ 1 to ≤ 3 or 2 substituted or unsubstituted unsaturated 5- to 7-member ring of a heterocycle.

According to one embodiment the substituted or unsubstituted aromatic fused ring systems of the organic hole transport compound of formula (IIIa) or formula (IIIb) may comprises at least ≥ 1 to ≤ 3 or 2 substituted or unsubstituted unsaturated 7-member ring of a heterocycle.

According to one embodiment the organic hole transport compound of formula (IIIa) or formula (IIIb) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, and wherein the aromatic fused ring system comprises substituted or unsubstituted unsaturated 5- to 7-member ring of a heterocycle.

According to one embodiment the organic hole transport compound of formula (IIIa) or formula (IIIb) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings, and wherein the aromatic fused ring system comprises substituted or unsubstituted unsaturated 5- to 7-member ring of a heterocycle.

According to one embodiment the organic hole transport compound of formula (IIIa) or formula (IIIb) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, and wherein the aromatic fused ring system comprises at least ≥ 1 to ≤ 3 or 2 substituted or unsubstituted unsaturated 5- to 7-member ring of a heterocycle.

According to one embodiment the organic hole transport compound of formula (IIIa) or formula (IIIb) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings, and wherein the aromatic fused ring system comprises at least ≥ 1 to ≤ 3 or 2 substituted or unsubstituted unsaturated 5- to 7-member ring of a heterocycle.

According to one embodiment the organic hole transport compound of formula (IIIa) or formula (IIIb) may comprises:
- a substituted or unsubstituted aromatic fused ring systems with at least ≥ 2 to ≤ 6, preferably ≥ 3 to ≤ 5, or 4 fused aromatic rings selected from the group comprising substituted or unsubstituted non-hetero aromatic rings, substituted or unsubstituted hetero 5-member rings, substituted or unsubstituted 6-member rings and/or substituted or unsubstituted unsaturated 5-to 7- member ring of a heterocycle; or
- an unsubstituted aromatic fused ring systems with at least ≥ 2 to ≤ 6, preferably ≥ 3 to ≤ 5, or 4 fused aromatic rings selected from the group comprising unsubstituted non-hetero aromatic rings, unsubstituted hetero 5-member rings, unsubstituted 6-member rings and/or unsubstituted unsaturated 5- to 7-member ring of a heterocycle.

It should be noted here that the wording "aromatic fused ring system" may include at least one aromatic ring and at least one substituted or unsubstituted unsaturated 5- to 7- member ring. It should be noted here that the substituted or unsubstituted unsaturated 5- to 7- member ring may not be an aromatic ring.

According to one embodiment the organic hole transport compound of formula (IIIa) or formula (IIIb) may comprises at least at least ≥ 1 to ≤ 6, preferably ≥ 2 to ≤ 5, or further preferred 3 or 4 of the substituted or unsubstituted aromatic fused ring systems with:
- at least one unsaturated 5-member ring, and/or
- at least one unsaturated 6-member ring, and/or
- at least one unsaturated 7-member ring; wherein preferably at least one unsaturated 5- and/or at least one unsaturated 7-member ring comprises at least 1 to 3, preferably 1 hetero-atom.

According to one embodiment the organic hole transport compound of formula (IIIa) or formula (IIIb) may comprises at least at least ≥ 1 to ≤ 6, preferably ≥ 2 to ≤ 5, or further preferred 3 or 4 of the substituted or unsubstituted aromatic fused ring systems with:
- at least one aromatic 5-member ring, and/or
- at least one aromatic 6-member ring, and/or
- at least one aromatic 7-member ring; wherein preferably at least one aromatic 5- and/or at least one aromatic 7-member ring comprises at least 1 to 3, preferably 1 hetero-atom;
wherein the substituted or unsubstituted aromatic fused ring system comprises at least ≥ 1 to ≤ 3 or 2 substituted or unsubstituted unsaturated 5- to 7-member ring of a heterocycle.

According to one embodiment the organic hole transport compound of formula (IIIa) or formula (IIIb) may comprises :
- at least ≥ 6 to ≤ 12, preferably ≥ 7 to ≤ 11, further preferred ≥ 8 to ≤ 10 or 9 aromatic rings; and/or
- at least ≥ 4 to ≤ 11, preferably ≥ 5 to ≤ 10, further preferred ≥ 6 to ≤ 9 or in addition preferred 7 or 8 non-hetero aromatic rings, preferably the non-hetero aromatic rings are aromatic C₆ rings; and/or
- at least ≥ 1 to ≤ 4, preferably 2 or 3 aromatic 5-member-rings, preferably hetero aromatic 5-member-rings; and/or
- at least 1 or 2 unsaturated 5- to 7-member-ring of a heterocycle, preferably at least 1 or 2 unsaturated 7-member-ring of a heterocycle;
- at least ≥ 6 to ≤ 12, preferably ≥ 7 to ≤ 11, further preferred ≥ 8 to ≤ 10 or 9 aromatic rings, wherein therefrom
   at least ≥ 4 to ≤ 11, preferably ≥ 5 to ≤ 10, further preferred ≥ 6 to ≤ 9 or in addition preferred 7 or 8 are non-hetero aromatic rings, and
   at least ≥ 1 to ≤ 4, preferably 2 or 3 aromatic rings are hetero aromatic rings, wherein the total number of non-hetero aromatic rings and hetero aromatic rings in total does not exceed 12 aromatic rings; and/or
- at least ≥ 6 to ≤ 12, preferably ≥ 7 to ≤ 11, further preferred ≥ 8 to ≤ 10 or 9 aromatic rings, wherein therefrom
   at least ≥ 4 to ≤ 11, preferably ≥ 5 to ≤ 10, further preferred ≥ 6 to ≤ 9 or in addition preferred 7 or 8 are non-hetero aromatic rings, and
   at least ≥ 1 to ≤ 4, preferably 2 or 3 aromatic rings are hetero aromatic rings, wherein the total number of non-hetero aromatic rings and hetero aromatic rings in total does not exceed 12 aromatic rings; and
      the hole transport compound or the hole transport compound according to formula (I) comprises at least ≥ 1 to ≤ 4, preferably 2 or 3 aromatic 5-member-rings, preferably hetero aromatic 5-member-rings, and/or
      the hole transport compound or the hole transport compound according to formula (I) comprises at least 1 or 2 unsaturated 5- to 7-member-ring of a heterocycle, preferably at least 1 or 2 unsaturated 7-member-ring of a heterocycle.

According to one embodiment the organic hole transport compound of formula (IIIa) or formula (IIIb) may comprises a hetero-atom, which may be selected from the group comprising O, S, N, B or P, preferably the hetero-atom may be selected from the group comprising O, S or N.

According to one embodiment the organic hole transport compound of formula (IIIa) or formula (IIIb) may comprises at least at least ≥ 1 to ≤ 6, preferably ≥ 2 to ≤ 5, or further preferred 3 or 4 of the substituted or unsubstituted aromatic fused ring systems with:
- at least one aromatic 5-member ring, and/or
- at least one aromatic 6-member ring, and/or
- at least one aromatic 7-member ring; wherein preferably at least one aromatic 5- and/or at least one aromatic 7-member ring comprises at least 1 to 3, preferably 1 hetero-atom;
wherein the substituted or unsubstituted aromatic fused ring system optional comprises at least ≥ 1 to ≤ 3 or 2 substituted or unsubstituted unsaturated 5- to 7-member ring of a heterocycle; and wherein the substituted or unsubstituted aromatic fused ring system comprises a hetero-atom, which may be selected from the group comprising O, S, N, B, P or Si, preferably the hetero-atom may be selected from the group comprising O, S or N.

According to one embodiment the organic hole transport compound of formula (IIIa) or formula (IIIb) may be free of hetero-atoms which are not part of an aromatic ring and/or part of an unsaturated 7-member-ring, preferably the hole transport compound or the hole transport compound according to formula (I) may be free on N-atoms except N-atoms which are part of an aromatic ring or are part of an unsaturated 7-member-ring.

According to an embodiment of the electronic device, wherein the compound of formula (IIIa) or formula (IIIb) are selected from F1 to F20:

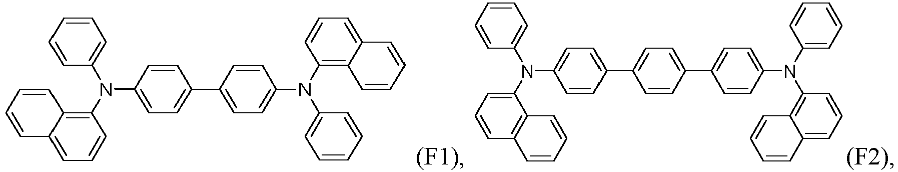

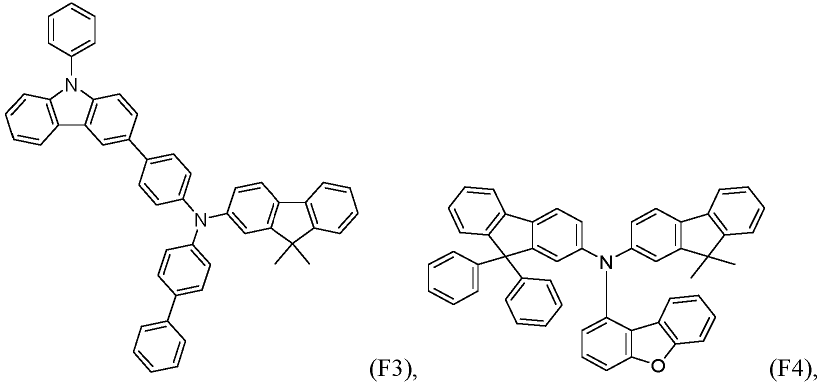

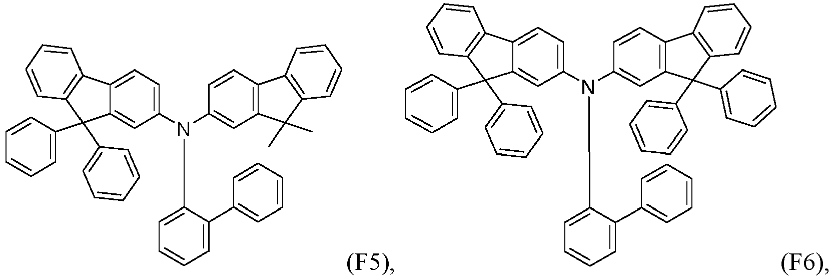

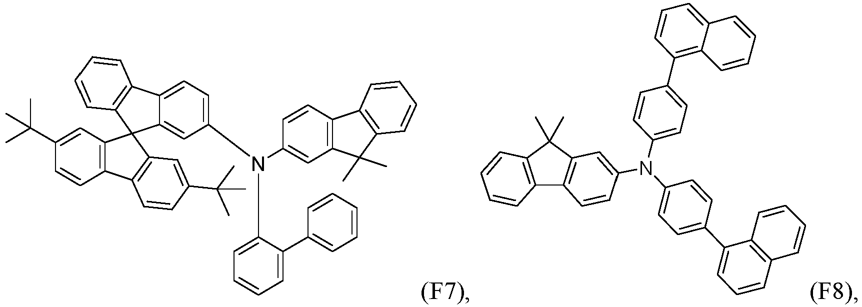

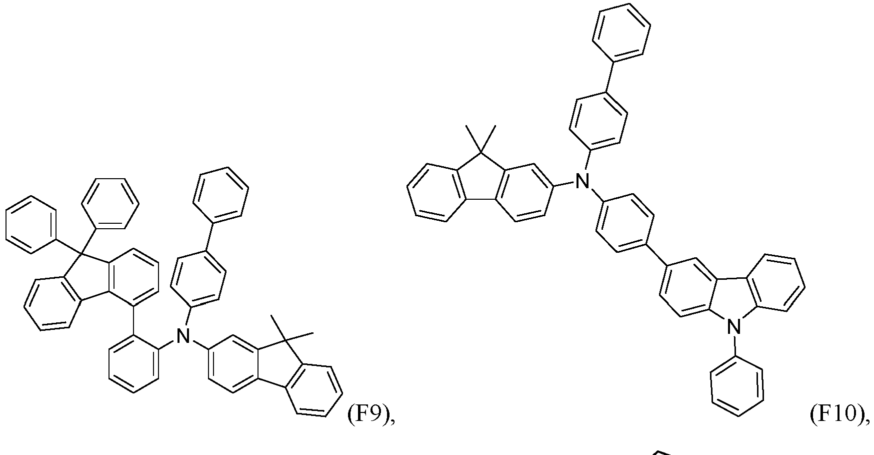

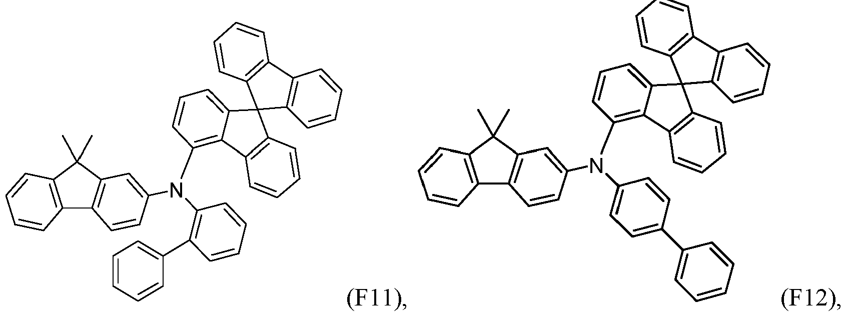

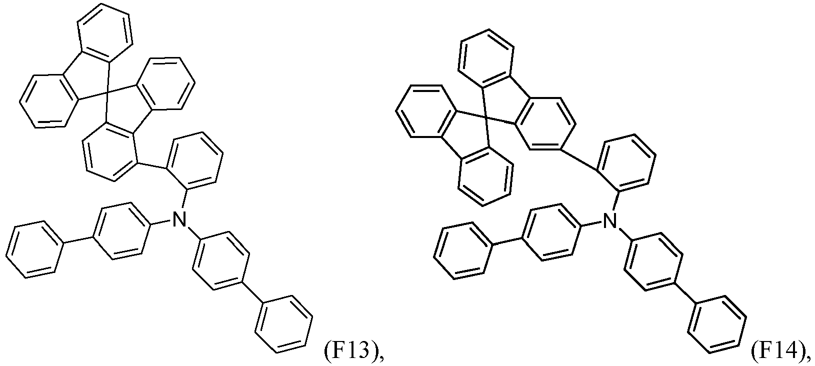

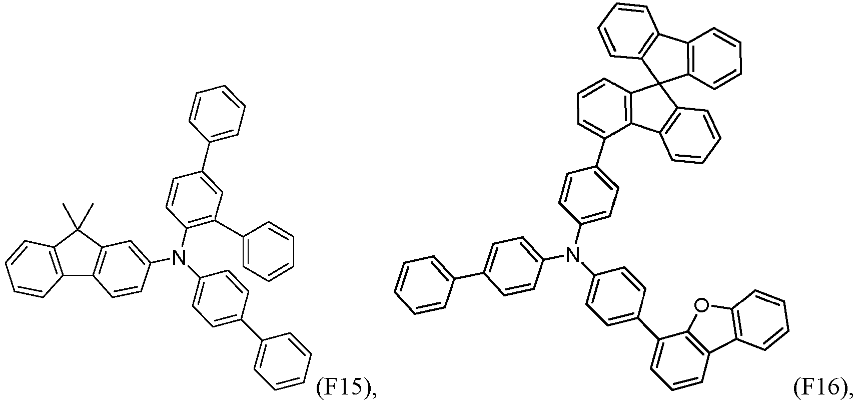

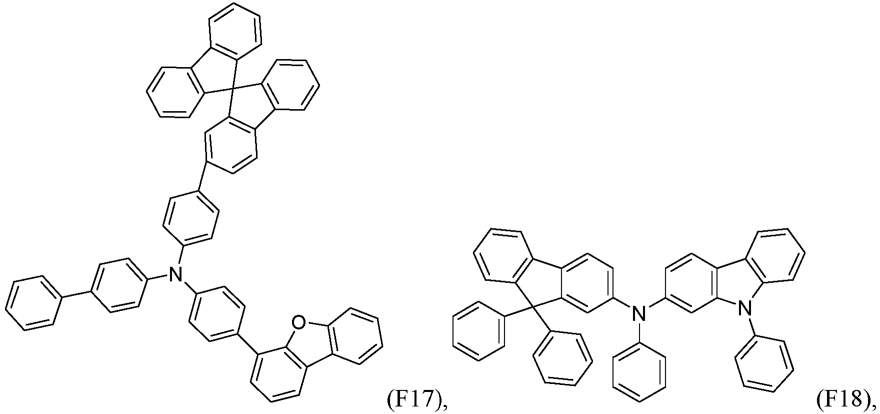

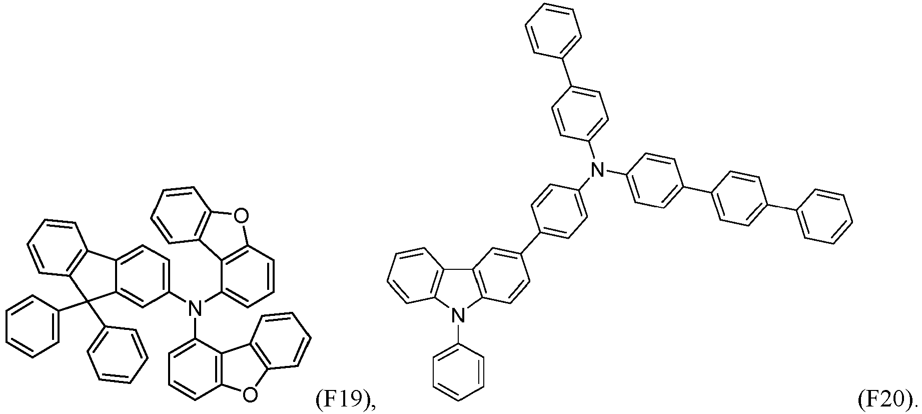

The organic hole transport compound may be free of HTM014, HTM081, HTM163, HTM222, EL-301, HTM226, HTM355, HTM133, HTM334, HTM604 and EL-22T. The abbreviations denote the manufacturers' names, for example, of Merck or Lumtec.

### p-type charge generation layer

The p-type charge generation layer may be formed on the n-type charge generation layer by vacuum deposition, spin coating, printing, casting, slot-die coating, Langmuir-Blodgett (LB) deposition, or the like. When the p-type charge generation layer is formed using vacuum deposition, the deposition conditions may vary according to the compound of formula (I) and/or hole transport compound that is used to form the p-type charge generation layer, and the desired structure and thermal properties of the p-type charge generation layer. In general, however, conditions for vacuum deposition may include a deposition temperature of 100° C to 350° C, a pressure of 10⁻⁸ to 10⁻³ Torr (1 Torr equals 133.322 Pa), and a deposition rate of 0.1 to 10 nm/sec.

When the p-type charge generation layer is formed using spin coating or printing, coating conditions may vary according to the hole transport compound that is used to form the p-type charge generation layer, and the desired structure and thermal properties of the p-type charge generation layer. For example, the coating conditions may include a coating speed of about 2000 rpm to about 5000 rpm, and a thermal treatment temperature of about 80° C to about 200° C. Thermal treatment removes a solvent after the coating is performed.

The p-type charge generation layer may be formed of a compound of formula (I) and an organic hole transport compound, wherein the organic hole transport compound is optionally a compound of formulae (IIIa) or (IIIb).

The thickness of the p-type charge generation layer may be in the range from about 5 nm to about 25 nm, and for example, from about 10 nm to about 15 nm.

When the thickness of the p-type charge generation layer is within this range, the p-type charge generation layer may have excellent hole generation characteristics, without a substantial penalty in operating voltage.

According to one embodiment, wherein the p-type charge generation layer may comprise:
- at least about ≥ 1 wt.-% to about ≤ 50 wt.-%, preferably about ≥ 5 wt.-% to about ≤ 40 wt.-%, and more preferred about ≥ 5 wt.-% to about ≤ 30 wt.-% of a compound of formula (I), and
- at least about ≥ 50 wt.-% to about ≤ 99 wt.-%, preferably about ≥ 60 wt.-% to about ≤ 95 wt.-%, and more preferred about ≥ 95 wt.-% to about ≤ 70 wt.-% of an organic hole transport compound, preferably an organic hole transport compound of formulae (IIIa) or (IIIb); preferably the wt.-% of the compound of formula (I) is lower than the wt.-% of the organic hole transport compound or an organic hole transport compound according to formulae (IIIa) or (IIIb); wherein the weight-% of the components are based on the total weight of the p-type charge generation layer.

Preferably, the p-type charge generation layer is free of ionic liquids, metal fluoride and/or metal oxides, wherein the metal in the metal oxide is selected from Re and/or Mo. Thereby, the p-type charge generation layer can be deposited under conditions suitable for mass production.

According to an embodiment of the organic electronic device, wherein the p-type charge generation layer is non-emissive.

It is to be understood that the p-type charge generation layer is not part of the n-type charge generation layer or hole transport layer.

### Organic electron transport compound

According to an embodiment, wherein the organic electron transport compound of the n-type charge generation layer may comprise at least one C₂ to C₂₄ N-heteroaryl or P=X group, with X being O, P, Se.

According to an embodiment, wherein the at least one C₂ to C₂₄ N-heteroaryl may be selected from a compound comprising at least one azine group, preferably at least two azine groups, also preferred three azine groups.

According to an embodiment, wherein the n-type charge generation layer may comprise an organic electron transport compound comprising at least one group selected from the list comprising of pyridine, pyrimidine, triazine, imidazole, benzimidazole, benzooxazole, quinone, benzoquinone, quinoxaline, benzoquinoxaline, acridine, phenanthroline, benzoacridine, dibenzoacridine.

According to an embodiment, wherein the n-type charge generation layer may comprise an organic electron transport compound comprising at least one phenanthroline group, preferably two phenanthroline groups.

According to an embodiment, wherein the organic electron transport compound of the n-type charge generation layer may comprise at least one P=O group.

According to another embodiment, wherein the organic electron transport compound differs in its chemical formula from the organic hole transport compound.

### Metal dopant

The metal dopant of the n-type charge generation layer may be selected from Li or Yb, preferably Yb.

According to an embodiment, the metal dopant is not a compound of formula (I). According to an another embodiment, the metal dopant is not a metal complex.

### n-type charge generation layer

The thickness of the n-type charge generation may be in the range from about 5 nm to about 25 nm, for example, in the range from about 10 nm to about 20 nm. When the thickness of the n-type charge generation layer is within this range, the n-type charge generation layer may have satisfactory electron-generating properties, without a substantial penalty in operating voltage.

According to one embodiment, the n-type charge generation layer may comprise
- at least about ≥ 0.5 to about ≤ 10 vol.-%, preferably about ≥ 1 to about ≤ 5 vol.-% metal dopant; and
- at least about ≤ 99.5 vol.-% to about ≥ 90 vol.-%, preferably ≤ 99 vol.-% to about ≥ 95 vol.-% organic electron transport compound.

### Charge generation layer

According to one embodiment, the n-type charge generation layer and the p-type charge generation layer may be arranged in direct contact.

According to one embodiment, wherein the p-type and/or n-type charge generation layer and/or the compound of formula (I) are non-emissive.

In the context of the present specification the term "essentially non-emissive" or "non-emissive" means that the contribution of organic electron transport compound, the organic hole transport compound, the organic hole transport compound of formula (IIIa) or formula (IIIb), the compound of formula (I) and/or layer, the p-type charge generation layer, as well as the n-type charge generation layer, to the visible emission spectrum from an organic electronic device, such as OLED or display device, is less than 10 %, preferably less than 5 % relative to the visible emission spectrum. The visible emission spectrum is an emission spectrum with a wavelength of about ≥ 380 nm to about ≤ 780 nm.

### Charge generation connecting layer

According to one embodiment, the charge generation layer (CGL) may further comprise a charge generation connecting layer (c-CGL), wherein the charge generation connecting layer is arranged between the n-type charge generation layer and the p-type charge generation layer. Preferably, the charge generation connecting layer is in direct contact with the n-type charge generation layer and the p-type charge generation layer.

The charge generation connecting layer may comprise or consist of a conductive compound.

According to an embodiment, the conductive compound is selected from HAT-CN or a metal complex, preferably metal phthalocyanine, zinc phthalocyanine (ZnPc) or copper phthalocyanine (CuPc).

The thickness of the charge generation connecting layer may be selected between about 0.5 and 5 nm, preferably 1 and 2.5 nm.

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate, a silicon substrate or a transistor backplane. Preferably, the substrate is a silicon substrate or transistor backplane.

### Anode layer

The anode layer may be formed by depositing or sputtering a material that is used to form the anode layer. The material used to form the anode layer may be a high work-function material, so as to facilitate hole injection. The anode material may also be selected from a low work function material (i.e. aluminum). The anode layer may be a transparent or reflective electrode. Transparent conductive oxides, such as indium tin oxide (ITO), indium zinc oxide (IZO), tindioxide (SnO2), aluminum zinc oxide (AlZO) and zinc oxide (ZnO), may be used to form the anode layer. The anode layer may also be formed using metals, typically silver (Ag), gold (Au), or metal alloys.

According to one embodiment, the anode layer may be formed on a substrate, preferably the anode layer is in direct contact with a substrate.

### Hole injection layer

A hole injection layer (HIL) may be formed on the anode layer by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10⁻⁸ to 10⁻³ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The thickness of the HIL may be in the range from about 1 nm to about 30 nm, and for example, from about 1 nm to about 15 nm. When the thickness of the HIL is within this range, the HIL may have excellent hole injecting characteristics, without a substantial penalty in driving voltage.

According to an embodiment of the electronic device, wherein the hole injection layer is non-emissive.

It is to be understood that the hole injection layer is not part of the anode layer.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

According to one embodiment, wherein the organic electronic device further comprises a hole injection layer, wherein the hole injection layer is arranged between the anode layer and the first emission layer, and wherein the hole injection layer comprises a compound of formula (I).

According to an embodiment, the compound of formula (I) in the HIL and in the EIL may be selected the same.

According to one embodiment, the organic electronic device further comprises a hole injection layer, wherein the hole injection layer is arranged between the anode layer and the first emission layer, wherein the hole injection layer comprises a p-type dopant. The p-type dopant concentrations can be selected from 1 to 20 wt.-%, more preferably from 3 wt.-% to 10 wt.-%.

In one embodiment, the p-type dopant is selected from a radialene compound and/or a quinodimethane compound, wherein the radialene compound and/or quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

Preferably the p-type dopant is selected from a radialene compound. In one embodiment, the radialene compound may have formula (IV) and/or the quinodimethane compound may have formula (V) or (VI): 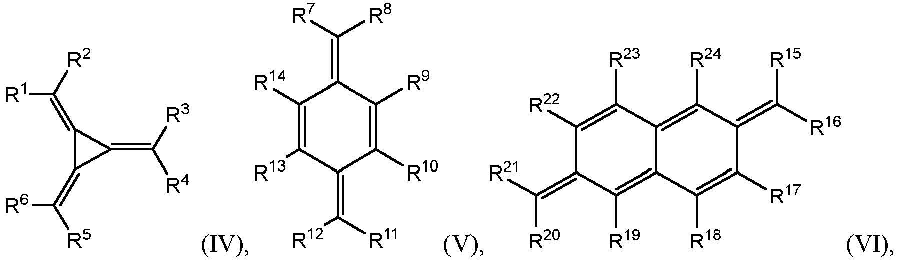 wherein R¹, R², R³, R⁴, R⁵, R⁶, R⁷, R⁸, R¹¹, R¹², R¹⁵, R¹⁶, R²⁰, R²¹ are independently selected from above mentioned electron withdrawing groups and R⁹, R¹⁰, R¹³, R¹⁴, R¹⁷, R¹⁸, R¹⁹, R²², R²³ and R²⁴ are independently selected from H, halogen and above mentioned electron withdrawing groups.

According to one embodiment, wherein the radialene compound is selected from formula (IVa): 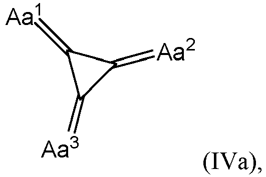 wherein in formula (IVa)
- Aa¹ is independently selected from a group of formula (IVb) 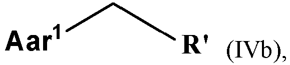
   wherein Aar¹ is independently selected from substituted or unsubstituted aryl and substituted or unsubstituted heteroaryl,
   wherein for the case that Aar¹ is substituted, one or more of the substituents are independently selected from the group comprising of D, an electron-withdrawing group, halogen, Cl, F, CN, -NO₂, substituted or unsubstituted C₁ to C₈ alkyl, partially fluorinated C₁ to C₈ alkyl, perfluorinated C₁ to C₈ alkyl, substituted or unsubstituted C₁ to C₈ alkoxy, partially fluorinated C₁ to C₈ alkoxy, perfluorinated C₁ to C₈ alkoxy, substituted or unsubstituted C₆ to C₃₀ aryl, and substituted or unsubstituted C₆ to C₃₀ heteroaryl and;
   wherein the one or more substituents of C₆ to C₃₀ aryl, C₆ to C₃₀ heteroaryl, C₁ to C₈ alkyl, C₁ to C₈ alkoxy are independently selected from D, an electron-withdrawing group, halogen, Cl, F, CN, - NO₂, partially fluorinated C₁ to C₈ alkyl, perfluorinated C₁ to C₈ alkyl, partially fluorinated C₁ to C₈ alkoxy, perfluorinated C₁ to C₈ alkoxy;
- Aa² is independently selected from a group of formula (IVc) 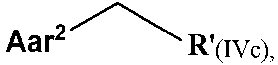
   wherein Aar² is independently selected from substituted or unsubstituted aryl and substituted or unsubstituted heteroaryl,
   wherein for the case that Aar² is substituted, one or more of the substituents are independently selected from the group comprising of D, an electron-withdrawing group, halogen, Cl, F, CN, -NO₂, substituted or unsubstituted C₁ to C₈ alkyl, partially fluorinated C₁ to C₈ alkyl, perfluorinated C₁ to C₈ alkyl, substituted or unsubstituted C₁ to C₈ alkoxy, partially fluorinated C₁ to C₈ alkoxy, perfluorinated C₁ to C₈ alkoxy, substituted or unsubstituted C₆ to C₃₀ aryl, and substituted or unsubstituted C₆ to C₃₀ heteroaryl and;
   wherein the one or more substituents of C₆ to C₃₀ aryl, C₆ to C₃₀ heteroaryl, C₁ to C₈ alkyl, C₁ to C₈ alkoxy are independently selected from D, an electron-withdrawing group, halogen, Cl, F, CN, - NO₂, partially fluorinated C₁ to C₈ alkyl, perfluorinated C₁ to C₈ alkyl, partially fluorinated C₁ to C₈ alkoxy, perfluorinated C₁ to C₈ alkoxy;
- Aa³ is independently selected from a group of formula (IVd) 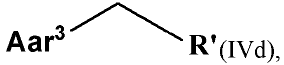
   wherein Aar³ is independently selected from substituted or unsubstituted aryl and substituted or unsubstituted heteroaryl,
   wherein for the case that Aar³ is substituted, one or more of the substituents are independently selected from the group comprising of D, an electron-withdrawing group, halogen, Cl, F, CN, -NO₂, substituted or unsubstituted C₁ to C₈ alkyl, partially fluorinated C₁ to C₈ alkyl, perfluorinated C₁ to Cs alkyl, substituted or unsubstituted C₁ to C₈ alkoxy, partially fluorinated C₁ to C₈ alkoxy, perfluorinated C₁ to C₈ alkoxy, substituted or unsubstituted C₆ to C₃₀ aryl, and substituted or unsubstituted C₆ to C₃₀ heteroaryl and;
      wherein the one or more substituents of C₆ to C₃₀ aryl, C₆ to C₃₀ heteroaryl, C₁ to C₈ alkyl, C₁ to C₈ alkoxy are independently selected from D, an electron-withdrawing group, halogen, Cl, F, CN, -NO₂, partially fluorinated C₁ to C₈ alkyl, perfluorinated C₁ to C₈ alkyl, partially fluorinated C₁ to C₈ alkoxy, perfluorinated C₁ to C₈ alkoxy;
   and wherein in Aa¹, Aa², Aa³ each R' is independently selected from substituted or unsubstituted C₆ to C₁₈ aryl, C₃ to C₁₈ heteroaryl, electron-withdrawing group, substituted or unsubstituted C₁ to C₈ alkyl, partially fluorinated C₁ to C₈ alkyl, perfluorinated C₁ to C₈ alkyl, halogen, F and CN; preferably the radialene compound is selected from 2,2',2"-(cyclopropane-1,2,3-triylidene)tris(2-(p-cyanotetrafluorophenyl)acetonitrile).

According to an embodiment, the p-type dopant is selected from a radialene compound, for example 2,2',2"-(cyclopropane-1,2,3-triylidene)tris(2-(p-cyanotetrafluorophenyl)acetonitrile).

The HIL may comprise an organic hole transport compound and a p-type dopant.

The p-type dopant concentrations can be selected from 1 to 50 wt.-%, more preferably from 3 wt.-% to 30 wt.-%.

The p-type dopant concentrations can be selected from 1 to 50 vol.-%, more preferably from 3 vol.-% to 30 vol.-%.

According to a preferred embodiment, wherein however, the HIL comprises a compound of formula (I) as described above.

According to a preferred embodiment, wherein the HIL may comprises the same compound of formula (I) as in the p-type charge generation layer.

According to a preferred embodiment, wherein the HIL may comprises an organic hole transport compound as described above.

According to a preferred embodiment, wherein the HIL may comprises a compound of formula (I), as described above, and an organic hole transport compound or a compound of formula (IIIa) or (IIIb), as described above.

According to a preferred embodiment, wherein the p-type charge generation layer and the hole injection layer may comprise an identical organic hole transport compound or compound of formula (IIIa) or (IIIb).

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in operating voltage.

### Hole transport layer

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

According to one embodiment, wherein the hole transport layer may comprise an organic hole transport compound as described above.

According to a preferred embodiment, wherein the hole injection layer and the hole transport layer may comprise an identical organic hole transport compound as described above.

According to one embodiment the p-type charge generation layer and the hole transport layer comprises an organic hole transport compound.

According to one embodiment the p-type charge generation layer and the hole transport layer comprise an organic hole transport compound, wherein the organic hole transport compound in the p-type charge generation layer and in the hole transport layer are selected the same.

According to one embodiment, wherein the hole transport layer may comprise a compound of formula (IIIa) or (IIIb) as described above.

According to a preferred embodiment, wherein the hole injection layer and the hole transport layer may comprise an identical compound of formula (IIIa) or (IIIb) as described above.

According to a preferred embodiment, wherein the p-type charge generation layer, the hole injection layer and the hole transport layer may comprise an identical organic hole transport compound.

According to a preferred embodiment, wherein the p-type charge generation layer, the hole injection layer and the hole transport layer may comprise an identical compound of formula (IIIa) or (IIIb) as described above.

According to a preferred embodiment, wherein the hole injection layer, the hole transport layer and the p-type charge generation layer may comprise an identical compound of formula (IIIa) or (IIIb) as described above; and the hole injection layer and the p-type charge generation layer may comprise an identical compound of formula (I), as described above.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in operating voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime may be improved. Typically, the electron blocking layer comprises a triarylamine compound.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer may be selected from triarylamine compounds with a triplet level above the triplet level of the phosphorescent emitter in the adjacent emission layer.

The thickness of the electron blocking layer may be selected between 2 and 20 nm.

### Emission layer (EML)

The first emission layer (EML), also referred to as at least one emission layer may be formed on the HTL or EBL by vacuum deposition, spin coating, slot-die coating, printing, casting, LB deposition, or the like. When the EML is formed using vacuum deposition or spin coating, the conditions for deposition and coating may be similar to those for the formation of the p-type charge generation layer. However, the conditions for deposition and coating may vary, according to the compound that is used to form the EML.

According to the present invention it is preferred that the organic electronic device comprises one emission layer that is named "first emission layer". However the organic electronic device optionally comprises two emission layers, wherein the first emission layer is named first emission layer and second emission layer is named second emission layer.

The first emission layer (EML) may be formed of a combination of a host and an emitter dopant. Example of the host are Alq3, 4,4'-N,N'-dicarbazole-biphenyl (HTC-10), poly(n-vinyl carbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)₂Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

According to a preferred embodiment, wherein the first emission layer comprises a compound of formula (VII) and an organic emitter host compound: 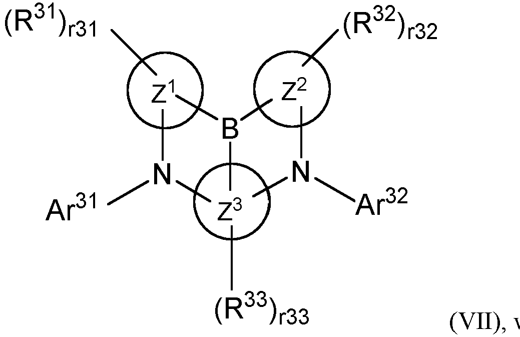 wherein
- Z¹, Z² and Z³: are the same as or selected different from each other, and are each independently selected from the group comprising a monocyclic to polycyclic aromatic hydrocarbon ring or monocyclic to polycyclic aromatic hetero ring;
- Ar³¹ and Ar³²: are the same as or selected different from each other, and are each independently and are each independently selected from the group comprising a substituted or unsubstituted alkyl group, a substituted or unsubstituted aryl group, or a substituted or unsubstituted heteroaryl group, or are bonded to an adjacent substituent to form a substituted or unsubstituted aromatic ring or a substituted or unsubstituted aliphatic ring;
- R³¹, R³² and R³³: are the same as or selected different from each other, and are each independently selected from the group comprising hydrogen, deuterium, a substituted or unsubstituted alkyl group, a substituted or unsubstituted silyl group, a substituted or unsubstituted amine group, a substituted or unsubstituted aryl group, or a substituted or unsubstituted heteroaryl group, or adjacent substituents are bonded to each other to form a substituted or unsubstituted aromatic ring or a substituted or unsubstituted aliphatic ring,
wherein one or more substituents selected from the group comprising of deuterium, an alkyl group having 1 to 6 carbon atoms, an alkyl silyl group having 1 to 30 carbon atoms, an aryl silyl group having 6 to 50 carbon atoms, an alkylamine group having 1 to 30 carbon atoms, an alkylarylamine group
having 1 to 50 carbon atoms, an arylamine group having 6 to 50 carbon atoms, an aryl group having 6 to 30 carbon atoms, and a heteroaryl group having 2 to 30 carbon atoms, or a substituent to which two or more substituents selected from the group are linked, or adjacent substituents are bonded to each other to form an aliphatic hydrocarbon ring having 3 to 60 carbon atoms, which is unsubstituted or substituted with the substituent;
- r31, r32 and r33: are each an integer from 0, 1, 2, 3 or 4, and when r31 to r33 are 2 or higher, substituents in the parenthesis are the same as or selected different from each other.

According to one embodiment, wherein the organic emitter host compound comprises:
- at least one condensed aromatic ring system comprising of at least ≥ 3 to ≤ 5 condensed aromatic rings, and
- at least ≥ 3 to ≤ 7 aromatic rings and/or at least ≥ 3 to ≤ 7 heteroaromatic rings,
   wherein at least ≥ 2 to ≤ 7 of the aromatic rings and/or at least ≥ 3 to ≤ 7 of the heteroaromatic rings are condensed to form a fused aromatic ring system and/or fused heteroaromatic ring systems, and
wherein the molecular weight Mw of the organic emitter host compound is in the range of ≥ 400 g/mol and ≤ 2000 g/mol.

According to one preferred embodiment, wherein the first emission layer comprises a compound of formula (VII) is selected from the compounds BD1 to BD9:

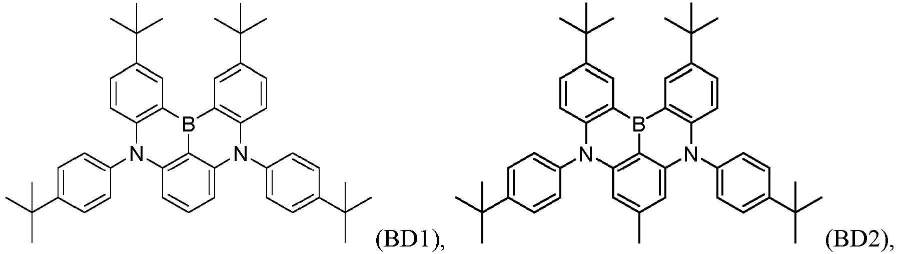

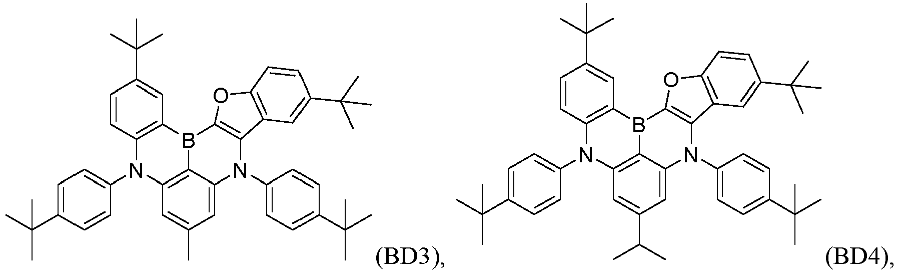

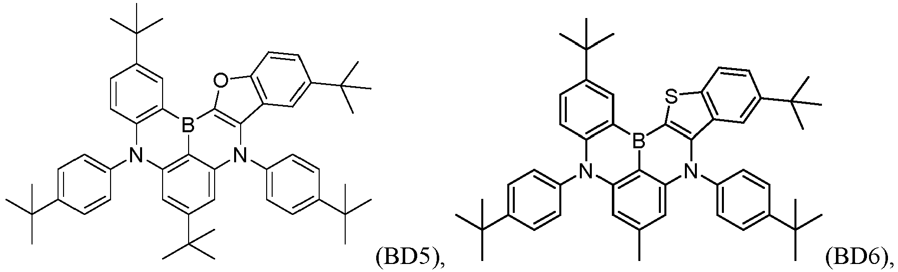

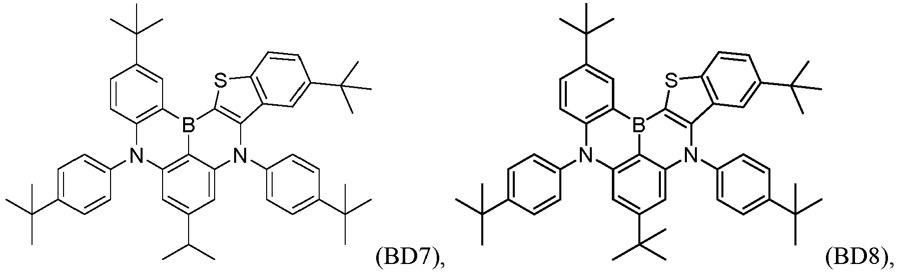

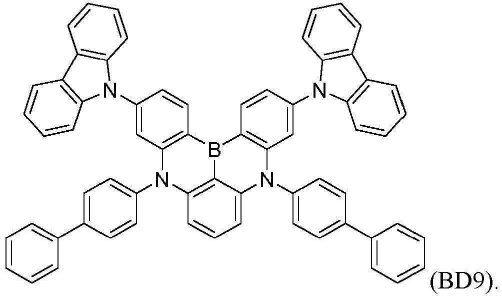

According to a preferred embodiment of the present invention, wherein the first emission layer comprises an organic emitter host compound, wherein the organic emitter host compound comprises
- at least one condensed aromatic ring system consisting of 3 to 5 rings, and
- 3 to 7 aromatic or heteroaromatic rings, wherein one or more sub-groups of the aromatic and/or heteroaromatic rings may be condensed to form fused aromatic or heteroaromatic ring systems;
wherein the molecular weight Mw of the organic emitter host compound is in the range of ≥ 400 and ≤ 2000 g/mol.

### Organic emitter host compound

According to a preferred embodiment of the present invention, wherein the organic emitter host compound has the formula (VIII) 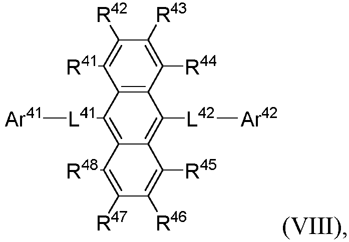 wherein
Ar⁴¹ and Ar⁴² are independently selected from substituted or unsubstituted C₆ to C₂₄ aryl, substituted or unsubstituted C₃ to C₂₄ heteroaryl;
L⁴¹ and L⁴² are independently selected from a direct bond or substituted or unsubstituted C₆ to C₂₄ arylene, substituted or unsubstituted C₃ to C₂₄ heteroarylene;
R⁴¹ to R⁴⁸ are independently selected from H, D, substituted or unsubstituted C₁ to C₁₂ alkyl, substituted or unsubstituted C₆ to C₁₉ aryl, substituted or unsubstituted C₃ to C₁₂ heteroaryl;
wherein
the substituents on Ar⁴¹, Ar⁴², L⁴¹, L⁴², R⁴¹ to R⁴⁸ are independently selected from D, C₆ to C₁₀ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, halogen, F or CN.

According to a preferred embodiment of the present invention, wherein the organic emitter host compound or the organic emitter host compound of formula (VIII) is selected from the compounds BH1 to BH13:

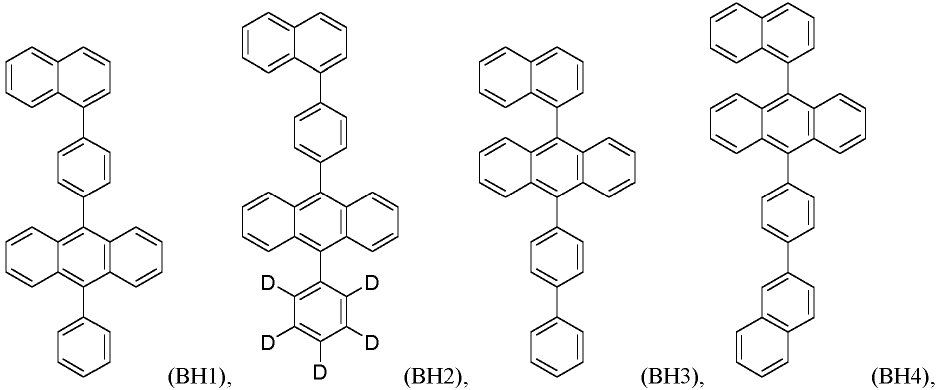

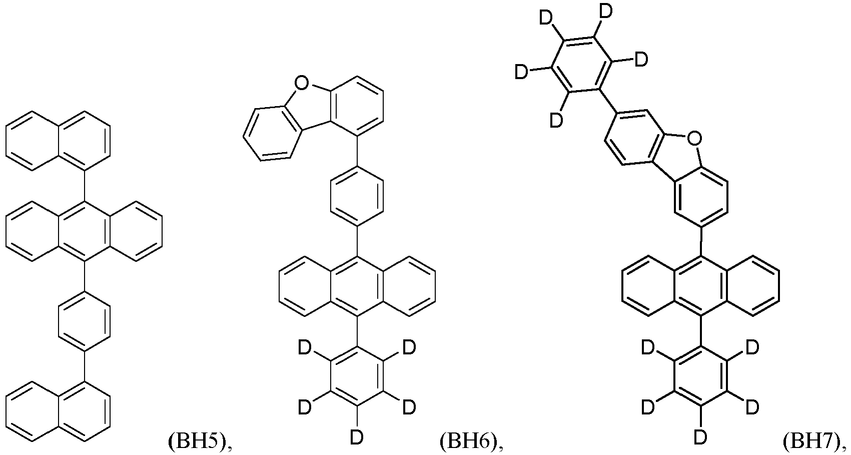

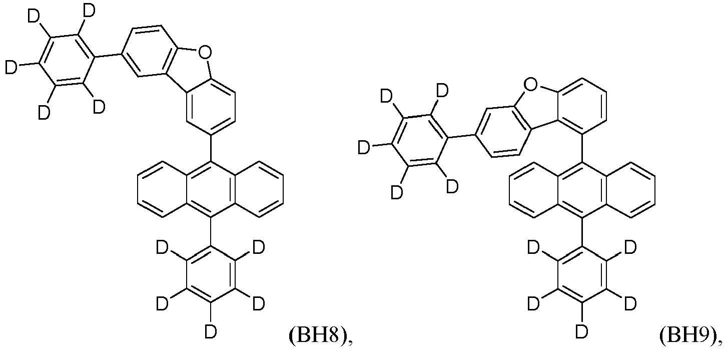

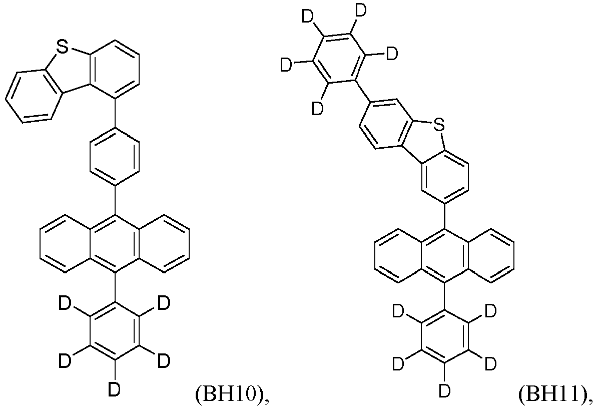

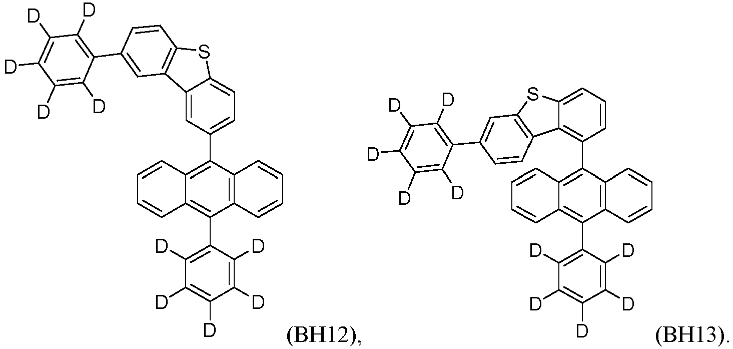

According to a preferred embodiment of the present invention, wherein the first emission layer comprises a compound of formula (VII) and an organic emitter host of formula (VIII).

According to a preferred embodiment of the present invention, wherein the p-type charge generation layer comprises a compound of formula (I) and a compound of formula (IIIa) or formula (IIIb), the hole transport layer comprises a compound of formula (IIIa) or formula (IIIb), preferably the p-type charge generation layer and the hole transport layer comprise the same compound of formula (IIIa) or formula (IIIb) and the first emission layer comprises a compound of formula (VII) and an organic emitter host of formula (VIII).

According to a preferred embodiment of the present invention, wherein the p-type charge generation layer comprises a compound of formula (I) and a compound of formula (IIIa) or formula (IIIb), the hole transport layer comprises a compound of formula (IIIa) or formula (IIIb), preferably the p-type charge generation layer and the hole transport layer comprise the same compound of formula (IIIa) or formula (IIIb) and the first emission layer comprises a compound of formula (VII) and an organic emitter host of formula (VIII);
wherein the anode layer may comprise a first anode sub-layer comprising Ag or Au having a thickness of 100 to 150 nm, a second anode sub-layer comprising or consisting of a transparent conductive oxide having a thickness of 3 to 20 nm and a third anode sub-layer comprising or consisting of a transparent conductive oxide having a thickness of 3 to 20 nm, preferably the transparent conductive oxide is selected from ITO or IZO.

The amount of the emitter dopant may be in the range from about 0.01 to about 50 parts by weight, based on 100 parts by weight of the host. Alternatively, the at first emission layer may consist of a light-emitting polymer. The EML may have a thickness of about 10 nm to about 100 nm, for example, from about 15 nm to about 60 nm. When the thickness of the EML is within this range, the EML may have excellent light emission, without a substantial penalty in operating voltage.

It may be provided that the at least one emission layer also referred to as first emission layer is free of the compound of formula (I) of the p-type charge generation layer.

It may be provided that the at least one emission layer does not comprise the compound of Formulae (IIIa) or (IIIb).

It may be provided that the first emission layer is free of H09 (Sun Fine Chemicals, Korea) and/or BD200 (Sun Fine Chemicals, Korea).

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the n-type charge generation. When the EML comprises a phosphorescent emitter dopant, the HBL may have also a triplet exciton blocking function.

When the HBL is formed using vacuum deposition or spin coating, the conditions for deposition and coating may be similar to those for the formation of the p-type charge generation layer. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and triazine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The organic electronic device according to the present invention may further comprise an electron transport layer (ETL).

According to another embodiment of the present invention, wherein the electron transport layer may comprise at least one compound selected from the group comprising an azine, triazine, phenanthroline, imidazole, benzimidazole and/or phosphine oxide compound, and preferably a phosphine oxide compound, and preferably an azine compound, or triazine compound, and more preferred a phosphine oxide compound.

In one embodiment, the ETL may comprise a dopant selected from an organic metal complex, preferably an alkali organic complex, preferably LiQ.

The thickness of the ETL may be in the range from about 15 nm to about 50 nm, for example, in the range from about 20 nm to about 40 nm.

### Electron injection layer (EIL)

An optional EIL, which may facilitates injection of electrons from the cathode, may be formed on the electron transport layer (ETL), preferably directly on the ETL . Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the n-type charge generation layer, although the deposition and coating conditions may vary, according to the material that is used to form the EIL.

The thickness of the EIL may be in the range from about 0.1 nm to about 30 nm, for example, in the range from about 0.5 nm to about 25 nm. When the thickness of the EIL is within this range, the EIL may have satisfactory electron-injecting properties, without a substantial penalty in operating voltage.

### Cathode layer

The cathode layer is formed on the p-type charge generation layer, ETL or optional EIL if present. The cathode layer may be formed of a metal, an alloy, an electrically conductive compound, or a mixture thereof. The cathode layer may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode layer may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer, second HTL and/or the p-type charge generation layer.

### Organic electronic device

According to one embodiment, the organic electronic device may further comprise a substrate, wherein the anode layer or the cathode layer is arranged on the substrate; preferably the anode layer or cathode layer is arranged in direct contact with the substrate.

According to one embodiment, the organic electronic device may further comprise a hole injection layer, wherein the hole injection layer is arranged between the anode layer and the n-type charge generation layer.

According to one embodiment, the organic electronic device may further comprise a first hole transport layer; wherein the first hole transport layer is arranged between the anode layer and the n-type charge generation layer.

According to one embodiment, the organic electronic device may further comprise a hole injection layer and a first hole transport layer; wherein the first hole transport layer is arranged between the hole injection layer and the n-type charge generation layer.

According to one embodiment, the organic electronic device may further comprise a first hole transport layer and a second hole transport layer, wherein the first hole transport layer is arranged between the anode layer and the n-type charge generation layer and the second hole transport layer is arranged between p-type charge generation layer and the cathode layer.

According to one embodiment, wherein the first and/or second hole transport layer comprises an organic hole transport compound.

According to one embodiment, wherein the hole transport layer comprises an organic hole transport compound and the p-type charge generation layer comprises an organic hole transport compound, wherein the organic hole transport compound in the p-type charge generation layer and hole transport layer are selected the same.

According to one embodiment, wherein the first and second hole transport layer comprise an organic hole transport compound and the p-type charge generation layer comprises an organic hole transport compound wherein the organic hole transport compound in the p-type charge generation layer and the first and second hole transport layer are selected the same.

According to one embodiment, wherein the first and second hole transport layer comprise an organic hole transport compound and the p-type charge generation layer comprises an organic hole transport compound wherein the organic hole transport compound in the p-type charge generation layer and the first and second hole transport layer are selected differently thereto.

According to one embodiment, the organic electronic device may further comprise at least one first emission layer, wherein the at least one first emission layer is arranged between the anode layer and the n-type charge generation layer.

According to one embodiment, the organic electronic device may further comprise a first and a second emission layer, wherein the first emission layer is arranged between the anode layer and the n-type charge generation layer and the second emission layer is arranged between the p-type charge generation layer and the cathode layer.

The organic electronic device according to the invention may be an organic light-emitting diode.

According to one aspect of the present invention, wherein there is provided an organic light-emitting diode (OLED) comprising: a substrate; an anode layer formed on the substrate; a charge generation layer according to invention, at least one emission layer and a cathode layer.

According to one aspect of the present invention, wherein there is provided an organic light-emitting diode (OLED) comprising: a substrate; an anode layer formed on the substrate; a charge generation layer according to invention, at least a first and a second emission layers and a cathode layer, wherein the charge generation layer is arranged between the first and the second emission layer.

According to one aspect of the present invention, wherein there is provided an organic light-emitting diode (OLED) comprising: a substrate; an anode layer formed on the substrate; a hole injection layer which may comprise a compound of formula (I), a hole transport layer, a first emission layer, an electron transport layer, a n-type charge generation layer, a p-type charge generation layer comprising a compound of formula (I), a hole transport layer, a second emission layer, an optional electron transport layer, an optional electron injection layer and a cathode layer.

According to various embodiments of the present invention, wherein there may be provided OLEDs layers arranged between the above mentioned layers, on the substrate or on the top electrode.

According to one aspect, the OLED may comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a p-type charge generating layer, the p-type charge generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional electron injection layer are arranged.

### Method of manufacturing

According to another aspect, wherein there is provided a method of manufacturing an organic electronic device, the method using at least three deposition sources, preferably four deposition sources.

The methods for deposition that can be suitable comprise:
- deposition via vacuum thermal evaporation;
- deposition via solution processing, preferably the processing may be selected from spin-coating, printing, casting; and/or
- slot-die coating.

According to various embodiments, wherein there is provided a method using:
- a first deposition source to release the organic electronic transport compounds,
- a second deposition source to release the metal dopant,
- a third deposition source to release the organic hole transport compound, and
- a fourth deposition source to release compound of formula (I).

The method comprising
- the step of forming the n-type charge generation layer; whereby for an organic electronic device the n-type charge generation layer is formed by releasing the organic electron transport compound from the first deposition source and the metal dopant from the second deposition source;
- followed by a step of forming the p-type charge generation layer; whereby for an organic electronic device the p-type charge generation layer is formed by releasing the organic hole transport compound from the third deposition source and compound of formula (I) from the fourth deposition source; in this order or in the reverse order.

According to various embodiments, wherein the method may further include the steps for forming an organic electronic device, wherein in that order the layers substrate, anode layer, hole injection layer HIL, first hole transport layer HTL, first electron blocking layer EBL, first emission layer EML, first hole blocking layer HBL, first electron transport layer ETL, n-type charge generation, p-type charge generation layer, second hole transport layer HTL, second electron blocking layer, second emission layer, second hole blocking layer, second electron transport layer, electron injection layer, and cathode layer are formed on each other starting with a substrate onto an anode layer is formed; in this order or in the reverse order.

### Electronic device

According to another aspect, it is provided an electronic device comprising at least one organic light emitting device according to any embodiment described throughout this application, preferably, the electronic device comprises the organic light emitting diode in one of embodiments described throughout this application. More preferably, the organic electronic device is a light emitting device or a display device, preferably a display device. According to one embodiment of the organic electronic device according, wherein light is emitted through the cathode layer.

According to one embodiment, the organic electronic device is a display device. The display device comprising an organic electronic device according to the invention.

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object are disclosed in the dependent claims and the following description of the respective figures which in an exemplary fashion show preferred embodiments according to the invention. Any embodiment does not necessarily represent the full scope, however, and reference is made therefore to the claims and herein for interpreting the scope. It is to be understood that both the foregoing general description and the following detailed description are exemplary and explanatory only and are intended to provide further explanation of the present invention as claimed.

### Figures 1 to 5

- FIG. 1: is a schematic sectional view of an OLED comprising a charge generation layer, according to an exemplary embodiment of the present invention.
- FIG. 2: is a schematic sectional view of an OLED comprising a charge generation layer, according to an exemplary embodiment of the present invention.
- FIG. 3: is a schematic sectional view of a stacked OLED comprising a charge generation layer, according to an exemplary embodiment of the present invention.
- FIG. 4: is a schematic sectional view of a stacked OLED comprising a charge generation layer, according to an exemplary embodiment of the present invention.
- FIG. 5: is a schematic sectional view of a stacked OLED comprising a charge generation layer, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

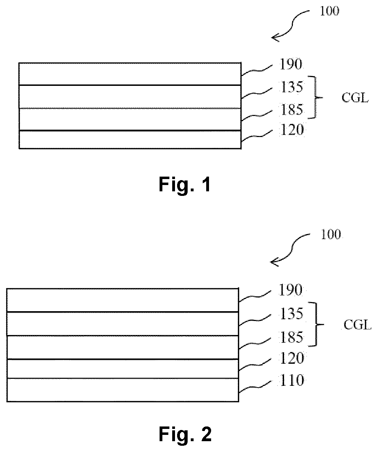

Fig. 1 is a schematic sectional view of an OLED 100, according to one exemplary embodiment of the present invention.

Referring to Fig. 1 the OLED 100 includes an anode layer 120, an n-type charge generation layer (n-CGL) 185, wherein the n-type charge generation layer (n-CGL) 185 comprises an electron transport compound and a metal dopant, a p-type charge generation layer (p-GCL) 135, wherein the p-type charge generation layer (p-GCL) 135 may comprise a compound of formula (I) and an organic hole transport compound, and a cathode layer 190.

Fig. 2 is a schematic sectional view of an OLED 100, according to one exemplary embodiment of the present invention.

Referring to Fig. 2 the OLED 100 includes a substrate 110, an anode layer 120, an n-type charge generation layer (n-CGL) 185, wherein the n-type charge generation layer (n-CGL) 185 comprises an electron transport compound and a metal dopant, a p-type charge generation layer (p-GCL) 135, wherein the p-type charge generation layer (p-GCL) 135 may comprise a compound of formula (I) and an organic hole transport compound, and a cathode layer 190.

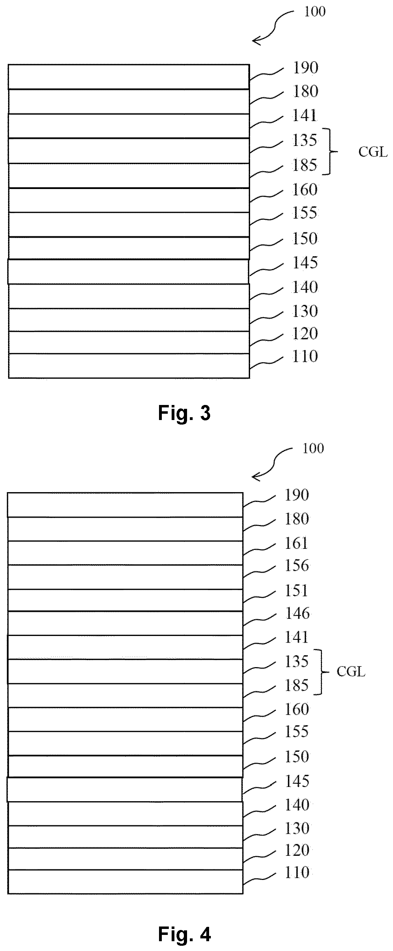

Fig. 3 is a schematic sectional view of an OLED 100, according to one exemplary embodiment of the present invention.

Referring to Fig. 3 the OLED 100 includes a substrate 110, an anode layer 120, a hole injection layer (HIL) 130, a first hole transport layer (HTL1) 140, an electron blocking layer (EBL) 145, an emission layer (EML) 150, a hole blocking layer (HBL) 155, an electron transport layer (ETL) 160, an n-type charge generation layer (n-CGL) 185, a p-type charge generation layer (p-GCL) 135, a second hole transport layer (HTL2) 141, and electron injection layer (EIL) 180 and a cathode layer 190. The HIL may comprise a compound of formula (I).

Fig. 4 is a schematic sectional view of a stacked OLED 100, according to another exemplary embodiment of the present invention. Fig. 4 differs from Fig. 3 in that the OLED 100 of Fig. 4 further comprises a second emission layer.

Referring to Fig. 4 the OLED 100 includes a substrate 110, an anode layer 120, a hole injection layer (HIL) 130, a first hole transport layer (HTL) 140, a first electron blocking layer (EBL) 145, a first emission layer (EML) 150, an optional first hole blocking layer (HBL) 155, a first electron transport layer (ETL) 160, an n-type charge generation layer (n-CGL) 185, a p-type charge generation layer (p-GCL) 135 , a second hole transport layer (HTL) 141, a second electron blocking layer (EBL) 146, a second emission layer (EML) 151, an optional second hole blocking layer (HBL) 156, a second electron transport layer (ETL) 161, an electron injection layer (EIL) 181 and a cathode layer 190. The HIL may comprise a compound of formula (I).

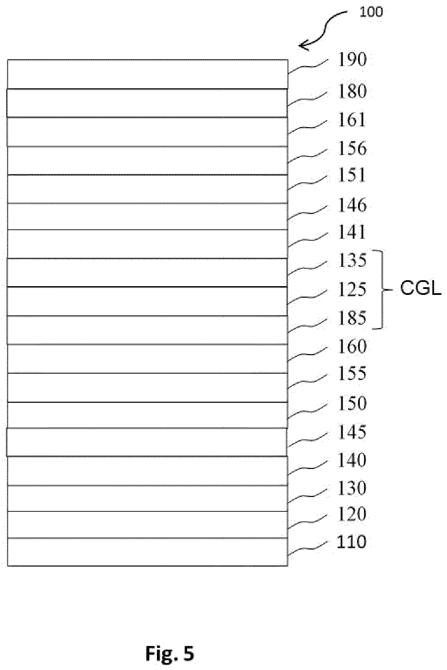

Fig. 5 is a schematic sectional view of a stacked OLED 100, according to another exemplary embodiment of the present invention. Fig. 5 differs from Fig. 4 in that the OLED 100 of Fig. 5 further comprises a charge generation connecting layer.

Referring to Fig. 5 the OLED 100 includes a substrate 110, an anode layer 120, a hole injection layer (HIL) 130, a first hole transport layer (HTL) 140, a first electron blocking layer (EBL) 145, a first emission layer (EML) 150, an optional first hole blocking layer (HBL) 155, a first electron transport layer (ETL) 160, an n-type charge generation layer (n-CGL) 185, a charge generation connecting layer 125 (c-CGL), a p-type charge generation layer (p-GCL) 135, a second hole transport layer (HTL) 141, a second electron blocking layer (EBL) 146, a second emission layer (EML) 151, an optional second hole blocking layer (HBL) 156, a second electron transport layer (ETL) 161, an electron injection layer (EIL) 181 and a cathode layer 190. The HIL may comprise a compound of formula (I).

While not shown in Fig. 1, Fig. 2, Fig. 3, Fig. 4 and Fig. 5, a capping and/or a sealing layer may further be formed on the cathode layer (190), in order to seal the organic electronic device 100. In addition, various other modifications may be applied thereto.

Hereinafter, the embodiments are illustrated in more detail with reference to examples. However, the present disclosure is not limited to the following examples.

### Detailed description

The invention is furthermore illustrated by the following examples which are illustrative only and non-binding.

The compounds of formula (I) and compounds of formulae (IIIa) and (IIIb), (IV) to (VIII) and may be prepared by methods known in the art.

### HOMO of substantially covalent matrix compounds and compounds of formula (IIIa) or (IIIb)

The HOMO energy of substantially covalent matrix compounds and compounds of formula (IIIa) or (IIIb) is calculated with the program package TURBOMOLE V6.5 (TURBOMOLE GmbH, Litzenhardtstrasse 19, 76135 Karlsruhe, Germany) from the optimized geometry by applying the hybrid functional B3LYP with a Def2-TZVP basis set in the gas phase and inclusion of dispersion forces as D3BJ. Geometry is optimized by applying the hybrid functional B3LYP with a Def2-SVP basis set in the gas phase and inclusion of dispersion forces as D3BJ. If more than one conformation is viable, the conformation with the lowest total energy is selected.

If calculated by this method, the HOMO of N2,N2,N2',N2',N7,N7,N7',N7'-octakis(4-methoxyphenyl)-9,9'-spirobi[fluorene]-2,2',7,7'-tetraamine is -4.27 eV.

### LUMO of compounds of formula (I)

The LUMO energy of the compound of formula (I) is calculated as single point energy calculation with the program package TURBOMOLE V6.5 (TURBOMOLE GmbH, Litzenhardtstrasse 19, 76135 Karlsruhe, Germany) from the optimized geometry by applying the hybrid functional B3LYP with a Def2-TZVP basis set in the gas phase and inclusion of dispersion forces as D3BJ. Geometry is optimized by applying the hybrid functional B3LYP with a Def2-SVP basis set in the gas phase and inclusion of dispersion forces as D3BJ. If more than one conformation is viable, the conformation with the lowest total energy is selected.

### Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 µL Mettler Toledo aluminum pan with lid, a <1 mm hole is pierced into the lid).

The melting point of compounds of formula (I) and comparative compounds can be seen in Table 1.

### Decomposition temperature T_{dec}

The decomposition temperature T_{dec} is measured by loading a sample of 9 to 11 mg into a Mettler Toledo 100 µL aluminum pan without lid under nitrogen in a Mettler Toledo TGA-DSC 1machine. The following heating program was used: 25°C isothermal for 3 min; 25°C to 600°C with 10 K/min.

The decomposition temperature was determined based on the onset of the decomposition in TGA.

The decomposition temperature indicates the temperature at which the compound decomposes. The higher the decomposition temperature the higher the thermal stability of a compound.

The decomposition temperature of compounds of formula (I) and comparative compounds can be seen in Table 1.

### HV-TGA 5% mass loss temperature

10 mg compound are loaded into 2 cm³ Al₂O₃ crucible, which is mounted in high vacuum - thermogravimetric analysis (HV-TGA) setup. The HV-TGA setup consists of an evaporation source (Creaphys DE-2-CF40), a thermocouple (Thermo Sensor GmbH NiCr-Ni, Typ K) placed inside the crucible and a quartz crystal microbalance (QCM, Inficon 750-1000-G10, 6 MHz). The HV-TGA setup is part of vacuum chamber system equipped with a scroll pump, a turbomolecular pump, a nitrogen inlet with mass flow controller and a progressive valve between scroll and turbomolecular pump. The combination of nitrogen inlet and pump valve allows pressures of 1e-2 mbar to 1e-6 mbar, whereas standard operational pressure is 1e-4 mbar with a stability of +/- 10%. Subsequent to reaching desired pressure the evaporation source temperature is ramped from room temperature to 600 °C at a rate of 10 °C/min. The compound is fully evaporated and detected by the QCM. The frequency shift of the QCM during the whole temperature ramp corresponds to a mass loss of 100%.

The reference temperature for dopant materials is taken at a mass loss of 5% as the obtained values have closest match to processing temperature in linear evaporation sources at mass production.

The higher the TGA 5% value the lower the volatility of the compound.

The TGA 5% values of compounds of formula (I) and comparative compounds can be seen in Table 1.

### Rate onset temperature T_{RO}

The rate onset temperature T_{RO} is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than 10⁻⁵ mbar and the temperature inside the source measured with a thermocouple. Evaporation of the compound is detected with a QCM detector which detects deposition of the compound on the quartz crystal of the detector. The deposition rate on the quartz crystal is measured in Ǻngstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 100 to 300 °C. If the rate onset temperature is below 100 °C the evaporation may be too rapid and therefore difficult to control. If the rate onset temperature is above 300 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

The T_{RO} of compounds of formula (I) and comparative compounds can be seen in Table 1.

### General procedure for fabrication of OLEDs comprising a n-CGL and a p-CGL

For all inventive and comparative examples (see Table 2 and 3), a glass substrate with an anode layer comprising a first anode sub-layer of 120 nm Ag, a second anode sub-layer of 8 nm ITO and a third anode sub-layer of 10 nm ITO was cut to a size of 50 mm x 50 mm x 0.7 mm, ultrasonically washed with water for 60 minutes and then with isopropanol for 20 minutes. The liquid film was removed in a nitrogen stream, followed by plasma treatment to prepare the anode layer. The plasma treatment was performed in an atmosphere comprising 97.6 vol.-% nitrogen and 2.4 vol.-% oxygen.

Then, a hole injection layer was formed on the anode layer in vacuum. In examples 1-1 to 1-4 and comparative examples 1-1 to 1-10, compound of formula (I) or comparative compound (collectively named "p-dopant") and compound of formula (IIIa) or (IIIb) are co-deposited on the anode layer to form a hole injection layer. In examples 1-5 to 1-9, a compound of formula (IV) (named "p-dopant") and compound of formula (IIIa) or (IIIb) are co-deposited on the anode layer to form a hole injection layer. The composition and thickness of the hole injection layer can be seen in Table 2.

Then, compound of formula (IIIa) or (IIIb) was vacuum deposited on the HIL, to form a first hole transport layer (HTL1). The composition and thickness of the HTL can be seen in Table 2.

Then N,N-di([1,1'-biphenyl]-4-yl)-3'-(9H-carbazol-9-yl)-[1,1'-biphenyl]-4-amine was vacuum deposited on the first HTL, to form a first electron blocking layer (EBL1) having a thickness of 5 nm.

Then 99 vol.-% BH9 as organic emitter host and 1 vol.-% BD8 as light-emitting compound were deposited on the EBL1, to form a first emission layer (EML1) with a thickness of 19 nm.

Then a first electron transport layer (ETL1) is formed on the first emission layer by depositing 2,2'-(1,3-Phenylene)bis[9-phenyl-1,10-phenanthroline] having a thickness of 15 nm.

Then, the n-type charge generation layer (n-CGL) having a thickness of 8 nm is formed on the ETL1 by co-depositing an organic electron transport compound ETM and a metal dopant. The composition and thickness of the n-CGL can be seen in Table 2.

Then, the p-type charge generation layer (p-CGL) is formed on the n-CGL by co-depositing an organic hole transport compound HTM and compound of formula (I) or comparative compound, that are named "p-dopant" herein. The composition and thickness of the p-CGL can be seen in Table 2.

Then, a second hole transport layer (HTL2) having a thickness of 41 nm is formed on the p-CGL by depositing the same organic hole transport compound as in the first hole transport layer. The composition of the second hole transport layer is the same as of the first hole transport layer.

Then N,N-di([1,1'-biphenyl]-4-yl)-3'-(9H-carbazol-9-yl)-[1,1'-biphenyl]-4-amine was vacuum deposited on the second HTL, to form a second electron blocking layer (EBL2) having a thickness of 5 nm.

Then 99 vol.-% % BH9 as organic emitter host and 1 vol.-% BD8 as light-emitting compound were deposited on the EBL2, to form a second emission layer (EML2) with a thickness of 19 nm.

Then a first hole blocking layer (HBL1) is formed on the second emission layer by depositing 4-([1,1'-biphenyl]-4-yl)-6-(3'-(9,9-dimethyl-9H-fluoren-4-yl)-[1,1'-biphenyl]-4-yl)-2-phenylpyrimidine having a thickness of 5 nm.

Then a second electron transport layer (ETL2) is formed on the HBL1 by co-depositing 50 vol.-% 6,6'-(naphthalene-1,2-diylbis(4,1-phenylene))bis(2,4-diphenyl-1,3,5-triazine) and 50 vol.-% LiQ having a thickness of 31 nm.

Then, the electron injection layer (EIL) having a thickness of 2 nm is formed on the second electron transport layer by depositing Yb.

Then the cathode layer having a thickness of 13 nm is formed on the electron injection layer by depositing Ag:Mg (90:10 vol.-%) at a rate of 0.01 to 1 Å/s at 10⁻⁷ mbar.

Then, N-([1,1'-biphenyl]-4-yl)-9,9-dimethyl-N-(4-(9-phenyl-9H-carbazol-3-yl)phenyl)-9H-fluoren-2-amine was deposited on the cathode layer to form a capping layer with a thickness of 75 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m² using an Instrument Systems CAS-140CT array spectrometer (calibrated by Deutsche Akkreditierungsstelle (DAkkS)) for each of the voltage values. The cd/A efficiency at 10 mA/cm² is determined by interpolating the luminance-voltage and current-voltage characteristics, respectively.

In bottom emission devices, the emission is predominately Lambertian and quantified in percent external quantum efficiency (EQE). The light is emitted through the anode layer. To determine the efficiency EQE in % the light output of the device is measured using a calibrated photodiode at 10 mA/cm².

In top emission devices, the emission is forward directed through the cathode layer, non-Lambertian and also highly dependent on the mirco-cavity. Therefore, the efficiency EQE will be higher compared to bottom emission devices. To determine the efficiency EQE in % the light output of the device is measured using a calibrated photodiode at 10 mA/cm².

The colour space is described by coordinates CIE-x and CIE-y (International Commission on Illumination 1931).

Lifetime LT of the device is measured at ambient conditions (20°C) and 30 mA/cm², using a Keithley 2400 source meter, and recorded in hours.

The brightness of the device is measured using a calibrated photo diode. The lifetime LT is defined as the time till the brightness of the device is reduced to 97 % of its initial value.

To determine the voltage stability over time U(100h)-(1h), a current density of at 30 mA/cm² was applied to the device. The operating voltage was measured after 1 hour and after 100 hours, followed by calculation of the voltage stability for the time period of 1 hour to 100 hours. A low value for U(100h)-(1h) denotes a low increase in operating voltage over time and thereby improved voltage stability.

### Technical Effect of the invention

In Table 1 are shown the thermal properties of compounds of formula (I) compared to comparative compound CC-1.

**Table 1: Thermal properties and rate onset temperature of compounds of formula (I) and comparative compound**

| Name | mp [°C] | TGA 5% [°C] | T_{dec} [°C] | T_{RO} [°C] |
|---|---|---|---|---|
| MC-10 | 287 | 323 | > 330 | 212 |
| MC-11 | 263 | 335 | > 360 | 202 |
| CC-1 | n.d. | 232 | > 250 | 190 |
| CC-2 | 198 | 267 | 450 | 236 |

The formula of CC-1 is 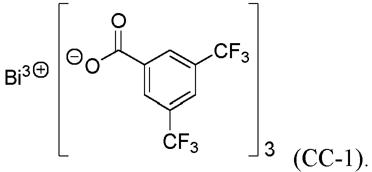 CC-2 has the formula KTFSI (CAS 90076-67-8).

As can be seen in Table 1, the thermal properties of compounds of formula (I) are substantially improved over the thermal properties of comparative compound CC-1, in particular the melting point, TGA 5% and the decomposition temperature are increased.

Additionally, the rate onset temperature of compounds of formula (I) is in the range suitable for mass production of organic electronic devices.

Improved thermal properties and a rate onset temperature in a suitable range are important for a high TACT time in mass production of organic electronic devices.

In Table 2 is shown the composition and layer thickness of an organic electronic device according to invention and comparative examples.

In Table 3 are shown data for an organic electronic device comprising a charge generation layer (CGL) according to invention and comparative examples.

In Example 1-1, the hole injection layer and the p-type CGL comprises matrix compound F3 and compound of formula (I) MC-10. The n-type CGL comprises electron transport material 2,2'-(1,3-Phenylene)bis[9-phenyl-1,10-phenanthroline] (ETM-1) and Yb as metal dopant. The operating voltage is 6.88 V, the EQE is 31.48%, the lifetime is 140 hours and the voltage stability over time U(100h)-(1h) is 0.251 V. The LUMO of F3 has been calculated as -4.89 eV.

In comparative example 1-1, the compound of formula (I) is replaced by comparative compound CC-2 in the hole injection layer and in the p-type CGL. As can be seen in Table 3, the operating voltage is > 10 V. Therefore, the EQE, LT and voltage stability over time were not determined.

In comparative example 1-1, the compound of formula (I) is replaced by comparative compound CC-1 in the hole injection layer and in the p-type CGL.

As can be seen in Table 3, the operating voltage is > 10 V. Therefore, the EQE, LT and voltage stability over time were not determined.

In Example 1-2, the hole injection layer and the p-type CGL comprises matrix compound F3 and compound of formula (I) MC-10. The n-type CGL comprises electron transport material ETM-1 and Li as metal dopant. The operating voltage is 6.63 V, the EQE is 31.42%, the lifetime is 119 hours and the voltage stability over time U(100h)-(1h) is 0.100 V. Surprisingly, efficient doping of F3 was achieved, even though the HOMO is further away from vacuum level compared to N2,N2,N2',N2',N7,N7,N7',N7'-octakis(4-methoxyphenyl)-9,9'-spirobi[fluorene]-2,2',7,7'-tetraamine.

In comparative example 1-3, the compound of formula (I) is replaced by comparative compound CC-2 in the hole injection layer and in the p-type CGL. As can be seen in Table 3, the operating voltage is > 10 V. Therefore, the EQE, LT and voltage stability over time were not determined.

In comparative example 1-4, the compound of formula (I) is replaced by comparative compound CC-1 in the hole injection layer and in the p-type CGL. As can be seen in Table 3, the operating voltage is > 10 V. Therefore, the EQE, LT and voltage stability over time were not determined.

In comparative example 1-5, the hole injection layer and the p-type CGL comprises matrix compound F3 and compound of formula (I) MC-10. The n-type CGL comprises electron transport material ETM-1 and Mg as metal dopant. As can be seen in Table 3, the operating voltage is 8.05 V and the EQE is 29.56 %, the lifetime is 51 hours and the voltage stability over time is 0.403 V.

In comparative example 1-6, the hole injection layer and the p-type CGL comprises matrix compound F18 and compound of formula (I) MC-10. The n-type CGL comprises electron transport material ETM-1 and Mg as metal dopant. As can be seen in Table 3, the operating voltage is 8.19 V and the EQE is 31.82 %, the lifetime is 48 hours and the voltage stability over time is 0.323 V.

In Example 1-3, the hole injection layer and the p-type CGL comprises matrix compound F18 and compound of formula (I) MC-10. The n-type CGL comprises electron transport material ETM-1 and Yb as metal dopant. The operating voltage is 6.98 V, the EQE is 33.04%, the lifetime is 115 hours and the voltage stability over time U(100h)-(1h) is 0.214 V. The HOMO of F18 has been calculated as -4.95 eV. Surprisingly, efficient doping of F18 was achieved, even though the HOMO is even further away from vacuum level compared to F3.

In comparative example 1-7, the compound of formula (I) is replaced by comparative compound CC-2 in the hole injection layer and in the p-type CGL. As can be seen in Table 3, the operating voltage is > 10 V. Therefore, the EQE, LT and voltage stability over time were not determined.

In comparative example 1-8, the compound of formula (I) is replaced by comparative compound CC-1 in the hole injection layer and in the p-type CGL. As can be seen in Table 3, the operating voltage is > 10 V. Therefore, the EQE, LT and voltage stability over time were not determined. In Example 1-4, the hole injection layer and the p-type CGL comprises matrix compound F18 and compound of formula (I) MC-10. The n-type CGL comprises electron transport material ETM-1 and Li as metal dopant. The operating voltage is 6.87 V, the EQE is 32.45%, the lifetime is 79 hours and the voltage stability over time U(100h)-(1h) is 0.132 V.

In comparative example 1-9, the compound of formula (I) is replaced by comparative compound CC-2 in the hole injection layer and in the p-type CGL. As can be seen in Table 3, the operating voltage is > 10 V. Therefore, the EQE, LT and voltage stability over time were not determined.

In comparative example 1-10, the compound of formula (I) is replaced by comparative compound CC-1 in the hole injection layer and in the p-type CGL. As can be seen in Table 3, the operating voltage is 9.87 V. Therefore, the LT and voltage stability over time were not determined.

In Example 1-5, the hole injection layer comprises matrix compound F3 and compound of formula (IV) 2,2',2"-(cyclopropane-1,2,3-triylidene)tris(2-(p-cyanotetrafluorophenyl)acetonitrile) (PD-1). The p-type CGL comprises matrix compound F3 and compound of formula (I) MC-10. The n-type CGL comprises electron transport material ETM-1 and Yb as metal dopant. The operating voltage is 6.88 V, the EQE is 31.24%, the lifetime is 140 hours and the voltage stability over time U(100h)-(1h) is 0.242 V.

In Example 1-6, the hole injection layer comprises matrix compound F3 and PD-1. The p-type CGL comprises matrix compound F3 and compound of formula (I) MC-10. The n-type CGL comprises electron transport material ETM-1 and Li as metal dopant. The operating voltage is 6.63 V, the EQE is 31.15%, the lifetime is 120 hours and the voltage stability over time U(100h)-(1h) is 0.126 V.

In Example 1-7, the hole injection layer comprises matrix compound F18 and PD-1. The p-type CGL comprises matrix compound F18 and compound of formula (I) MC-10. The n-type CGL comprises electron transport material ETM-1 and Yb as metal dopant. The operating voltage is 6.98 V, the EQE is 32.82%, the lifetime is 112 hours and the voltage stability over time U(100h)-(1h) is 0.218 V.

In Example 1-8, the hole injection layer comprises matrix compound F18 and PD-1. The p-type CGL comprises matrix compound F18 and compound of formula (I) MC-10. The n-type CGL comprises electron transport material ETM-1 and Li as metal dopant. The operating voltage is 6.87 V, the EQE is 32.33%, the lifetime is 75 hours and the voltage stability over time U(100h)-(1h) is 0.138 V.

In Example 1-9, the hole injection layer comprises matrix compound F18 and PD-1. The p-type CGL comprises matrix compound F18 and compound of formula (I) MC-11. The n-type CGL comprises electron transport material ETM-1 and Yb as metal dopant. The operating voltage is 6.9 V, the EQE is 30.82%, the lifetime is 121 hours and the voltage stability over time U(100h)-(1h) is 0.363 V.

In summary, the organic electronic device according to invention may have improved operating voltage, external quantum efficiency EQE, lifetime and/or voltage stability over time compared to the art.

A low operating voltage and/or high EQE may be beneficial for reduced power consumption of organic electronic devices, in particular mobile devices.

An improved lifetime and/or improved voltage stability over time may be beneficial for improved stability of organic electronic devices.

**Table 2: Composition and layer thicknesses of organic electronic devices comprising a charge generation layer (CGL)**

| | HIL | | | | | HTL1 | | n-type CGL | | | | | p-type CGL | | | | |
|---|---|---|---|---|---|---|---|---|---|---|---|---|---|---|---|---|---|
| | HTM | Concentration HTM (wt.-%) | p-dopant | Concentration p-dopant (wt.-%) | Thickness (nm) | HTM | Thickness (nm) | ETM | Concentration ETM (vol.-%) | Metal dopant | Concentration metal dopant (vol. %) | Thickness (nm) | HTM | Concentration HTM (wt.-%) | p-dopant | Concentration p-dopant (wt.-%) | Thickness (nm) |
| Example 1-1 | F3 | 98 | MC -10 | 2 | 10 | F3 | 29 | ET M-1 | 98 | Yb | 2 | 8 | F3 | 75 | MC -10 | 25 | 10 |
| Comparative Example 1-1 | F3 | 98 | CC-2 | 2 | 10 | F3 | 29 | ET M-1 | 98 | Yb | 2 | 8 | F3 | 75 | CC-2 | 25 | 10 |
| Comparative Example 1-2 | F3 | 98 | CC-1 | 2 | 10 | F3 | 29 | ET M-1 | 98 | Yb | 2 | 8 | F3 | 75 | CC-1 | 25 | 10 |
| Example 1-2 | F3 | 98 | MC -10 | 2 | 10 | F3 | 29 | ET M-1 | 99 | Li | 1 | 8 | F3 | 75 | MC -10 | 25 | 10 |
| Comparative Example 1-3 | F3 | 98 | CC-2 | 2 | 10 | F3 | 29 | ET M-1 | 99 | Li | 1 | 8 | F3 | 75 | CC-2 | 25 | 10 |
| Comparative Example 1-4 | F3 | 98 | CC-1 | 2 | 10 | F3 | 29 | ET M-1 | 99 | Li | 1 | 8 | F3 | 75 | CC-1 | 25 | 10 |
| Comparative Example 1-5 | F3 | 98 | MC -10 | 2 | 10 | F3 | 29 | ET M-1 | 88 | Mg | 12 | 8 | F3 | 75 | MC -10 | 25 | 10 |
| Comparative Example 1-6 | F18 | 98 | MC -10 | 2 | 10 | F18 | 29 | ET M-1 | 88 | Mg | 12 | 8 | F18 | 75 | MC -10 | 25 | 10 |
| Example 1-3 | F18 | 98 | MC -10 | 2 | 10 | F18 | 29 | ET M-1 | 98 | Yb | 2 | 8 | F18 | 75 | MC -10 | 25 | 10 |
| Comparative Example 1-7 | F18 | 98 | CC-2 | 2 | 10 | F18 | 29 | ET M-1 | 98 | Yb | 2 | 8 | F18 | 75 | CC-2 | 25 | 10 |
| Comparative Example 1-8 | F18 | 98 | CC-1 | 2 | 10 | F18 | 29 | ET M-1 | 98 | Yb | 2 | 8 | F18 | 75 | CC-1 | 25 | 10 |
| Example 1-4 | F18 | 98 | MC -10 | 2 | 10 | F18 | 29 | ET M-1 | 99 | Li | 1 | 8 | F18 | 75 | MC -10 | 25 | 10 |
| Comparative Example 1-9 | F18 | 98 | CC-2 | 2 | 10 | F18 | 29 | ET M-1 | 99 | Li | 1 | 8 | F18 | 75 | CC-2 | 25 | 10 |
| Comparative Example 1-10 | F1 8 | 98 | CC-1 | 2 | 10 | F1 8 | 29 | ET M-1 | 99 | Li | 1 | 8 | F1 8 | 75 | CC-1 | 25 | 10 |
| Example 1-5 | F3 | 98 | PD-1 | 2 | 10 | F3 | 29 | ET M-1 | 98 | Yb | 2 | 8 | F3 | 75 | MC -10 | 25 | 10 |
| Exampl e 1-6 | F3 | 98 | PD-1 | 2 | 10 | F3 | 29 | ET M-1 | 99 | Li | 1 | 8 | F3 | 75 | MC -10 | 25 | 10 |
| Example 1-7 | F1 8 | 98 | PD-1 | 2 | 10 | F1 8 | 29 | ET M-1 | 98 | Yb | 2 | 8 | F1 8 | 75 | MC -10 | 25 | 10 |
| Example 1-8 | F1 8 | 98 | PD-1 | 2 | 10 | F1 8 | 29 | ET M-1 | 99 | Li | 1 | 8 | F1 8 | 75 | MC -10 | 25 | 10 |
| Example 1-9 | F1 8 | 98 | PD-1 | 2 | 10 | F1 8 | 29 | ET M-1 | 98 | Yb | 2 | 8 | F1 8 | 81 | MC -11 | 19 | 10 |

**Table 3: Performance of an organic electronic device comprising a charge generation layer (CGL)**

| | Voltage at 10 mA/cm² [V] | EQE at 10 mA/cm² [%] | LT97 at 30 mA/cm² [h] | Voltage rise (30mA/cm², 1-100h) [V] |
|---|---|---|---|---|
| Example 1-1 | 6.88 | 31.48 | 140 | 0.251 |
| Comparative Example 1-1 | >10 | n.d. ¹⁾ | n.d. | n.d. |
| Comparative Example 1-2 | >10 | n.d. | n.d. | n.d. |
| Example 1-2 | 6.63 | 31.42 | 119 | 0.100 |
| Comparative Example 1-3 | >10 | n.d. | n.d. | n.d. |
| Comparative Example 1-4 | >10 | n.d. | n.d. | n.d. |
| Comparative Example 1-5 | 8.05 | 29.56 | 51 | 0.403 |
| Comparative Example 1-6 | 8.19 | 31.82 | 48 | 0.323 |
| Example 1-3 | 6.98 | 33.04 | 115 | 0.214 |
| Comparative Example 1-7 | >10 | n.d. | n.d. | n.d. |
| Comparative Example 1-8 | >10 | n.d. | n.d. | n.d. |
| Example 1-4 | 6.87 | 32.45 | 79 | 0.132 |
| Comparative Example 1-9 | >10 | n.d. | n.d. | n.d. |
| Comparative Example 1-10 | 9.87 | 28.64 | n.d. | n.d. |
| Example 1-5 | 6.88 | 31.24 | 140 | 0.242 |
| Example 1-6 | 6.63 | 31.15 | 120 | 0.126 |
| Example 1-7 | 6.98 | 32.82 | 112 | 0.218 |
| Example 1-8 | 6.87 | 32.33 | 75 | 0.138 |
| Example 1-9 | 6.9 | 30.82 | 121 | 0.363 |

| | | | | |
|---|---|---|---|---|
| ¹⁾ n.d. = not determined | | | | |

Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims. Furthermore, reference signs used in the description and claims do not limit the scope of the invention as claimed.

## Claims

1. An organic electronic device (100) comprising an anode layer (120), a cathode layer (190), a hole injection layer (130), a first emission layer (150) and a charge generation layer (135, 185), wherein the charge generation layer (135, 185) comprises a p-type charge generation layer (135) and a n-type charge generation layer (185),
- the n-type charge generation layer (185) comprising an organic electron transport compound and a metal dopant, wherein the metal dopant is selected from an alkali or rare earth metal;
- the p-type charge generation layer (135) comprises:
- an organic hole transport compound, and
- a compound of formula (I):
M^{n⊕}(L^{⊖})ₙ(AL)ₘ (I),
wherein
M is a metal ion;
n is the valency of M and selected from 1 to 3;
L is a ligand of formula (II) 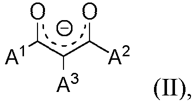
wherein
A¹ and A² are independently selected from substituted or unsubstituted C₁ to C₁₂ alkyl, substituted or unsubstituted C₆ to C₁₂ aryl, substituted or unsubstituted C₃ to C₁₂ heteroaryl;
wherein the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, SO₂R¹, halogen, F or CN,
wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
A³ is selected from H, D, CN, substituted or unsubstituted C₁ to C₄ alkyl, partially or perfluorinated C₁ to C₄ alkyl, substituted or unsubstituted C₆ to C₁₀ aryl, substituted or unsubstituted C₃ to C₉ heteroaryl, or whereby A³ form a substituted or unsubstituted cyclic structure with either A¹ or A²,
wherein the one or more substituent of A³ or the substituents of the substituted cyclic structure are independently selected from halogen, F, Cl, CN, CF₃;
AL is an ancillary ligand which coordinates to the metal M;
m is an integer selected from 0 to 2;
wherein
the hole injection layer (130), the first emission layer (150) and the charge generation layer (135, 185) are arranged between the anode layer (120) and the cathode layer (190), and
the n-type charge generation layer (185) is arranged closer to the anode layer (120) and the p-type charge generation layer (135) is arranged closer to the cathode layer (190).

2. The organic electronic device (100) according to claim 1, wherein A³ is selected form a cyclic structure with either A¹ or A² of 5- or 6- membered aliphatic or aromatic rings, whereby the ring optional comprise one or more heteroatoms, preferably selected from the group comprising N, O and S.

3. The organic electronic device (100) according to claim 1 or 2, wherein
A¹ and A² are independently selected from substituted or unsubstituted C₁ to C₁₂ alkyl, substituted or unsubstituted C₆ to C₁₂ aryl, substituted or unsubstituted C₃ to C₁₂ heteroaryl, preferably substituted C₁ to C₁₂ alkyl, substituted C₆ to C₁₂ aryl, substituted C₃ to C₁₂ heteroaryl;
wherein the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, halogen, F or CN,
wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
A³ is selected from H, D,CN, CH₃, substituted or unsubstituted C₆ aryl or substituted or unsubstituted C₅ heteroaryl, preferably H or CN;
wherein the one or more substituent of A³ are independently selected from halogen, F, Cl, CN, CF3.

4. The organic electronic device (100) according to any of the preceding claims 1 to 3, wherein the sum of A¹ and A² comprise ≥ 3 carbon atoms and ≤ 30 carbon atoms.

5. The organic electronic device (100) according to any of the preceding claims 1 to 4, wherein the organic hole transport compound is selected from at least one organic compound comprising 1 to 3 triarylamine moieties, and 8 to 12 aromatic or heteroaromatic rings, at least one arylamine compound, diarylamine compound, triarylamine compound, a compound of formula (IIIa) or a compound of formula (IIIb): 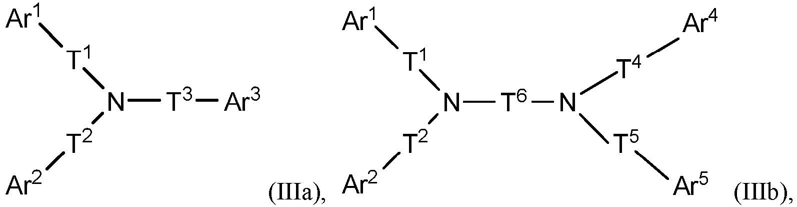 wherein:
T¹, T², T³, T⁴ and T⁵ are independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein R² is selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl; and
wherein the molecular weight Mw of the organic hole transport compound is in the range of ≥ 400 and ≤ 2000 g/mol.

6. The organic electronic device (100) according to any of the preceding claims 1 to 5, wherein the ligand L of formula I comprising per ligand L at least 14 covalently bound atoms; or
the ligand L of formula I comprising:
- at least three carbon atoms, alternatively at least four carbon atoms, and/or
- at least two oxygen atoms or one oxygen and one nitrogen atom, two to four oxygen atoms, two to four oxygen atoms and zero to two nitrogen atoms, and/or
- at least one or more groups selected from halogen, F, CN, substituted or unsubstituted C₁ to C₆ alkyl, substituted or unsubstituted C₁ to C₆ alkoxy, alternatively two or more groups selected from halogen, F, CN, substituted or unsubstituted C₁ to C₆ alkyl, substituted or unsubstituted C₁ to C₆ alkoxy, at least one or more groups selected from halogen, F, CN, substituted C₁ to C₆ alkyl, substituted C₁ to C₆ alkoxy, alternatively two or more groups selected from halogen, F, CN, perfluorinated C₁ to C₆ alkyl, perfluorinated C₁ to C₆ alkoxy, one or more groups selected from substituted or unsubstituted C₁ to C₆ alkyl, substituted or unsubstituted C₆ to C₁₂ aryl, and/or substituted or unsubstituted C₃ to C₁₂ heteroaryl,
wherein the substituents are selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR³, COOR³, halogen, F or CN;
wherein R³ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

7. The organic electronic device (100) according to any of the preceding claims 1 to 6, wherein A¹ and/or A² are selected from the following Formulas D1 to D68: 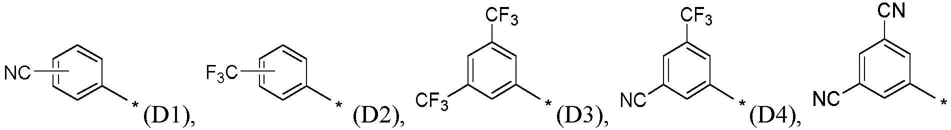 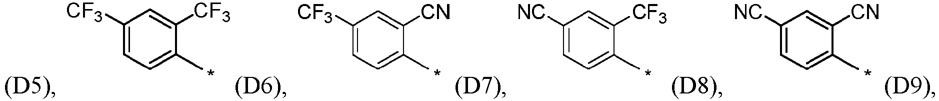 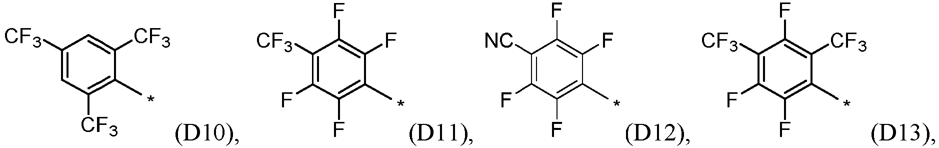 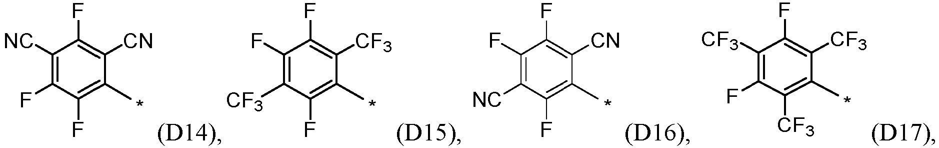 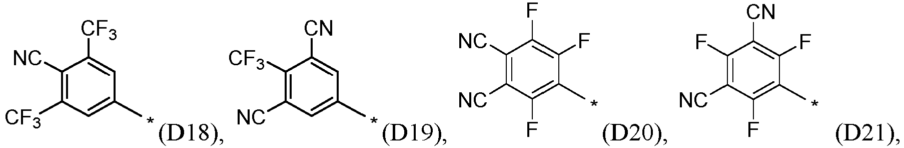 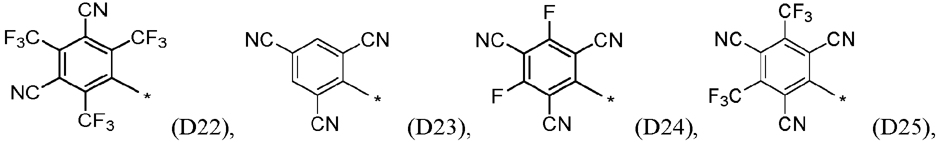 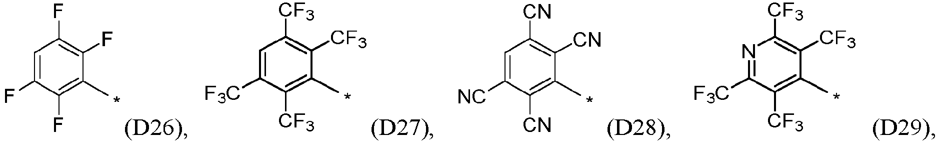 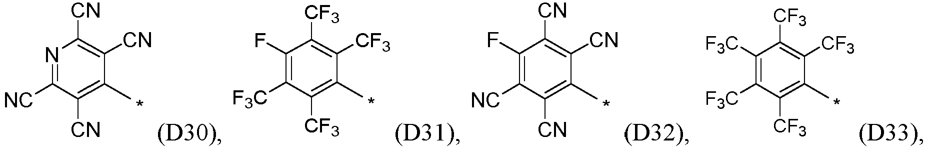 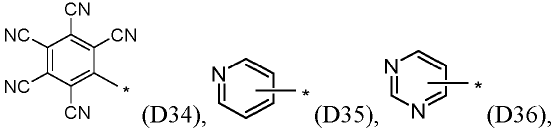 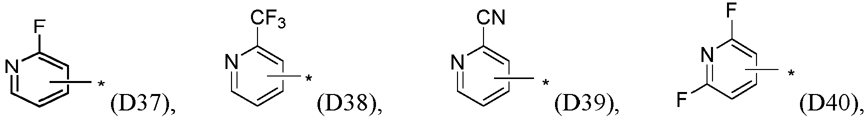 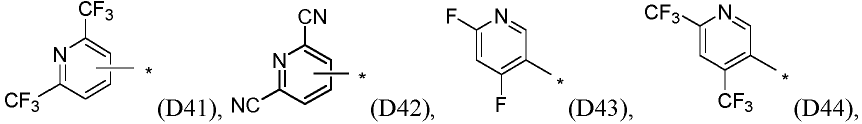 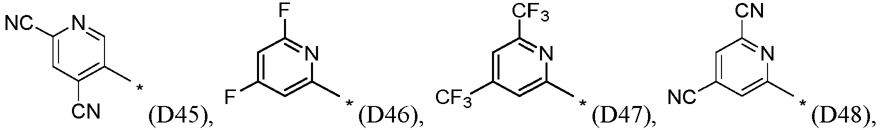 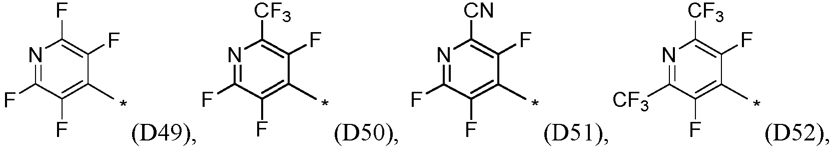 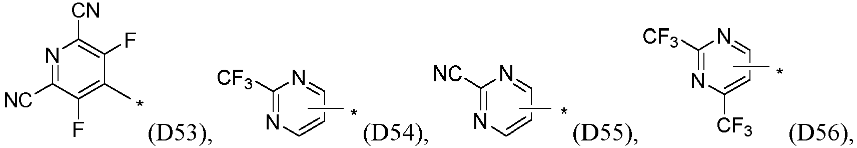 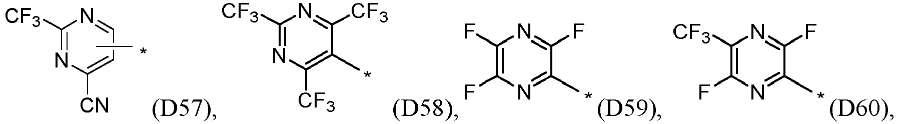 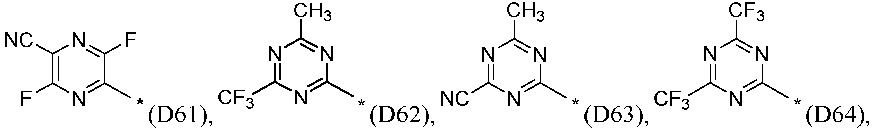 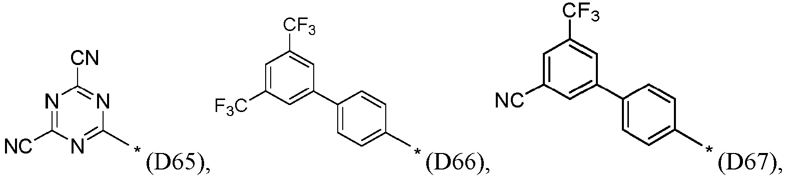 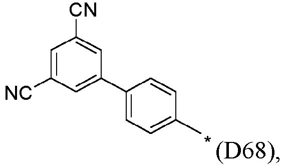 wherein the "*" denotes the binding position.

8. The organic electronic device (100) according to any of the preceding claims 1 to 7, wherein the ligand L of formula (I) is selected from G1 to G73:

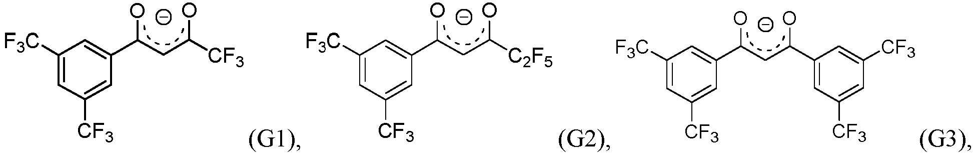

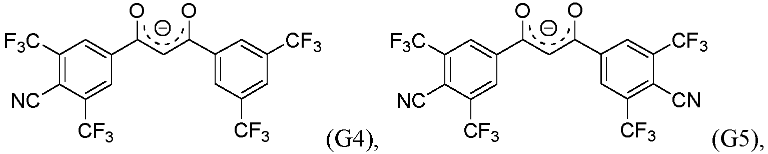

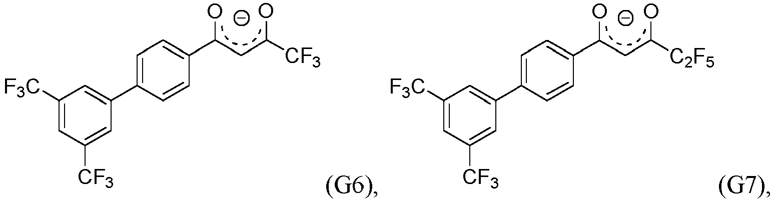

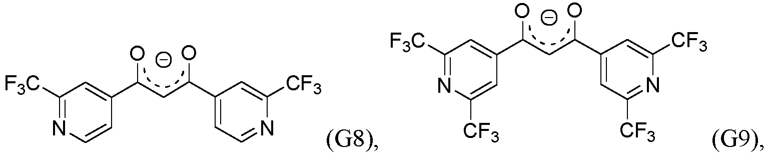

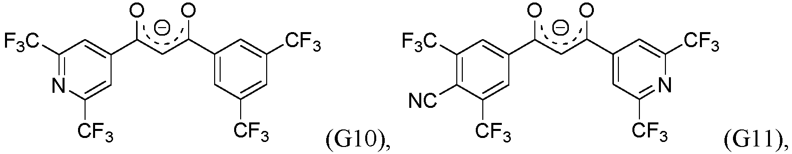

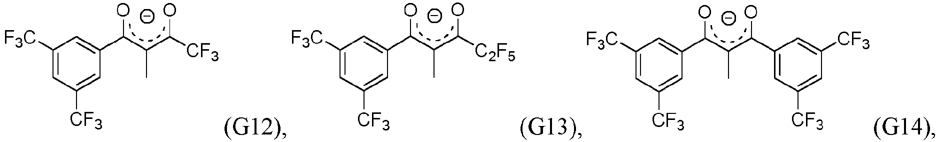

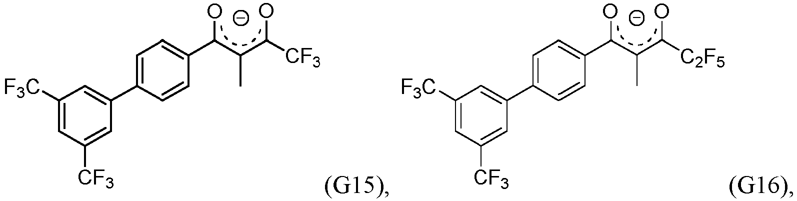

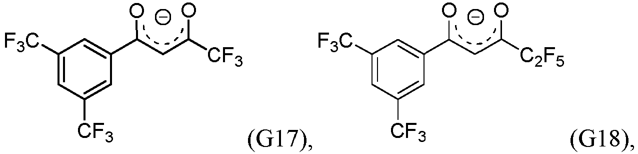

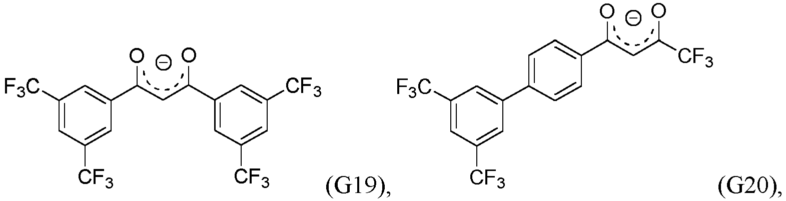

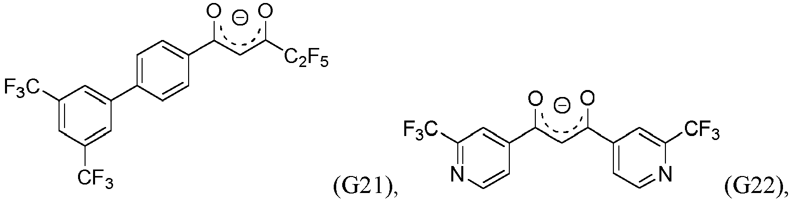

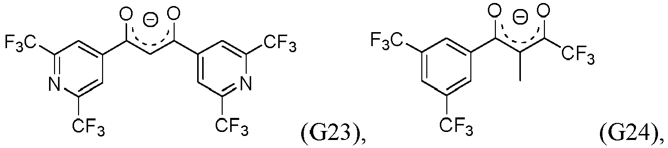

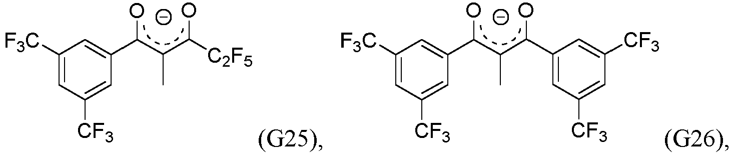

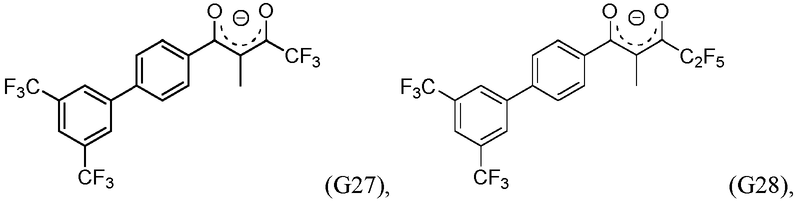

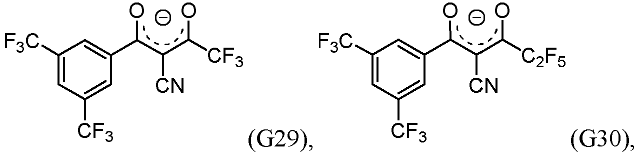

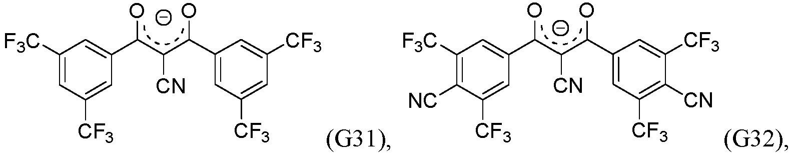

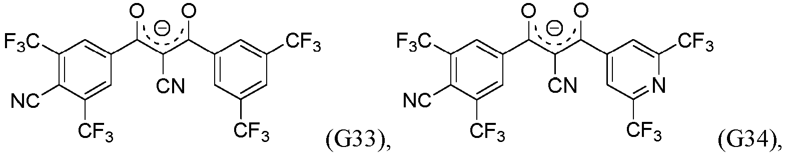

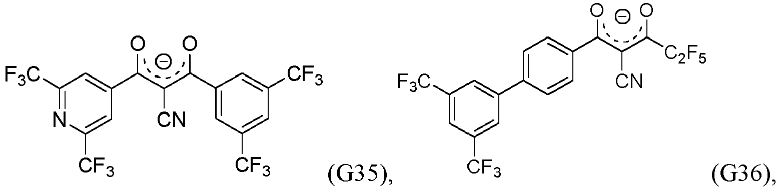

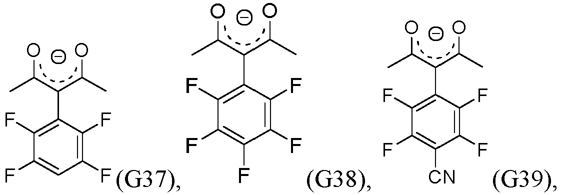

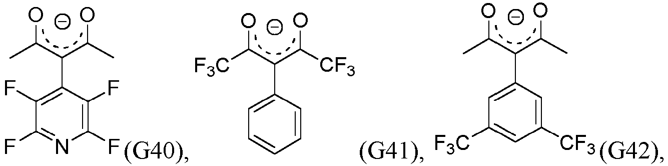

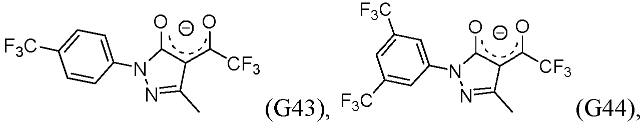

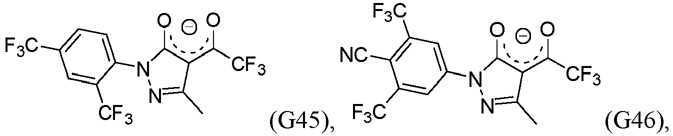

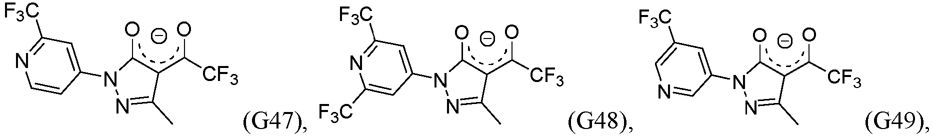

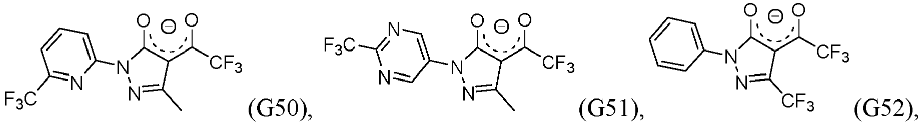

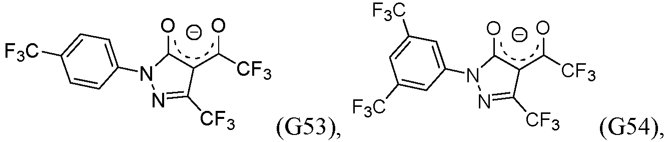

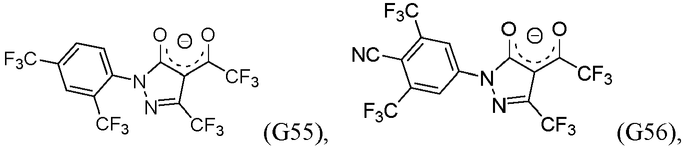

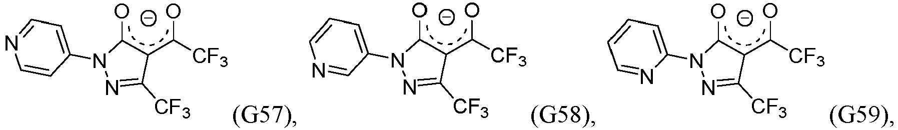

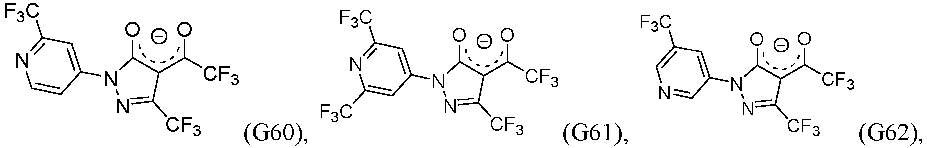

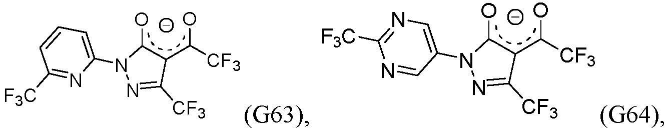

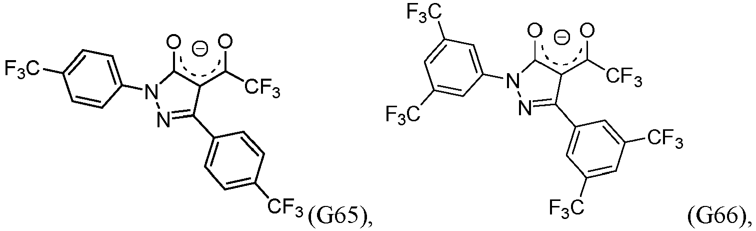

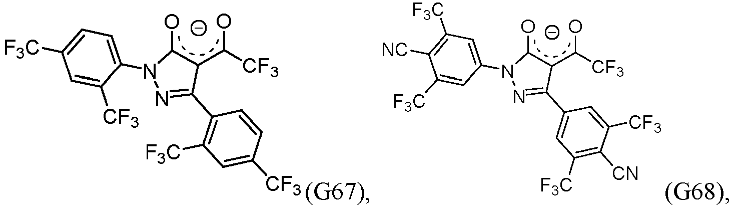

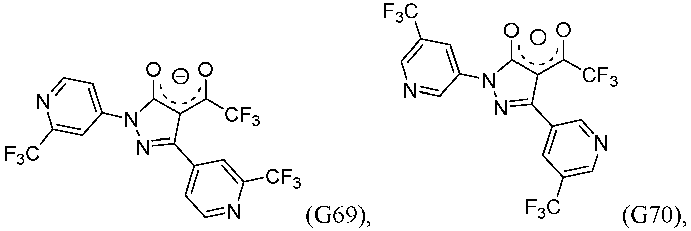

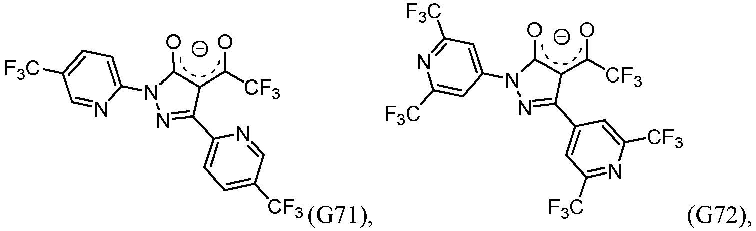

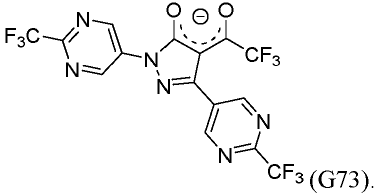

9. The organic electronic device (100) according to any of the preceding claims 1 to 8, wherein the compound of formula (I) is selected from the group of MC-1 to MC-17:

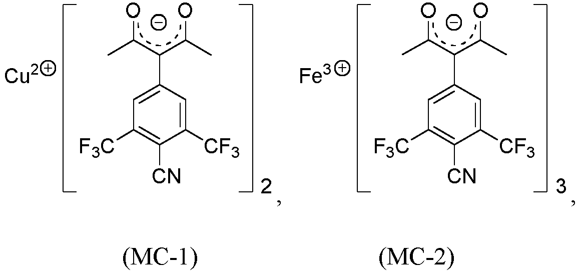

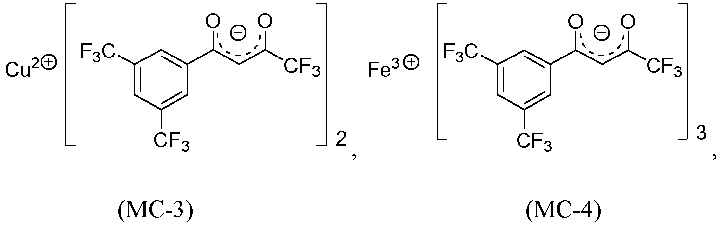

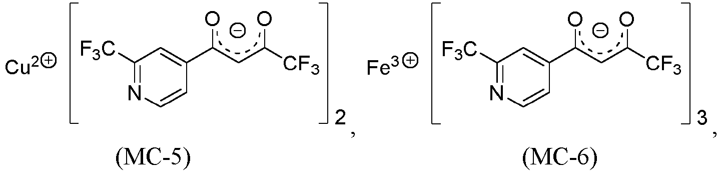

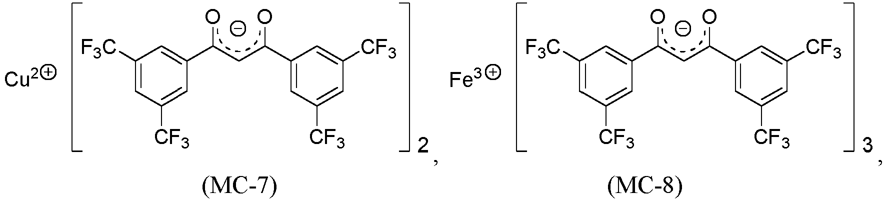

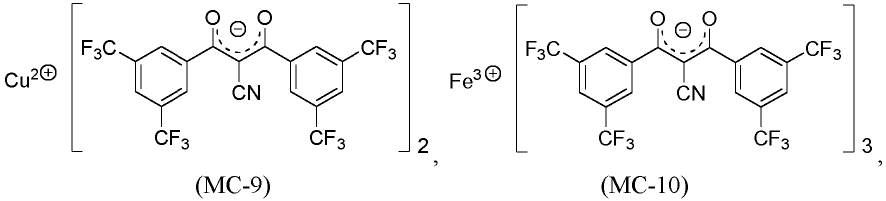

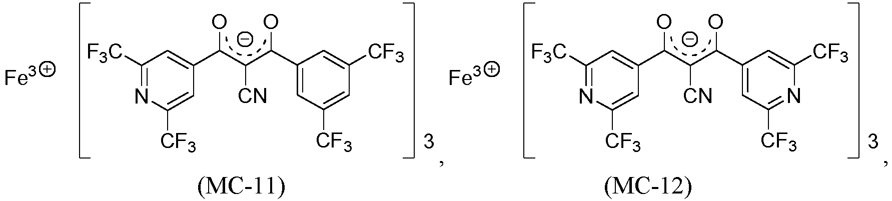

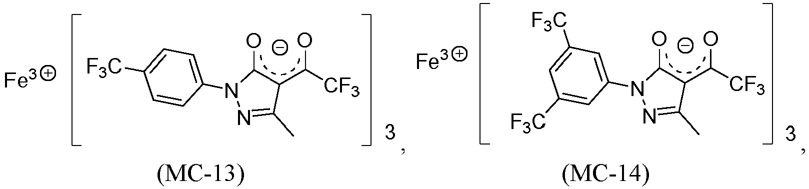

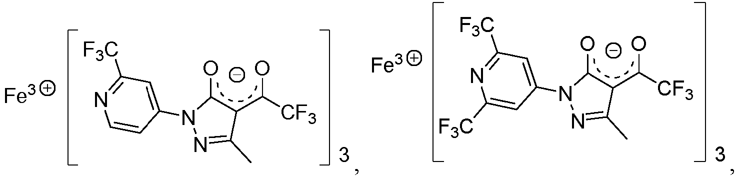

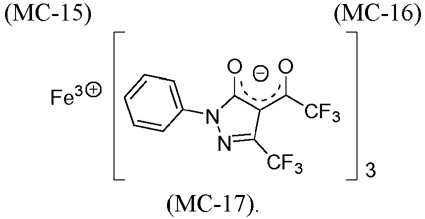

10. The organic electronic device (100) according to any of the preceding claims 1 to 9, wherein the hole injection layer (130) comprises a compound of formula (I).

11. The organic electronic device (100) according to any of the preceding claims 1 to 10, wherein the first emission layer (150) comprises a compound of formula (VII) and an organic emitter host compound: 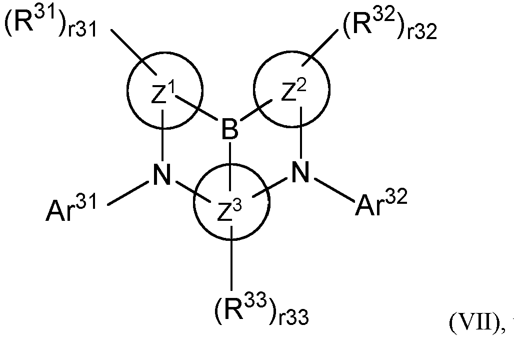 wherein
Z¹, Z² and Z³ are the same as or selected different from each other, and are each independently selected from the group comprising a monocyclic to polycyclic aromatic hydrocarbon ring or monocyclic to polycyclic aromatic hetero ring;
Ar³¹ and Ar³² are the same as or selected different from each other, and are each independently and are each independently selected from the group comprising a substituted or unsubstituted alkyl group, a substituted or unsubstituted aryl group, or a substituted or unsubstituted heteroaryl group, or are bonded to an adjacent substituent to form a substituted or unsubstituted aromatic ring or a substituted or unsubstituted aliphatic ring;
R³¹, R³² and R³³ are the same as or selected different from each other, and are each independently selected from the group comprising hydrogen, deuterium, a substituted or unsubstituted alkyl group, a substituted or unsubstituted silyl group, a substituted or unsubstituted amine group, a substituted or unsubstituted aryl group, or a substituted or unsubstituted heteroaryl group, or adjacent substituents are bonded to each other to form a substituted or unsubstituted aromatic ring or a substituted or unsubstituted aliphatic ring,
wherein one or more substituents selected from the group consisting of deuterium, an alkyl group having 1 to 6 carbon atoms, an alkyl silyl group having 1 to 30 carbon atoms, an aryl silyl group having 6 to 50 carbon atoms, an alkylamine group having 1 to 30 carbon atoms, an alkylarylamine group
having 1 to 50 carbon atoms, an arylamine group having 6 to 50 carbon atoms, an aryl group having 6 to 30 carbon atoms, and a heteroaryl group having 2 to 30 carbon atoms, or a substituent to which two or more substituents selected from the group are linked, or adjacent substituents are bonded to each other to form an aliphatic hydrocarbon ring having 3 to 60 carbon atoms, which is unsubstituted or substituted with the substituent;
r31, r32 and r33 are each an integer from 0, 1, 2, 3 or 4, and when r31 to r33 are 2 or higher, substituents in the parenthesis are the same as or selected different from each other.

12. The organic electronic device (100) according to any of the preceding claims 1 to 11, wherein the organic emitter host compound comprises:
- at least one condensed aromatic ring system comprising of at least ≥ 3 to ≤ 5 condensed aromatic rings, and
- at least ≥ 3 to ≤ 7 aromatic rings and/or at least ≥ 3 to ≤ 7 heteroaromatic rings,
wherein at least ≥ 2 to ≤ 7 of the aromatic rings and/or at least ≥ 3 to ≤ 7 of the heteroaromatic rings are condensed to form a fused aromatic ring system and/or fused heteroaromatic ring systems, and
wherein the molecular weight Mw of the organic emitter host compound is in the range of ≥ 400 g/mol and ≤ 2000 g/mol.

13. The organic electronic device (100) according to any of the preceding claims 1 to 12, wherein the organic emitter host compound has the formula (VIII): 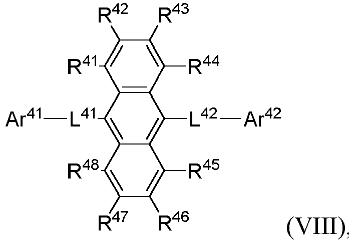 , wherein
Ar⁴¹ and Ar⁴² are independently selected from substituted or unsubstituted C₆ to C₂₄ aryl, substituted or unsubstituted C₃ to C₂₄ heteroaryl;
L⁴¹ and L⁴² are independently selected from a direct bond or substituted or unsubstituted C₆ to C₂₄ arylene, substituted or unsubstituted C₃ to C₂₄ heteroarylene;
R⁴¹ to R⁴⁸ are independently selected from H, D, substituted or unsubstituted C₁ to C₁₂ alkyl, substituted or unsubstituted C₆ to C₁₉ aryl, substituted or unsubstituted C₃ to C₁₂ heteroaryl;
wherein
the substituents on Ar⁴¹, Ar⁴², L⁴¹, L⁴², R⁴¹ to R⁴⁸ are independently selected from D, C₆ to C₁₀ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, halogen, F or CN.

14. The organic electronic device (100) according to any of the preceding claims 1 to 13, wherein light is emitted through the anode layer (190).

15. A display device comprising an organic electronic device (100) according to any of the preceding claims 1 to 14.

## Patentansprüche

1. Organische elektronische Vorrichtung (100) umfassend eine Anodenschicht (120), eine Kathodenschicht (190), eine Lochinjektionsschicht (130), eine erste Emissionsschicht (150) und eine Ladungserzeugungsschicht (135, 185), wobei die Ladungserzeugungsschicht (135, 185) eine p-Typ-Ladungserzeugungsschicht (135) und eine n-Typ-Ladungserzeugungsschicht (185) umfasst,
- die n-Typ-Ladungserzeugungsschicht (185) eine organische Elektronentransportverbindung und einen Metalldotierstoff umfasst, wobei der Metalldotierstoff ausgewählt ist aus einem Alkali- oder Seltenerdmetall;
- die p-Typ-Ladungserzeugungsschicht (135) umfasst:
- eine organische Lochtransportverbindung und
- eine Verbindung der Formel (I):
M^{n⊕}(L^{⊖})ₙ(AL)ₘ (I),
wobei
M ein Metallion ist;
n die Wertigkeit von M ist und ausgewählt ist aus 1 bis 3;
L ein Ligand der Formel (II) ist 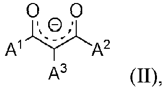
wobei
A¹ und A² unabhängig ausgewählt sind aus substituiertem oder unsubstituiertem C₁- bis C₁₂-Alkyl, substituiertem oder unsubstituiertem C₆- bis C₁₂-Aryl, substituiertem oder unsubstituiertem C₃- bis C₁₂-Heteroaryl;
wobei die Substituenten von A¹ und A² unabhängig ausgewählt sind aus D, C₆-Aryl, C₃- bis C₉-Heteroaryl, C₁- bis C₆-Alkyl, C₁-bis C₆-Alkoxy, verzweigtem C₃- bis C₆-Alkyl, cyclischem C₃- bis C₆-Alkyl, verzweigtem C₃-bis C₆-Alkoxy, cyclischem C₃- bis C₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₆-Alkyl, teil- oder perdeuteriertem C₁- bis C₆-Alkoxy, COR¹, COOR¹, SO₂R¹, Halogen, F oder CN,
wobei R¹ ausgewählt ist aus C₆-Aryl, C₃- bis C₉-Heteroaryl, C₁- bis C₆-Alkyl, C₁- bis C₆-Alkoxy, verzweigtem C₃- bis C₆-Alkyl, cyclischem C₃- bis C₆-Alkyl, verzweigtem C₃-bis C₆-Alkoxy, cyclischem C₃- bis C₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₆-Alkyl, teil- oder perdeuteriertem C₁- bis C₆-Alkoxy;
A³ ausgewählt ist aus H, D, CN, substituiertem oder unsubstituiertem C₁- bis C4-Alkyl, teil- oder perfluoriertem C₁- bis C4-Alkyl, substituiertem oder unsubstituiertem C₆- bis C₁₀-Aryl, substituiertem oder unsubstituiertem C₃- bis C₉-Heteroaryl, oder wobei A³ eine substituierte oder unsubstituierte cyclische Struktur mit entweder A¹ oder A² bildet,
wobei der eine oder die mehreren Substituenten von A³ oder die Substituenten der substituierten cyclischen Struktur unabhängig ausgewählt sind aus Halogen, F, Cl, CN, CF₃;
AL ein Hilfsligand ist, der an das Metall M koordiniert;
m eine ganze Zahl ausgewählt aus 0 bis 2 ist;
wobei
die Lochinjektionsschicht (130), die erste Emissionsschicht (150) und die Ladungserzeugungsschicht (135, 185) zwischen der Anodenschicht (120) und der Kathodenschicht (190) angeordnet sind und die n-Typ-Ladungserzeugungsschicht (185) näher an der Anodenschicht (120) angeordnet ist und die p-Typ-Ladungserzeugungsschicht (135) näher an der Kathodenschicht (190) angeordnet ist.

2. Organische elektronische Vorrichtung (100) nach Anspruch 1, wobei A³ ausgewählt ist aus einer zyklischen Struktur mit entweder A¹ oder A² von 5- oder 6-gliedrigen aliphatischen oder aromatischen Ringen, wobei der Ring gegebenenfalls ein oder mehrere Heteroatome, vorzugsweise ausgewählt aus der Gruppe umfassend N, O und S, umfasst.

3. Organische elektronische Vorrichtung (100) nach Anspruch 1 oder 2, wobei
A¹ und A² unabhängig ausgewählt sind aus substituiertem oder unsubstituiertem C₁- bis C₁₂-Alkyl, substituiertem oder unsubstituiertem C₆- bis C₁₂-Aryl, substituiertem oder unsubstituiertem C₃- bis C₁₂-Heteroaryl, vorzugsweise substituiertem C₁- bis C₁₂-Alkyl, substituiertem C₆- bis C₁₂-Aryl, substituiertem C₃- bis C₁₂-Heteroaryl; wobei die Substituenten von A¹ und A² unabhängig ausgewählt sind aus D, C₆-Aryl, C₃- bis C₉-Heteroaryl, C₁- bis C₆-Alkyl, C₁-bis C₆-Alkoxy, verzweigtem C₃- bis C₆-Alkyl, cyclischem C₃- bis C₆-Alkyl, verzweigtem C₃-bis C₆-Alkoxy, cyclischem C₃- bis C₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₆-Alkyl, teil- oder perdeuteriertem C₁- bis C₆-Alkoxy, COR¹, COOR¹, Halogen, F oder CN, wobei R¹ ausgewählt ist aus C₆-Aryl, C₃- bis C₉-Heteroaryl, C₁- bis C₆-Alkyl, C₁- bis C₆-Alkoxy, verzweigtem C₃- bis C₆-Alkyl, cyclischem C₃- bis C₆-Alkyl, verzweigtem C₃-bis C₆-Alkoxy, cyclischem C₃- bis C₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₆-Alkyl, teil- oder perdeuteriertem C₁- bis C₆-Alkoxy;
A³ ausgewählt ist aus H, D, CN, CH₃, substituiertem oder unsubstituiertem C₆-Aryl oder substituiertem oder unsubstituiertem C₅-Heteroaryl, vorzugsweise H oder CN; wobei der eine oder die mehreren Substituenten von A³ unabhängig ausgewählt sind aus Halogen, F, Cl, CN, CF3.

4. Organische elektronische Vorrichtung (100) nach einem der vorstehenden Ansprüche 1 bis 3, wobei die Summe von A¹ und A² ≥ 3 Kohlenstoffatome und ≤ 30 Kohlenstoffatome umfasst.

5. Organische elektronische Vorrichtung (100) nach einem der vorstehenden Ansprüche 1 bis 4, wobei die organische Lochtransportverbindung ausgewählt ist aus wenigstens einer organischen Verbindung umfassend 1 bis 3 Triarylamineinheiten, und 8 bis 12 aromatische oder heteroaromatischen Ringe, wenigstens einer Arylaminverbindung, Diarylaminverbindung, Triarylaminverbindung, einer Verbindung der Formel (IIIa) oder einer Verbindung der Formel (IIIb): 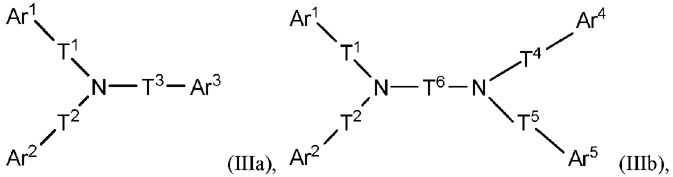 wobei:
T¹, T², T³, T⁴ und T⁵ unabhängig ausgewählt sind aus einer Einfachbindung, Phenylen, Biphenylen, Terphenylen oder Naphthenylen, vorzugsweise einer Einfachbindung oder Phenylen;
T⁶ Phenylen, Biphenylen, Terphenylen oder Naphthenylen ist;
Ar¹, Ar², Ar³, Ar⁴ und Ar⁵ unabhängig ausgewählt sind aus substituiertem oder unsubstituiertem C₆- bis C₂₀-Aryl oder substituiertem oder unsubstituiertem C₃- bis C₂₀-Heteroarylen, substituiertem oder unsubstituiertem Biphenylen, substituiertem oder unsubstituiertem Fluoren, substituiertem 9-Fluoren, substituiertem 9,9-Fluoren, substituiertem oder unsubstituiertem Naphthalin, substituiertem oder unsubstituiertem Anthracen, substituiertem oder unsubstituiertem Phenanthren, substituiertem oder unsubstituiertem Pyren, substituiertem oder unsubstituiertem Perylen, substituiertem oder unsubstituiertem Triphenylen, substituiertem oder unsubstituiertem Tetracen, substituiertem oder unsubstituiertem Tetraphen, substituiertem oder unsubstituiertem Dibenzofuran, substituiertem oder unsubstituiertem Dibenzothiophen, substituiertem oder unsubstituiertem Xanthen, substituiertem oder unsubstituiertem Carbazol, substituiertem 9-Phenylcarbazol, substituiertem oder unsubstituiertem Azepin, substituiertem oder unsubstituiertem Dibenzo[b,f]azepin, substituiertem oder unsubstituiertem 9,9'-Spirobi[fluoren], substituiertem oder unsubstituiertem Spiro[fluoren-9,9'-xanthen] oder einem substituierten oder unsubstituierten aromatischen verschmolzenen Ringsystem umfassend wenigstens drei substituierte oder unsubstituierte aromatische Ringe ausgewählt aus der Gruppe umfassend substituierte oder unsubstituierte nichthetero-, substituierte oder unsubstituierte hetero-5-gliedrige Ringe, substituierte oder unsubstituierte 6-gliedrige Ringe und/oder substituierte oder unsubstituierte 7-gliedrige Ringe, substituiertes oder unsubstituiertes Fluoren, oder ein verschmolzenes Ringsystem umfassend 2 bis 6 substituierte oder unsubstituierte 5- bis 7-gliedrige Ringe, und wobei die Ringe ausgewählt sind aus der Gruppe umfassend (i) einen ungesättigten 5-bis 7-gliedrigen Ring eines Heterocyclus, (ii) 5- bis 6-Glieder eines aromatischen Heterocyclus, (iii) ungesättigten 5- bis 7-gliedrigen Ring eines Nicht-Heterocyclus, (iv) 6-gliedrigen Ring eines aromatischen Nicht-Heterocyclus;
wobei
die Substituenten von Ar¹, Ar², Ar³, Ar⁴ und Ar⁵ gleich oder verschieden ausgewählt sind aus der Gruppe umfassend H, D, F, C(-O)R², CN, Si(R²)₃, P(-O)(R²)₂, OR², S(-O)R², S(-O)₂R², substituiertes oder unsubstituiertes geradkettiges Alkyl mit 1 bis 20 Kohlenstoffatomen, substituiertes oder unsubstituiertes verzweigtes Alkyl mit 1 bis 20 Kohlenstoffatomen, substituiertes oder unsubstituiertes cyclisches Alkyl mit 3 bis 20 Kohlenstoffatomen, substituierte oder unsubstituierte Alkenyl- oder Alkinylgruppen mit 2 bis 20 Kohlenstoffatomen, substituierte oder unsubstituierte Alkoxygruppen mit 1 bis 20 Kohlenstoffatomen, substituierte oder unsubstituierte aromatische Ringsysteme mit 6 bis 40 aromatischen Ringatomen und substituierte oder unsubstituierte heteroaromatische Ringsysteme mit 5 bis 40 aromatischen Ringatomen, unsubstituiertes C₆- bis C₁₈-Aryl, unsubstituiertes C₃-bis C₁₈-Heteroaryl, ein anelliertes Ringsystem mit 2 bis 6 unsubstituierten 5- bis 7-gliedrigen Ringen, wobei die Ringe ausgewählt sind aus der Gruppe umfassend einen ungesättigten 5- bis 7-gliedrigen Ring eines Heterocyclus, einen 5- bis 6-gliedrigen Ring eines aromatischen Heterocyclus, einen ungesättigten 5- bis 7-gliedrigen Ring eines Nichtheterocyclus und einen 6-gliedrigen Ring eines aromatischen Nichtheterocyclus,
wobei R² ausgewählt ist aus H, D, geradkettigem Alkyl mit 1 bis 6 Kohlenstoffatomen, verzweigtem Alkyl mit 1 bis 6 Kohlenstoffatomen, cyclischem Alkyl mit 3 bis 6 Kohlenstoffatomen, Alkenyl- oder Alkinylgruppen mit 2 bis 6 Kohlenstoffatomen, C₆- bis C₁₈-Aryl oder C₃- bis C₁₈-Heteroaryl; und
wobei das Molekulargewicht Mw der organischen Lochtransportverbindung in dem Bereich von ≥ 400 und ≤ 2000 g/mol liegt.

6. Organische elektronische Vorrichtung (100) nach einem der vorstehenden Ansprüche 1 bis 5, wobei der Ligand L der Formel I pro Ligand L wenigstens 14 kovalent gebundene Atome umfasst; oder
der Ligand L der Formel I umfasst:
- wenigstens drei Kohlenstoffatome, alternativ wenigstens vier Kohlenstoffatome, und/oder
- wenigstens zwei Sauerstoffatome oder ein Sauerstoff- und ein Stickstoffatom, zwei bis vier Sauerstoffatome, zwei bis vier Sauerstoffatome und null bis zwei Stickstoffatome, und/oder
- wenigstens eine oder mehrere Gruppen ausgewählt aus Halogen, F, CN, substituiertem oder unsubstituiertem C₁-bis C₆-Alkyl, substituiertem oder unsubstituiertem C₁-bis C₆-Alkoxy, alternativ zwei oder mehr Gruppen ausgewählt aus Halogen, F, CN, substituiertem oder unsubstituiertem C₁- bis C₆-Alkyl, substituiertem oder unsubstituiertem C₁- bis C₆-Alkoxy, wenigstens eine oder mehr Gruppen ausgewählt aus Halogen, F, CN, substituiertem C₁- bis C₆-Alkyl, substituiertem C₁- bis C₆-Alkoxy, alternativ zwei oder mehr Gruppen ausgewählt aus Halogen, F, CN, perfluoriertem C₁- bis C₆-Alkyl, perfluoriertem C₁- bis C₆-Alkoxy, eine oder mehrere Gruppen ausgewählt aus substituiertem oder unsubstituiertem C₁- bis C₆-Alkyl, substituiertem oder unsubstituiertem C₆- bis C₁₂-Aryl und/oder substituiertem oder unsubstituiertem C₃- bis C₁₂-Heteroaryl,
wobei die Substituenten ausgewählt sind aus D, C₆-Aryl, C₃- bis C₉-Heteroaryl, C₁- bis C₆-Alkyl, C₁- bis C₆-Alkoxy, verzweigtem C₃- bis C₆-Alkyl, cyclischem C₃- bis C₆-Alkyl, verzweigtem C₃- bis C₆-Alkoxy, cyclischem C₃- bis C₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₆-Alkyl, teil- oder perdeuteriertem C₁- bis C₆-Alkoxy, COR³, COOR³, Halogen, F oder CN;
wobei R³ ausgewählt ist aus C₆-Aryl, C₃- bis C₉-Heteroaryl, C₁- bis C₆-Alkyl, C₁- bis C₆-Alkoxy, verzweigtem C₃- bis C₆-Alkyl, cyclischem C₃- bis C₆-Alkyl, verzweigtem C₃- bis C₆-Alkoxy, cyclischem C₃- bis C₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₆-Alkyl, teil- oder perdeuteriertem C₁- bis C₆-Alkoxy.

7. Organische elektronische Vorrichtung (100) nach einem der vorstehenden Ansprüche 1 bis 6, wobei A¹ und/oder A² ausgewählt sind aus den folgenden Formeln D1 bis D68: 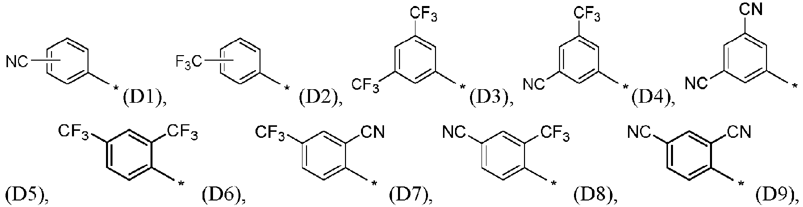 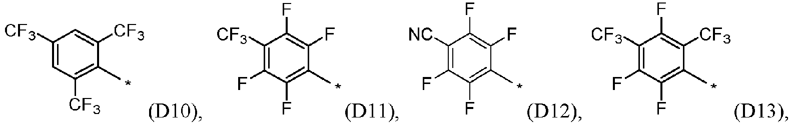 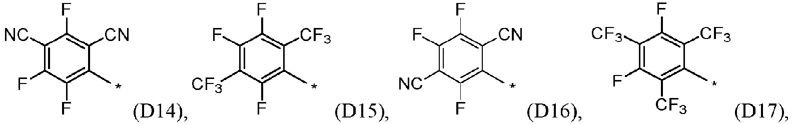 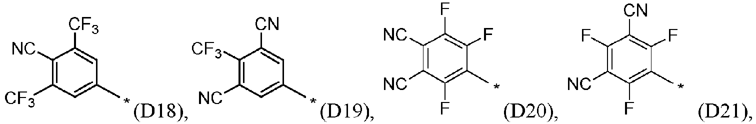 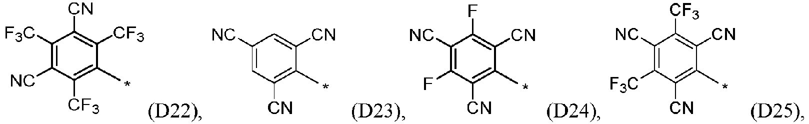 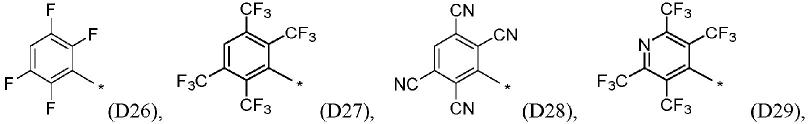 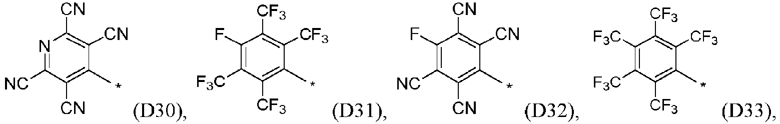 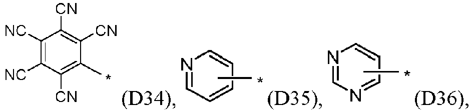 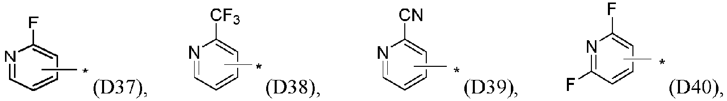 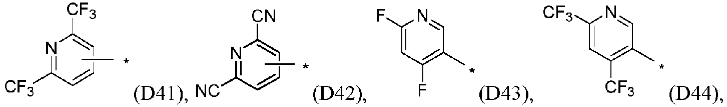 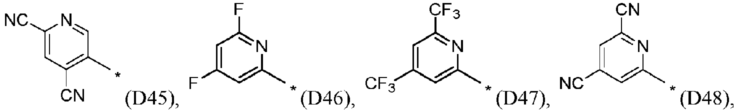 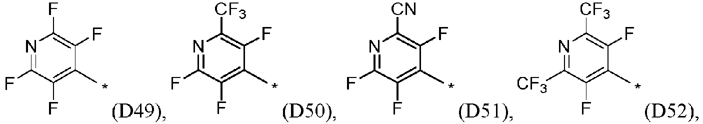 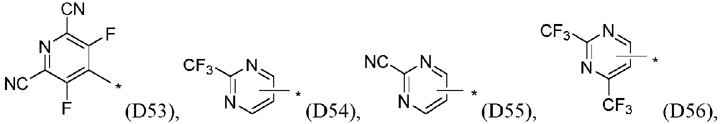 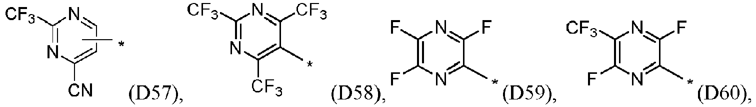 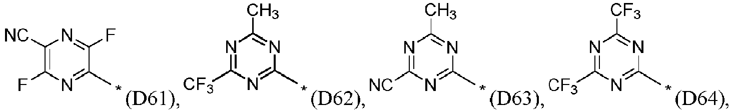 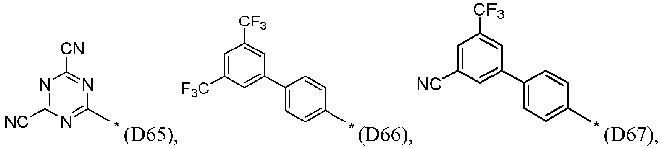 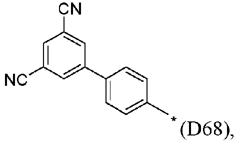 wobei das "*" die Bindungsposition bezeichnet.

8. Organische elektronische Vorrichtung (100) nach einem der vorstehenden Ansprüche 1 bis 7, wobei der Ligand L der Formel (I) ausgewählt ist aus G1 bis G73:

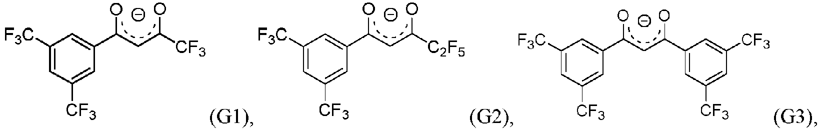

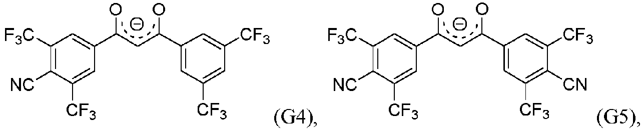

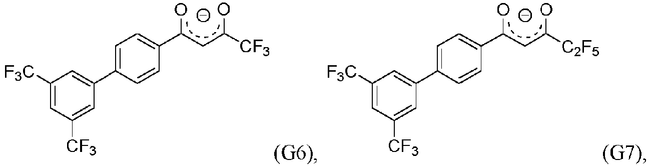

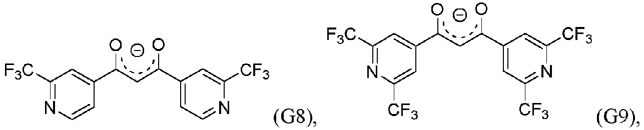

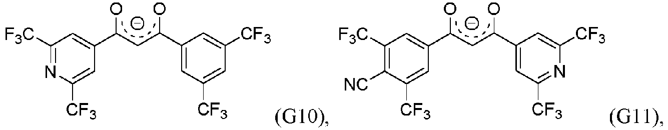

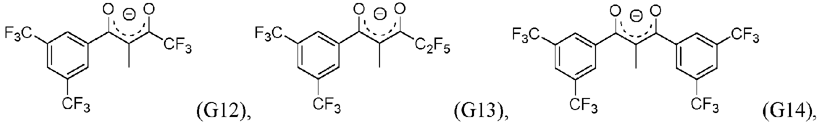

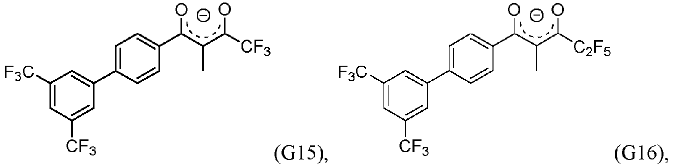

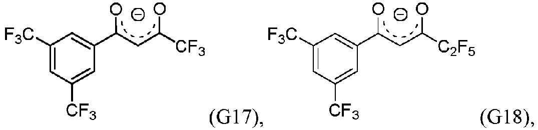

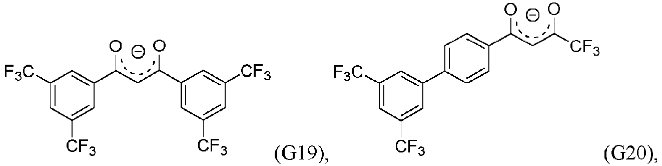

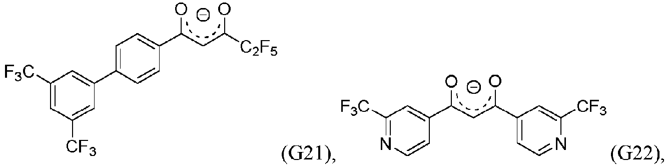

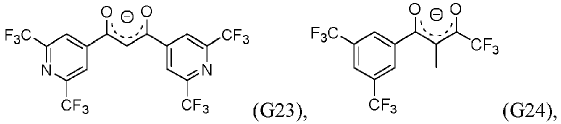

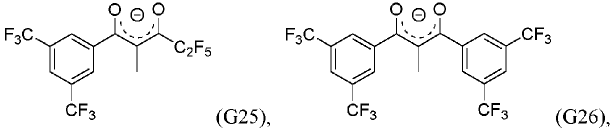

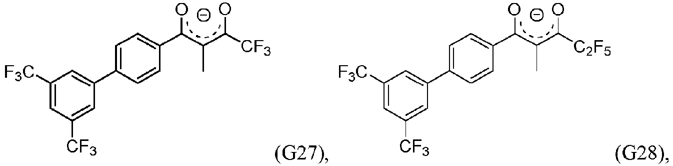

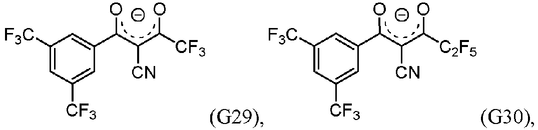

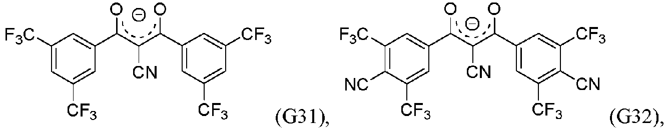

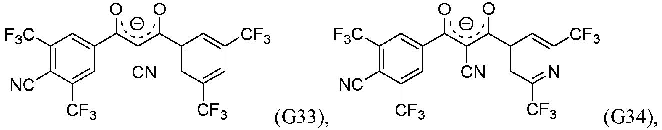

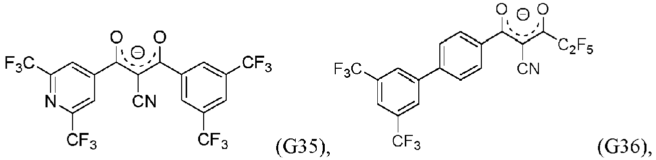

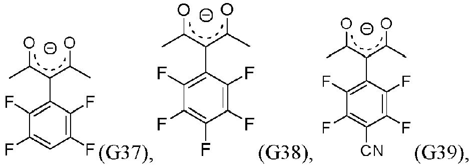

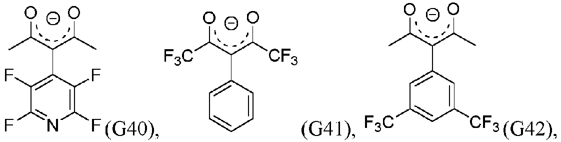

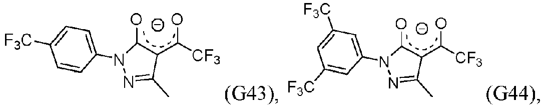

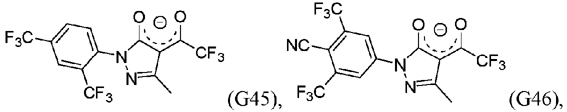

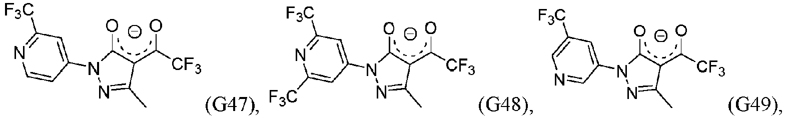

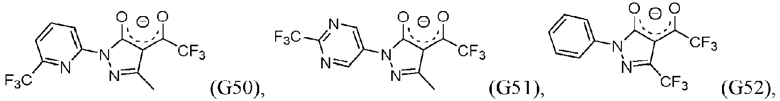

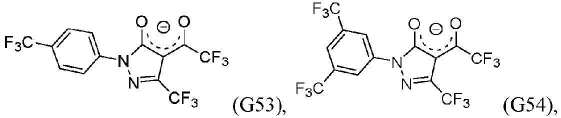

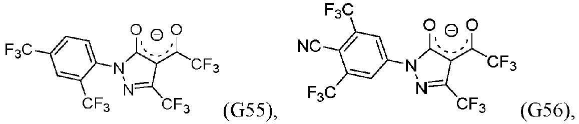

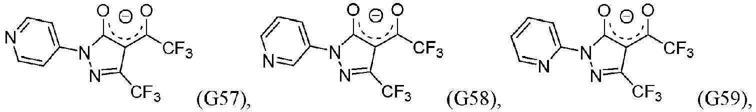

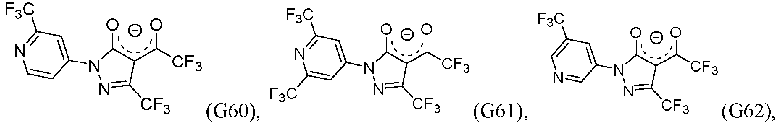

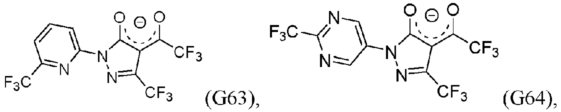

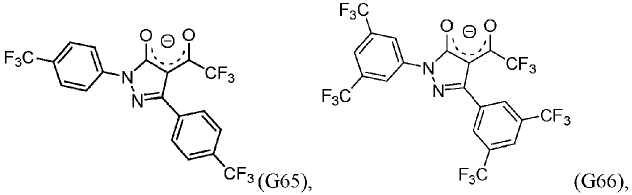

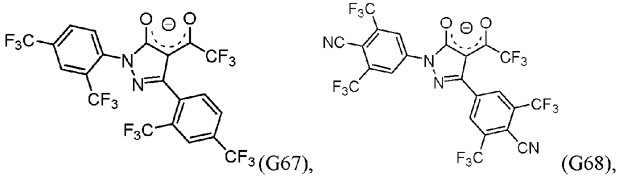

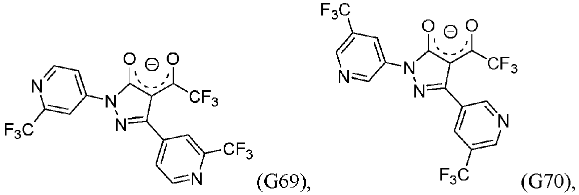

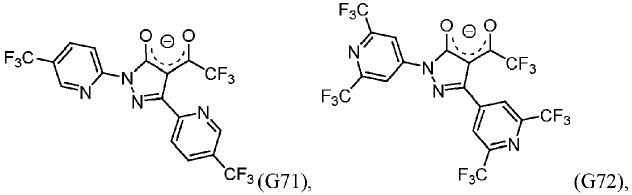

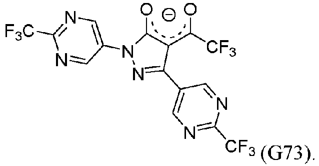

9. Organische elektronische Vorrichtung (100) nach einem der vorstehenden Ansprüche 1 bis 8, wobei die Verbindung der Formel (I) ausgewählt ist aus der Gruppe MC-1 bis MC-17:

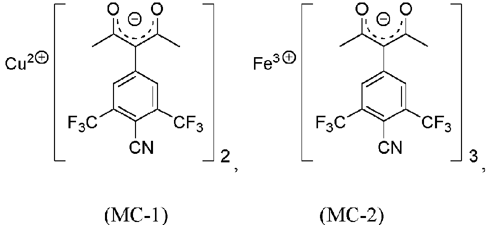

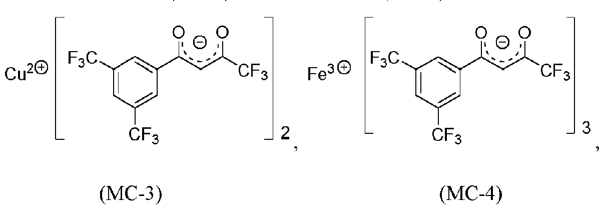

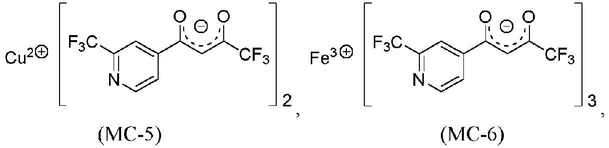

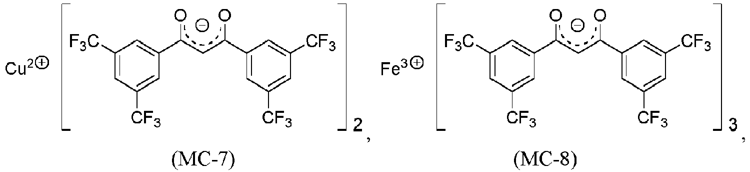

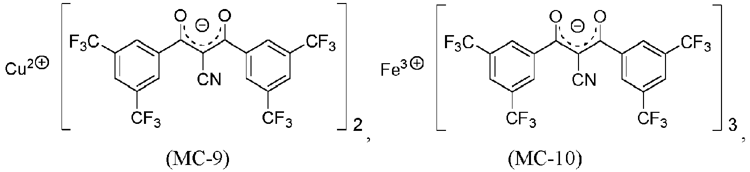

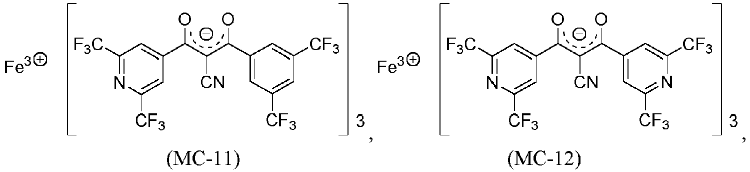

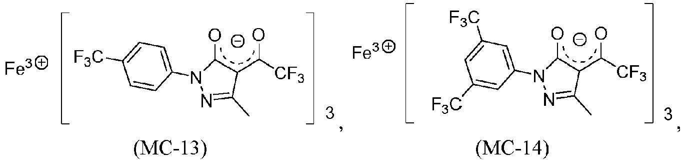

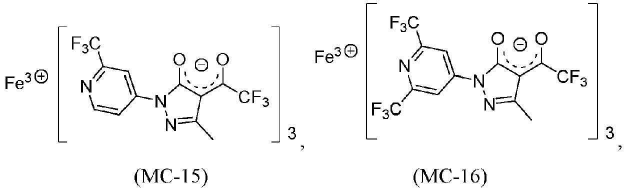

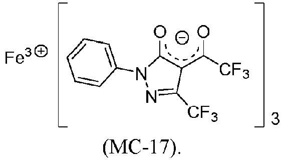

10. Organische elektronische Vorrichtung (100) nach einem der vorstehenden Ansprüche 1 bis 9, wobei die Lochinjektionsschicht (130) eine Verbindung der Formel (I) umfasst.

11. Organische elektronische Vorrichtung (100) nach einem der vorstehenden Ansprüche 1 bis 10, wobei die erste Emissionsschicht (150) eine Verbindung der Formel (VII) und eine organische Emitterwirtsverbindung umfasst: 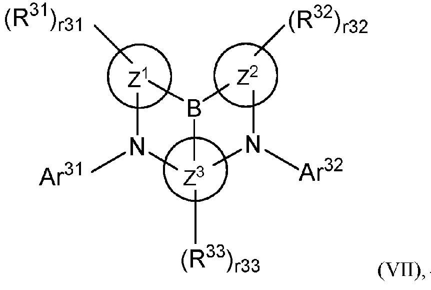 wobei
Z¹, Z² und Z³ gleich oder voneinander verschieden ausgewählt sind und und jeweils unabhängig ausgewählt sind aus der Gruppe umfassend einen monozyklischen bis polyzyklischen aromatischen Kohlenwasserstoffring oder einen monozyklischen bis polyzyklischen aromatischen Heteroring;
Ar³¹ und Ar³² gleich oder voneinander verschieden ausgewählt sind und jeweils unabhängig ausgewählt sind aus der Gruppe umfassend eine substituierte oder unsubstituierte Alkylgruppe, eine substituierte oder unsubstituierte Arylgruppe oder eine substituierte oder unsubstituierte Heteroarylgruppe, oder an einen benachbarten Substituenten gebunden sind, um einen substituierten oder unsubstituierten aromatischen Ring oder einen substituierten oder unsubstituierten aliphatischen Ring zu bilden;
R³¹, R³² und R³³ gleich oder verschieden voneinander ausgewählt sind und jeweils unabhängig ausgewählt sind aus der Gruppe umfassend Wasserstoff, Deuterium, eine substituierte oder unsubstituierte Alkylgruppe, eine substituierte oder unsubstituierte Silylgruppe, eine substituierte oder unsubstituierte Amingruppe, eine substituierte oder unsubstituierte Arylgruppe oder eine substituierte oder unsubstituierte Heteroarylgruppe, oder benachbarte Substituenten miteinander verbunden sind, um einen substituierten oder unsubstituierten aromatischen Ring oder einen substituierten oder unsubstituierten aliphatischen Ring zu bilden, wobei ein oder mehrere Substituenten ausgewählt sind aus der Gruppe bestehend aus Deuterium, einer Alkylgruppe mit 1 bis 6 Kohlenstoffatomen, einer Alkylsilylgruppe mit 1 bis 30 Kohlenstoffatomen, einer Arylsilylgruppe mit 6 bis 50 Kohlenstoffatomen, einer Alkylamingruppe mit 1 bis 30 Kohlenstoffatomen, einer Alkylarylamingruppe mit 1 bis 50 Kohlenstoffatomen, einer Arylamingruppe mit 6 bis 50 Kohlenstoffatomen, einer Arylgruppe mit 6 bis 30 Kohlenstoffatomen und einer Heteroarylgruppe mit 2 bis 30 Kohlenstoffatomen, oder einem Substituenten, mit dem zwei oder mehr Substituenten, die aus der Gruppe ausgewählt sind, verbunden sind, oder benachbarten Substituenten, die miteinander verbunden sind, um einen aliphatischen Kohlenwasserstoffring mit 3 bis 60 Kohlenstoffatomen zu bilden, der unsubstituiert oder mit dem Substituenten substituiert ist;
r31, r32 und r33 jeweils eine ganze Zahl von 0, 1, 2, 3 oder 4 sind, und wenn r31 bis r33 2 oder größer sind, Substituenten in der Klammer gleich oder voneinander verschieden ausgewählt sind.

12. Organische elektronische Vorrichtung (100) nach einem der vorstehenden Ansprüche 1 bis 11, wobei die organische Emitterwirtsverbindung umfasst:
- wenigstens ein kondensiertes aromatisches Ringsystem, das wenigstens ≥ 3 bis ≤ 5 kondensierte aromatische Ringe umfasst, und
- wenigstens 3 bis ≥ ≤ 7 aromatische Ringe und/oder wenigstens 3 bis ≥ ≤ 7 heteroaromatische Ringe, wobei wenigstens ≥ 2 bis ≤ 7 der aromatischen Ringe und/oder wenigstens ≥ 3 bis ≤ 7 der heteroaromatischen Ringe zu einem anellierten aromatischen Ringsystem und/oder anellierten heteroaromatischen Ringsystemen kondensiert sind, und
wobei das Molekulargewicht Mw der organischen Emitterwirtsverbindung in dem Bereich von ≥ 400 g/mol und ≤ 2000 g/mol liegt.

13. Organische elektronische Vorrichtung (100) nach einem der vorstehenden Ansprüche 1 bis 12, wobei die organische Emitterwirtsverbindung die Formel (VIII) aufweist: 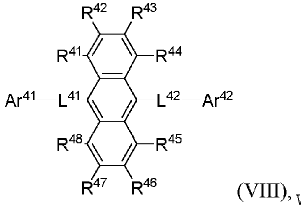 wobei
Ar⁴¹ und Ar⁴² unabhängig ausgewählt sind aus substituiertem oder unsubstituiertem C₆- bis C₂₄-Aryl, substituiertem oder unsubstituiertem C₃- bis C₂₄-Heteroaryl;
L⁴¹ und L⁴² unabhängig ausgewählt sind aus einer direkten Bindung oder substituiertem oder unsubstituiertem C₆-C₂₄-Arylen, substituiertem oder unsubstituiertem C₃-C₂₄-Heteroarylen;
R⁴¹ bis R⁴⁸ unabhängig ausgewählt sind aus H, D, substituiertem oder unsubstituiertem C₁- bis C₁₂-Alkyl, substituiertem oder unsubstituiertem C₆- bis C₁₉-Aryl, substituiertem oder unsubstituiertem C₃- bis C₁₂-Heteroaryl;
wobei
die Substituenten an Ar⁴¹, Ar⁴², L⁴¹, L⁴², R⁴¹ bis R⁴⁸ unabhängig ausgewählt sind aus D, C₆- bis C₁₀-Aryl, C₃-bis C₉-Heteroaryl, C₁- bis C₆-Alkyl, C₁- bis C₆-Alkoxy, verzweigtem C₃- bis C₆-Alkyl, cyclischem C₃- bis C₆-Alkyl, verzweigtem C₃- bis C₆-Alkoxy, cyclischem C₃- bis C₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₆-Alkyl, teil- oder perdeuteriertem C₁- bis C₆-Alkoxy, Halogen, F oder CN.

14. Organische elektronische Vorrichtung (100) nach einem der vorstehenden Ansprüche 1 bis 13, wobei Licht durch die Anodenschicht (190) emittiert wird.

15. Anzeigevorrichtung umfassend eine organische elektronische Vorrichtung (100) nach einem der vorstehenden Ansprüche 1 bis 14.

## Revendications

1. Dispositif électronique organique (100) comprenant une couche d'anode (120), une couche de cathode (190), une couche d'injection de trous (130), une première couche d'émission (150), et une couche de génération de charge (135, 185), dans lequel la couche de génération de charge (135, 185) comprend une couche de génération de charge de type p (135) et une couche de génération de charge de type n (185),
- la couche de génération de charge de type n (185) comprenant un composé organique de transport d'électrons et un dopant métallique, dans lequel le dopant métallique est sélectionné parmi un métal alcalin ou des terres rares ;
- la couche de génération de charge de type p (135) comprend :
- un composé organique de transport de trous, et
- un composé de formule (I) :
M^{n⊕}(L^{⊖})ₙ(AL)ₘ (I),
dans laquelle
M est un ion métallique ;
n est la valence de M et choisi parmi 1 à 3 ;
L est un ligand de formule (II) 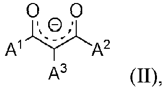
dans laquelle
A¹ et A² sont sélectionnés indépendamment parmi alkyle en C₁ à C₁₂ substitué ou non substitué, aryle en C₆ à C₁₂ substitué ou non substitué, hétéroaryle en C₃ à C₁₂ substitué ou non substitué ;
dans laquelle les substituants de A¹ et A² sont sélectionnés indépendamment parmi D, aryle en C₆, hétéroaryle en C₃ à C₉, alkyle en C₁ à C₆, alcoxy en C₁ à C₆, alkyle ramifié en C₃ à C₆, alkyle cyclique en C₃ à C₆, alcoxy ramifié en C₃ à C₆, alcoxy cyclique en C₃ à C₆, alkyle en C₁ à C₁₆ partiellement ou perfluoré, alcoxy en C₁ à C₁₆ partiellement ou perfluoré, alkyle en C₁ à C₆ partiellement ou perdeutéré, alcoxy en C₁ à C₆ partiellement ou perdeutéré, COR¹, COOR¹, SO₂R¹, halogène, F ou CN,
dans laquelle R¹ est sélectionné parmi aryle en C₆, hétéroaryle en C₃ à C₉, alkyle en C₁ à C₆, alcoxy en C₁ à C₆, alkyle ramifié en C₃ à C₆, alkyle cyclique en C₃ à C₆, alcoxy ramifié en C₃ à C₆, alcoxy cyclique en C₃ à C₆, alkyle en C₁ à C₁₆ partiellement ou perfluoré, alcoxy en C₁ à C₁₆ partiellement ou perfluoré, alkyle en C₁ à C₆ partiellement ou perdeutéré, alcoxy en C₁ à C₆ partiellement ou perdeutéré ;
A³ est choisi parmi H, D, CN, alkyle en C₁ à C4 substitué ou non substitué, alkyle en C₁ à C4 partiellement ou perfluoré, aryle en C₆ à C₁₀ substitué ou non substitué, hétéroaryle en C₃ à C₉ substitué ou non substitué, ou dans lequel A³ forme une structure cyclique substituée ou non substituée avec A¹ ou A²,
dans lequel le ou les substituants de A³ ou les substituants de la structure cyclique substituée sont indépendamment choisis parmi halogène, F, Cl, CN, CF₃ ;
AL est un ligand auxiliaire qui coordine au métal M ;
m est un entier choisi parmi 0 à 2 ;
dans laquelle
la couche d'injection de trous (130), la première couche d'émission (150) et la couche de génération de charge (135, 185) sont agencées entre la couche d'anode (120) et la couche de cathode (190), et la couche de génération de charge de type n (185) est agencée plus près de la couche d'anode (120) et la couche de génération de charge de type p (135) est agencée plus près de la couche de cathode (190).

2. Dispositif électronique organique (100) selon la revendication 1, dans lequel A³ est choisi parmi une structure cyclique avec A¹ ou A² de cycles aliphatiques ou aromatiques à 5 ou 6 chaînons, le cycle comprenant éventuellement un ou plusieurs hétéroatomes, de préférence choisis dans le groupe comprenant N, O et S.

3. Dispositif électronique organique (100) selon la revendication 1 ou 2, dans lequel
A¹ et A² sont indépendamment choisis parmi alkyle en C₁ à C₁₂ substitué ou non substitué, aryle en C₆ à C₁₂ substitué ou non substitué, hétéroaryle en C₃ à C₁₂ substitué ou non substitué, alkyle en C₁ à C₁₂ préférablement substitué, aryle en C₆ à C₁₂ substitué, hétéroaryle en C₃ à C₁₂ substitué ;
dans lequel les substituants de A¹ et A² sont sélectionnés indépendamment parmi D, aryle en C₆, hétéroaryle en C₃ à C₉, alkyle en C₁ à C₆, alcoxy en C₁ à C₆, alkyle ramifié en C₃ à C₆, alkyle cyclique en C₃ à C₆, alcoxy ramifié en C₃ à C₆, alcoxy cyclique en C₃ à C₆, alkyle en C₁ à C₁₆ partiellement ou perfluoré, alcoxy en C₁ à C₁₆ partiellement ou perfluoré, alkyle en C₁ à C₆ partiellement ou perdeutéré, alcoxy en C₁ à C₆ partiellement ou perdeutéré, COR¹, COOR¹, halogène, F ou CN,
dans laquelle R¹ est sélectionné parmi aryle en C₆, hétéroaryle en C₃ à C₉, alkyle en C₁ à C₆, alcoxy en C₁ à C₆, alkyle ramifié en C₃ à C₆, alkyle cyclique en C₃ à C₆, alcoxy ramifié en C₃ à C₆, alcoxy cyclique en C₃ à C₆, alkyle en C₁ à C₁₆ partiellement ou perfluoré, alcoxy en C₁ à C₁₆ partiellement ou perfluoré, alkyle en C₁ à C₆ partiellement ou perdeutéré, alcoxy en C₁ à C₆ partiellement ou perdeutéré ;
A³ est choisi parmi H, D, CN, CH₃, aryle en C₆ substitué ou non substitué, ou hétéroaryle en C₅ substitué ou non substitué, de préférence H ou CN ;
dans lequel le ou les substituants de A³ sont indépendamment choisis parmi halogène, F, Cl, CN, CF3.

4. Dispositif électronique organique (100) selon l'une quelconque des revendications précédentes 1 à 3, dans lequel la somme de A¹ et A² comprend ≥ 3 atomes de carbone et ≤ 30 atomes de carbone.

5. Dispositif électronique organique (100) selon l'une quelconque des revendications précédentes 1 à 4, dans lequel le composé organique de transport de trous est choisi parmi au moins un composé organique comprenant 1 à 3 groupements triarylamine, et 8 à 12 cycles aromatiques ou hétéroaromatiques, au moins un composé arylamine, un composé diarylamine, un composé triarylamine, un composé de formule (IIIa) ou un composé de formule (IIIb) : 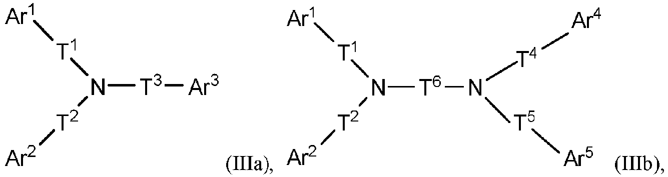 dans lesquelles :
T¹, T², T³, T⁴ et T⁵ sont indépendamment choisis parmi une simple liaison, un phénylène, un biphénylène, un terphénylène ou un naphténylène, de préférence une simple liaison ou un phénylène ;
T⁶ est phénylène, biphénylène, terphénylène ou naphténylène ;
Ar¹, Ar², Ar³, Ar⁴ et Ar⁵ sont indépendamment choisis parmi un aryle en C₆ à C₂₀ substitué ou non substitué, un hétéroarylène en C₃ à C₂₀ substitué ou non substitué, un biphénylène substitué ou non substitué, un fluorène substitué ou non substitué, un 9-fluorène substitué, un 9,9-fluorène substitué, un naphtalène substitué ou non substitué, un anthracène substitué ou non substitué, un phénanthrène substitué ou non substitué, un pyrène substitué ou non substitué, un pérylène substitué ou non substitué, un triphénylène substitué ou non substitué, un tétracène substitué ou non substitué, un tétraphène substitué ou non substitué, un dibenzofurane substitué ou non substitué, un dibenzothiophène substitué ou non substitué, un xanthène substitué ou non substitué, un carbazole substitué ou non substitué, un 9-phénylcarbazole substitué, une azépine substituée ou non substituée, une dibenzo[b,f]azépine substituée ou non substituée, un 9,9'-spirobi[fluorène] substitué ou non substitué, un spiro[fluorène-9,9'-xanthène] substitué ou non substitué, et un système de cycles aromatiques substitués ou non substitués condensés comprenant au moins trois cycles aromatiques substitués ou non substitués choisis dans le groupe comprenant les cycles à 5 chaînons non hétéroatomiques substitués ou non substitués, hétéroatomiques substitués ou non substitués, les cycles à 6 chaînons substitués ou non substitués et/ou les cycles à 7 chaînons substitués ou non substitués, un fluorène substitué ou non substitué, et un système de cycles condensés comprenant 2 à 6 cycles à 5 à 7 chaînons substitués ou non substitués, et les cycles sont choisis dans le groupe comprenant (i) un cycle à 5 à 7 chaînons insaturé d'un hétérocycle, (ii) 5 à 6 chaînons d'un hétérocycle aromatique, (iii) un cycle à 5 à 7 chaînons insaturé d'un non hétérocycle, (iv) un cycle à 6 chaînons d'un non hétérocycle aromatique ;
dans lesquelles
les substituants de Ar¹, Ar², Ar³, Ar⁴ et Ar⁵ sont sélectionnés identiques ou différents dans le groupe comprenant H, D, F, C(-O)R², CN, Si(R²)₃, P(-O)(R²)₂, OR², S(-O)R², S(-O)₂R², alkyle à chaîne linéaire substitué ou non substitué ayant 1 à 20 atomes de carbone, alkyle ramifié substitué ou non substitué ayant 1 à 20 atomes de carbone, alkyle cyclique substitué ou non substitué ayant 3 à 20 atomes de carbone, groupes alcényle ou alcynyle substitués ou non substitués ayant 2 à 20 atomes de carbone, groupes alcoxy substitués ou non substitués ayant 1 à 20 atomes de carbone, systèmes à cycles aromatiques substitués ou non substitués ayant 6 à 40 atomes de cycles aromatiques, et systèmes à cycles hétéroaromatiques substitués ou non substitués ayant 5 à 40 atomes à cycles aromatiques, aryle en C₆ à C₁₈ non substitué, hétéroaryle en C₃ à C₁₈ non substitué, un système à cycles fusionnés comprenant 2 à 6 cycles à 5 à 7 chaînons non substitués et les cycles sont sélectionnés dans le groupe comprenant un cycle à 5 à 7 chaînons insaturé d'un hétérocycle, 5 à 6 chaînons d'un hétérocycle aromatique, un cycle à 5 à 7 chaînons insaturé d'un non hétérocycle, et un cycle à 6 chaînons d'un non hétérocycle aromatique,
dans lesquelles R² est sélectionné parmi H, D, alkyle à chaîne linéaire ayant 1 à 6 atomes de carbone, alkyle ramifié ayant 1 à 6 atomes de carbone, alkyle cyclique ayant 3 à 6 atomes de carbone, groupes alcényle ou alcynyle ayant 2 à 6 atomes de carbone, aryle en C₆ à C₁₈, ou hétéroaryle en C₃ à C₁₈ ; et
dans lequel le poids moléculaire Mw du composé organique de transport de trous est dans la plage de ≥ 400 à ≤ 2 000 g/mole.

6. Dispositif électronique organique (100) selon l'une quelconque des revendications précédentes 1 à 5, dans lequel le ligand L de formule I comprend par ligand L au moins 14 atomes liés de manière covalente ; ou
le ligand L de formule I comprenant :
- au moins trois atomes de carbone, en variante au moins quatre atomes de carbone, et/ou
- au moins deux atomes d'oxygène ou un atome d'oxygène et un atome d'azote, deux à quatre atomes d'oxygène, deux à quatre atomes d'oxygène et zéro à deux atomes d'azote, et/ou
- au moins un ou plusieurs groupes choisis parmi halogène, F, CN, alkyle en C₁ à C₆ substitué ou non substitué, alcoxy en C₁ à C₆ substitué ou non substitué, en variante deux ou plusieurs groupes choisis parmi halogène, F, CN, alkyle en C₁ à C₆ substitué ou non substitué, alcoxy en C₁ à C₆ substitué ou non substitué,
au moins un ou plusieurs groupes choisis parmi halogène, F, CN, alkyle en C₁ à C₆ substitué, alcoxy en C₁ à C₆ substitué, en variante deux ou plusieurs groupes choisis parmi halogène, F, CN, alkyle en C₁ à C₆ perfluoré, alcoxy en C₁ à C₆ perfluoré, un ou plusieurs groupes choisis parmi alkyle en C₁ à C₆ substitué ou non substitué, aryle en C₆ à C₁₂ substitué ou non substitué, et/ou hétéroaryle en C₃ à C₁₂ substitué ou non substitué,
dans lequel les substituants sont sélectionnés parmi D, aryle en C₆, hétéroaryle en C₃ à C₉, alkyle en C₁ à C₆, alcoxy en C₁ à C₆, alkyle ramifié en C₃ à C₆, alkyle cyclique en C₃ à C₆, alcoxy ramifié en C₃ à C₆, alcoxy cyclique en C₃ à C₆, alkyle en C₁ à C₁₆ partiellement ou perfluoré, alcoxy en C₁ à C₁₆ partiellement ou perfluoré, alkyle en C₁ à C₆ partiellement ou perdeutéré, alcoxy en C₁ à C₆ partiellement ou perdeutéré, COR³, COOR³, halogène, F ou CN ;
dans lequel R³ est sélectionné parmi aryle en C₆, hétéroaryle en C₃ à C₉, alkyle en C₁ à C₆, alcoxy en C₁ à C₆, alkyle ramifié en C₃ à C₆, alkyle cyclique en C₃ à C₆, alcoxy ramifié en C₃ à C₆, alcoxy cyclique en C₃ à C₆, alkyle en C₁ à C₁₆ partiellement ou perfluoré, alcoxy en C₁ à C₁₆ partiellement ou perfluoré, alkyle en C₁ à C₆ partiellement ou perdeutéré, alcoxy en C₁ à C₆ partiellement ou perdeutéré.

7. Dispositif électronique organique (100) selon l'une quelconque des revendications précédentes 1 à 6, dans lequel A¹ et/ou A² sont choisis parmi les formules suivantes D1 à D68 : 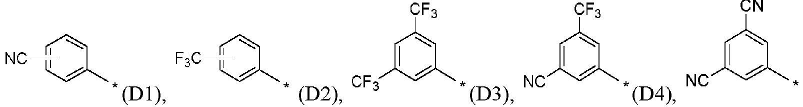 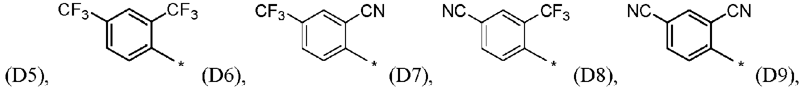 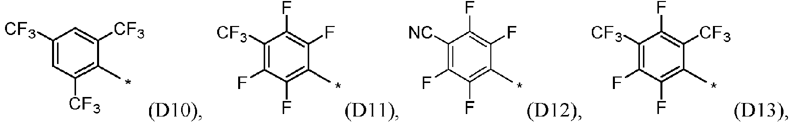 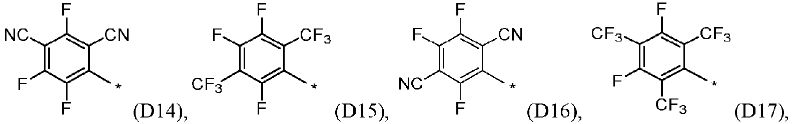 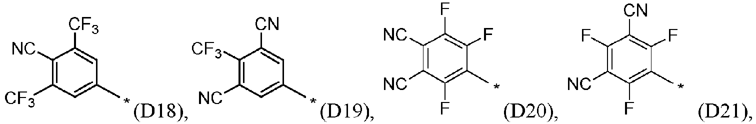 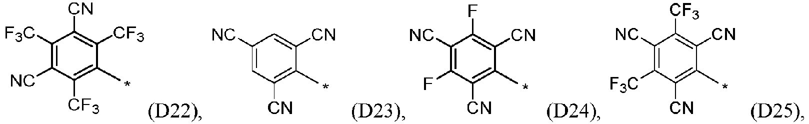 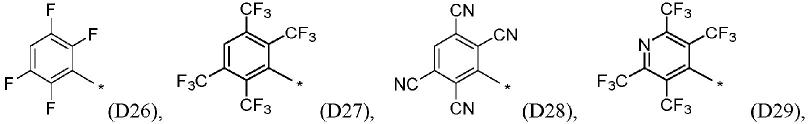 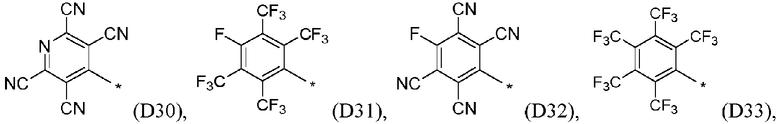 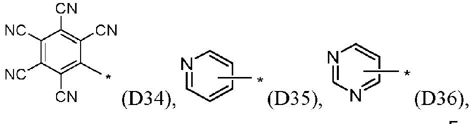 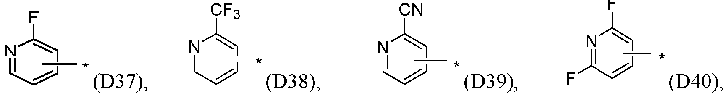 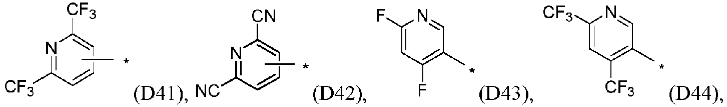 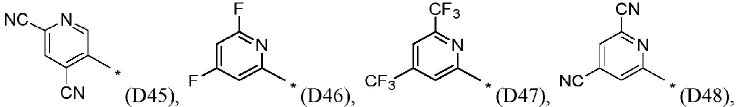 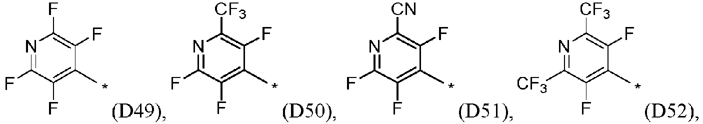 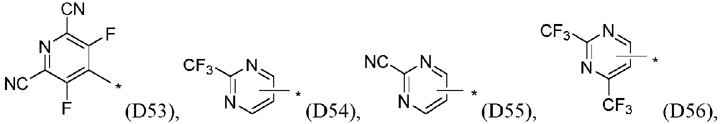 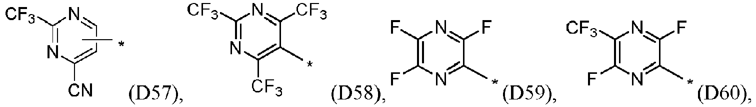 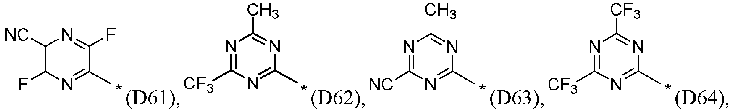 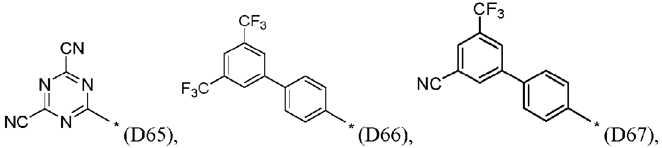 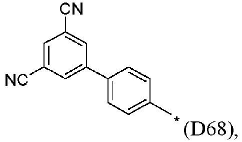 dans lesquelles « * » désigne la position de liaison.

8. Dispositif électronique organique (100) selon l'une quelconque des revendications précédentes 1 à 7, dans lequel le ligand L de formule (I) est choisi parmi G1 à G73 :

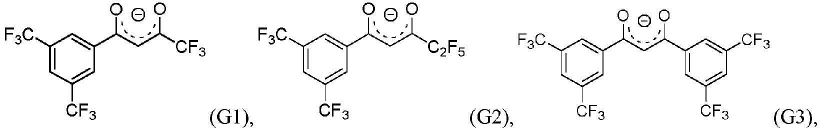

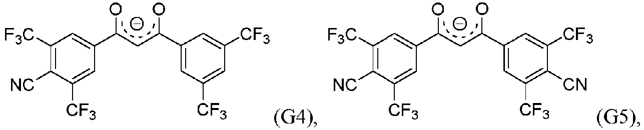

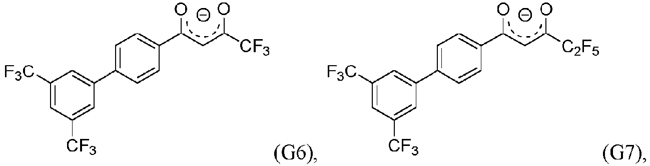

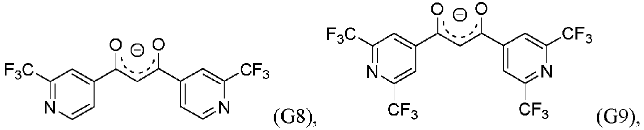

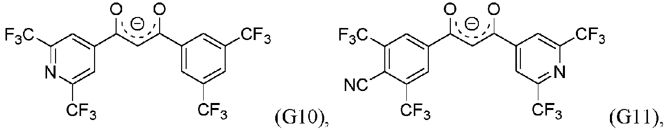

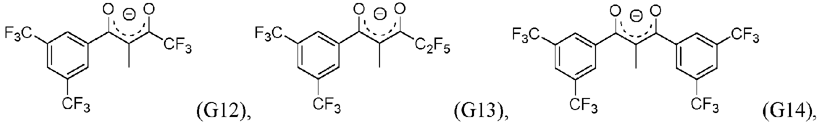

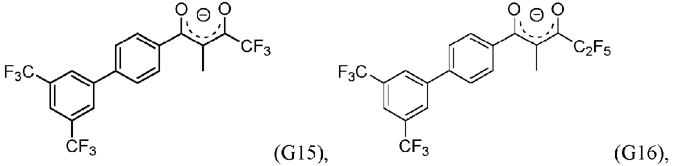

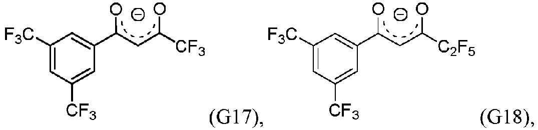

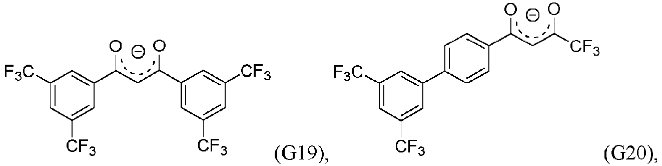

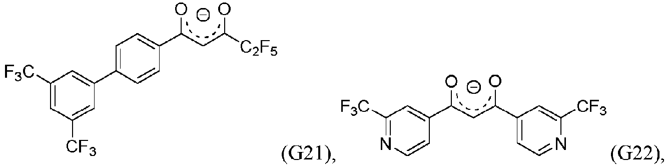

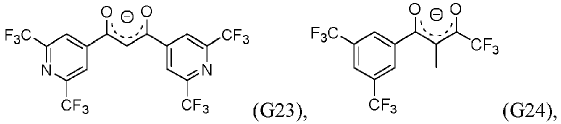

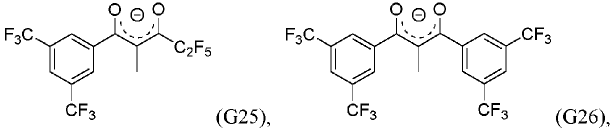

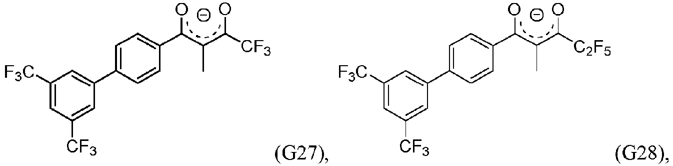

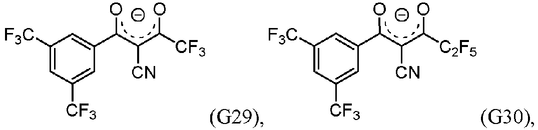

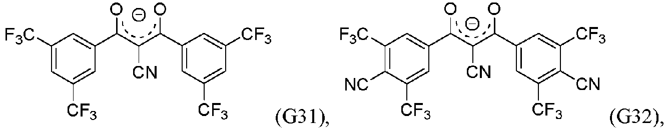

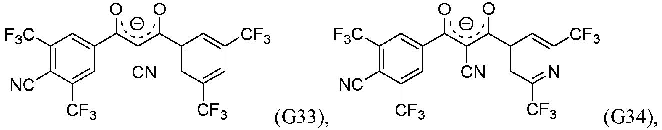

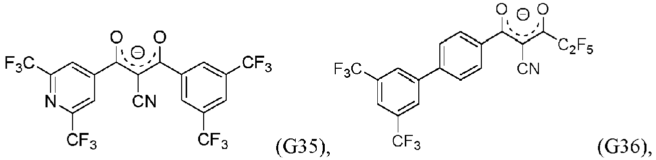

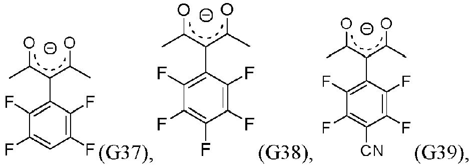

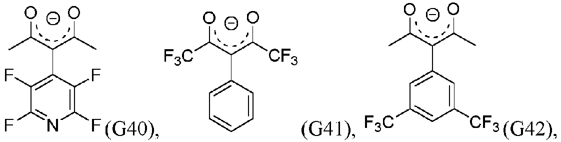

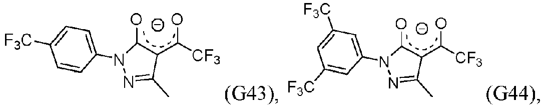

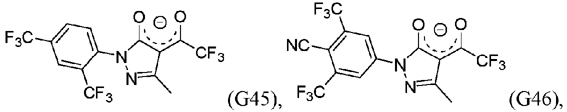

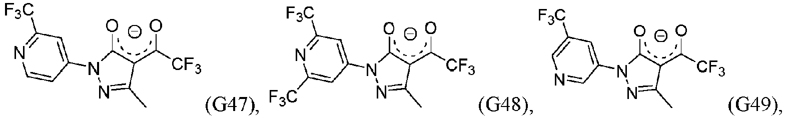

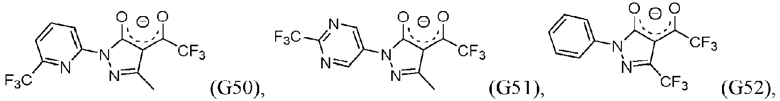

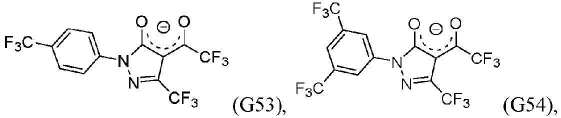

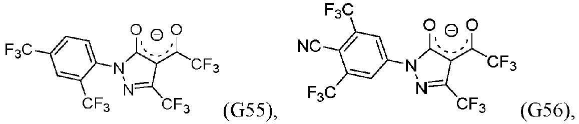

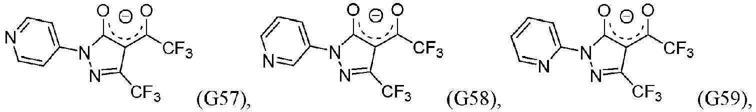

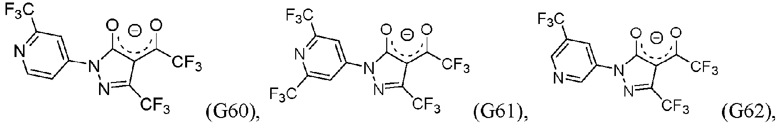

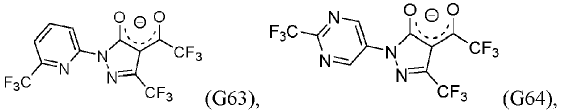

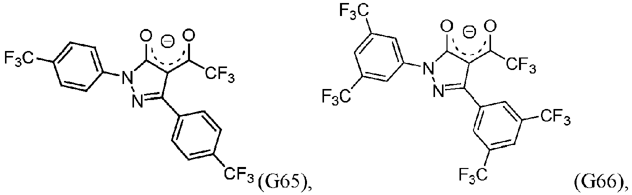

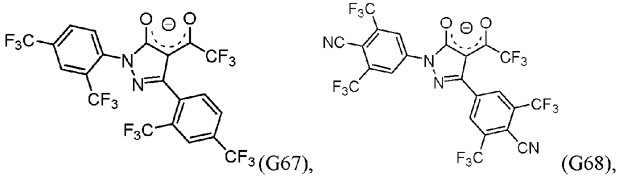

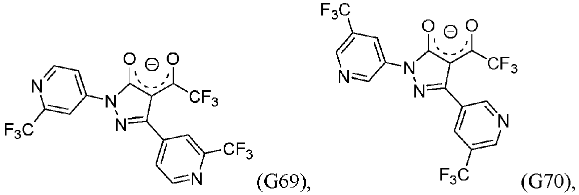

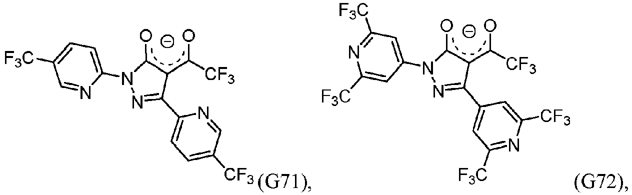

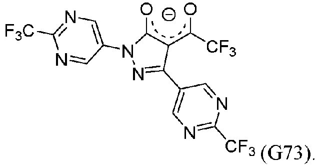

9. Dispositif électronique organique (100) selon l'une quelconque des revendications précédentes 1 à 8, dans lequel le composé de formule (I) est choisi dans le groupe de MC-1 à MC-17 :

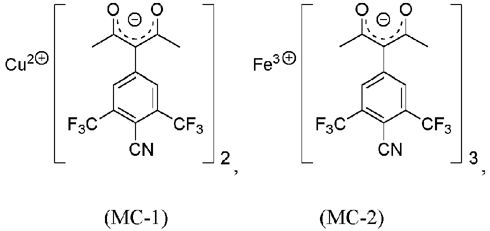

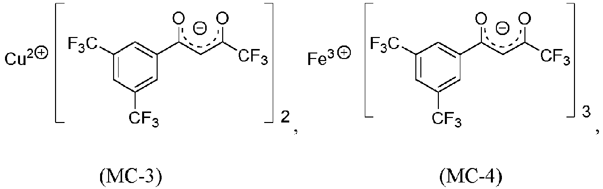

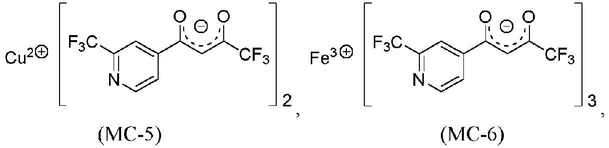

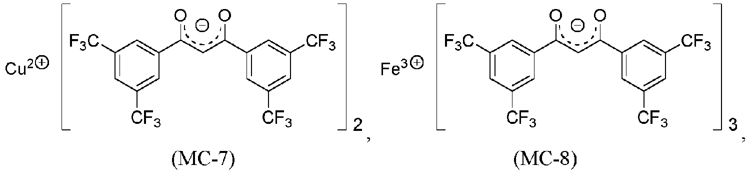

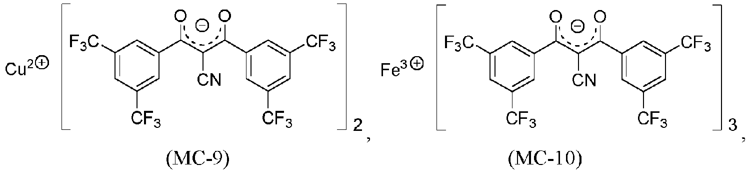

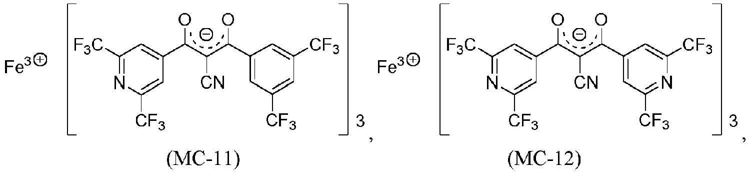

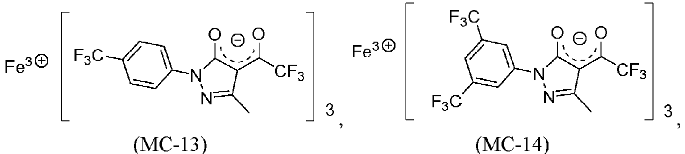

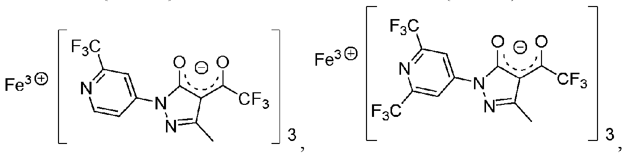

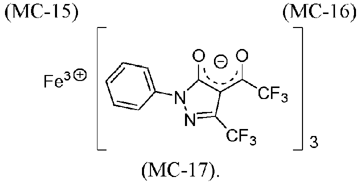

10. Dispositif électronique organique (100) selon l'une quelconque des revendications précédentes 1 à 9, dans lequel la couche d'injection de trous (130) comprend un composé de formule (I).

11. Dispositif électronique organique (100) selon l'une quelconque des revendications précédentes 1 à 10, dans lequel la première couche d'émission (150) comprend un composé de formule (VII) et un composé hôte émetteur organique : 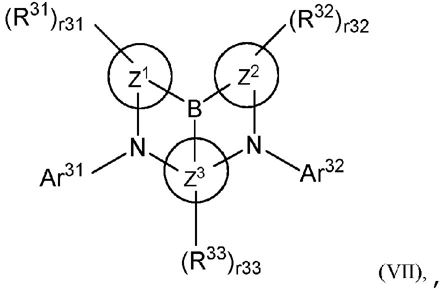 dans laquelle
Z¹, Z² et Z³ sont identiques ou différents les uns des autres, et sont chacun indépendamment choisis dans le groupe comprenant un cycle hydrocarboné aromatique monocyclique à polycyclique et un hétérocycle aromatique monocyclique à polycyclique ;
Ar³¹ et Ar³² sont identiques ou différents les uns des autres, et sont chacun indépendamment choisis dans le groupe comprenant un groupe alkyle substitué ou non substitué, un groupe aryle substitué ou non substitué ou un groupe hétéroaryle substitué ou non substitué, ou sont liés à un substituant adjacent pour former un cycle aromatique substitué ou non substitué ou un cycle aliphatique substitué ou non substitué ;
R³¹, R³² et R³³ sont identiques ou différents les uns des autres, et sont chacun indépendamment choisis dans le groupe comprenant l'hydrogène, le deutérium, un groupe alkyle substitué ou non substitué, un groupe silyle substitué ou non substitué, un groupe amino substitué ou non substitué, un groupe aryle substitué ou non substitué et un groupe hétéroaryle substitué ou non substitué, ou des substituants adjacents sont liés les uns aux autres pour former un cycle aromatique substitué ou non substitué ou un cycle aliphatique substitué ou non substitué, un ou plusieurs substituants choisis dans le groupe constitué par le deutérium, un groupe alkyle ayant 1 à 6 atomes de carbone, un groupe alkylsilyle ayant 1 à 30 atomes de carbone, un groupe arylsilyle ayant 6 à 50 atomes de carbone, un groupe alkylamino ayant 1 à 30 atomes de carbone, un groupe alkylarylamino ayant 1 à 50 atomes de carbone, un groupe arylamino ayant 6 à 50 atomes de carbone, un groupe aryle ayant 6 à 30 atomes de carbone et un groupe hétéroaryle ayant 2 à 30 atomes de carbone, ou un substituant auquel au moins deux substituants choisis dans le groupe sont liés, ou des substituants adjacents étant liés les uns aux autres pour former un cycle hydrocarboné aliphatique ayant 3 à 60 atomes de carbone, qui est non substitué ou substitué par le substituant ;
r31, r32 et r33 sont chacun un entier de 0, 1, 2, 3 ou 4, et lorsque r31 à r33 sont 2 ou plus, les substituants entre parenthèses sont identiques ou différents les uns des autres.

12. Dispositif électronique organique (100) selon l'une quelconque des revendications précédentes 1 à 11, dans lequel le composé hôte émetteur organique comprend :
- au moins un système cyclique aromatique condensé comprenant d'au moins ≥ 3 à ≤ 5 cycles aromatiques condensés, et
- au moins ≥ 3 à ≤ 7 cycles aromatiques et/ou au moins ≥ 3 à ≤ 7 cycles hétéroaromatiques, où au moins ≥ 2 à ≤ 7 des cycles aromatiques et/ou au moins ≥ 3 à ≤ 7 des cycles hétéroaromatiques sont condensés pour former un système cyclique aromatique condensé et/ou des systèmes cycliques hétéroaromatiques condensés, et
dans lequel le poids moléculaire Mw du composé hôte émetteur organique est dans la plage de ≥ 400 g/mole à ≤ 2 000 g/mole.

13. Dispositif électronique organique (100) selon l'une quelconque des revendications précédentes 1 à 12, dans lequel le composé hôte émetteur organique possède la formule (VIII) : 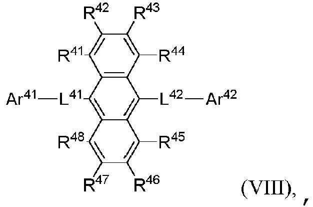 dans laquelle
Ar⁴¹ et Ar⁴² sont indépendamment choisis parmi aryle en C₆ à C₂₄ substitué ou non substitué, hétéroaryle en C₃ à C₂₄ substitué ou non substitué ;
L⁴¹ et L⁴² sont choisis indépendamment parmi une liaison directe ou arylène en C₆ à C₂₄ substitué ou non substitué, hétéroarylène en C₃ à C₂₄ substitué ou non substitué ;
R⁴¹ à R⁴⁸ sont indépendamment choisis parmi H, D, alkyle en C₁ à C₁₂ substitué ou non substitué, aryle en C₆ à C₁₉ substitué ou non substitué, hétéroaryle en C₃ à C₁₂ substitué ou non substitué ;
dans laquelle
les substituants sur Ar⁴¹, Ar⁴², L⁴¹, L⁴², R⁴¹ à R⁴⁸ sont sélectionnés indépendamment parmi D, aryle en C₆ à C₁₀, hétéroaryle en C₃ à C₉, alkyle en C₁ à C₆, alcoxy en C₁ à C₆, alkyle ramifié en C₃ à C₆, alkyle cyclique en C₃ à C₆, alcoxy ramifié en C₃ à C₆, alcoxy cyclique en C₃ à C₆, alkyle en C₁ à C₁₆ partiellement ou perfluoré, alcoxy en C₁ à C₁₆ partiellement ou perfluoré, alkyle en C₁ à C₆ partiellement ou perdeutéré, alcoxy en C₁ à C₆ partiellement ou perdeutéré, halogène, F ou CN.

14. Dispositif électronique organique (100) selon l'une quelconque des revendications précédentes 1 à 13, dans lequel de la lumière est émise à travers la couche d'anode (190).

15. Dispositif d'affichage comprenant un dispositif électronique organique (100) selon l'une quelconque des revendications précédentes 1 à 14.
